# EUROPEAN PATENT APPLICATION

(11) **EP 2 390 262 A1**
(43) Date of publication of application: **30.11.2011**
(21) Application number: 11174221.9
(22) Date of filing: 13.05.2004
(51) Int. Cl.: C07K 14/52, A61K 38/19

(54) **Synthetic chemokine receptor ligands and methods of use thereof**

(30) Priority: 16.05.2003 US 471404 P
(62) Divisional of application: 04776018.6
(71) Applicant: Intermune, Inc., Brisbane, CA 94005 (US)
(72) Inventor: Blatt, Lawrence M., San Francisco, CA California 94121 (US)
(74) Representative: Viering, Jentschura & Partner

(57) **Abstract**

The present invention provides synthetic CXCR3 ligands, including consensus CXCR3 ligands and hybrid CXCR3 ligands; and compositions comprising the ligands. The present invention provides polynucleotides encoding the synthetic CXCR3 ligands; expression vectors comprising the polynucleotides; and host cells comprising the polynucleotidess. The present invention provides methods of treating fibrotic disorders; methods of treating angiogenic disorders; methods of treating cancer; and methods of treating bacterial infections. The methods generally involve administering to an individual in need thereof an effective amount of a subject synthetic CXCR3 ligand.

## Description

### CROSS-REFERENCE

This application claims the benefit of U.S. Provisional Patent Application No. 60/471,404, filed May 16, 2003, which application is incorporated herein by reference in its entirety.

### FIELD OF THE INVENTION

The present invention is in the field of ligands for chemokine receptors, and in the treatment of fibrotic disorders, angiogenic disorder, cancer, and bacterial infections.

### BACKGROUND OF THE INVENTION

Chemokines constitute a family of small cytokines that are produced in inflammation and regulate leukocyte recruitment. Chemokines are capable of selectively inducing chemotaxis of the formed elements of the blood (other than red blood cells), including leukocytes such as neutrophils, monocytes, macrophages, eosinophils, basophils, mast cells, and lymphocytes, such as T cells and B cells. In addition to stimulating chemotaxis, other changes can be selectively induced by chemokines in responsive cells, including changes in cell shape, transient rises in the concentration of intracellular free calcium ions, granule exocytosis, integrin upregulation, formation of bioactive lipids (e.g., leukotrienes) and respiratory burst, associated with leukocyte activation. Thus, the chemokines are early triggers of the inflammatory response, causing inflammatory mediator release, chemotaxis and extravasation to sites of infection or inflammation.

Four families of chemokines have been identified, grouped according to the number and arrangement of conserved amino-terminal cysteine motifs: C, CC, CXC, and CX₃C, where "X" is a nonconserved amino acid residue. CXC chemokines include IL-8, IP-10, Mig, PF4, ENA-78, GCP-2, GROα, GROβ, GROγ, NAP-2, NAP-4'. Many CXC chemokines attract neutrophil leukocytes. For example, the CXC chemokines interleukin 8 (IL-8), platelet factor 4 (PF4), and neutrophil-activating peptide 2 (NAP-2) are potent chemoattractants and activators of neutrophils. The CXC chemokines designated Mig (monokine induced by gamma interferon) and IP-10 (interferon-gamma inducible 10 kDa protein) are active in inducing chemotaxis of activated peripheral blood lymphocytes.

CC and CXC chemokines act through receptors which belong to a superfamily of seven transmembrane spanning G protein-coupled receptors. This family of G-protein coupled (serpentine) receptors comprises a large group of integral membrane proteins, containing seven transmembrane-spanning regions. The receptors are coupled to G proteins, which are heterotrimeric regulatory proteins capable of binding GTP and mediating signal transduction from coupled receptors, for example, by the production of intracellular mediators.

The CXC chemokine receptors 1 through 4 (CXCR1-4) bind CXC chemokines. CXCR3 (CD183) is the receptor for IP10, Mig, and I-TAC. Signaling through CXCR3 induces chemotactic migration of inflammation-associated effector T cells.

There is a need in the art for improved treatments for disorders such as cancer, fibrotic disorders, and bacterial infections. The present invention addresses this need.

### Literature

U.S. Patent No. 6,184,358; U.S. Patent No. 6,491,906; U.S. Patent No. 5,871,723; Cole et al. (2001) J. Immunol. 167:623-627.

### SUMMARY OF THE INVENTION

The present invention provides synthetic CXCR3 ligands, including consensus CXCR3 ligands and hybrid CXCR3 ligands; and compositions comprising the ligands. The present invention provides polynucleotides encoding the synthetic CXCR3 ligands; expression vectors comprising the polynucleotides; and host cells comprising the polynucleotidess. The present invention provides methods of treating fibrotic disorders; methods of treating angiogenic disorders; methods of treating cancer; and methods of treating bacterial infections. The methods generally involve administering to an individual in need thereof an effective amount of a subject synthetic CXCR3 ligand.

### BRIEF DESCRIPTION OF THE DRAWING

Figure 1 provides the amino acid sequences of exemplary CXCR3 consensus ligands "IP-10 consensus sequence" (SEQ ID NO:01), "I-TAC consensus sequence" (SEQ ID NO:02), "Mig consensus sequence" (SEQ ID NO:03); and "Majority" sequence (SEQ ID NO:11).

Figure 2 provides an alignment of amino acid sequences of IP-10 (SEQ ID NO:12); iTAC (SEQ ID NO:13); and MIG (SEQ ID NO:14).

Figure 3 provides the amino acid sequence of an exemplary hybrid CXCR3 ligand (SEQ ID NO:15).

Figure 4 provides the amino acid sequence of an exemplary hybrid CXCR3 ligand (SEQ ID NO:16).

Figure 5 provides the amino acid sequence of an exemplary hybrid CXCR3 ligand (SEQ ID NO:17).

Figure 6 provides the amino acid sequence of an exemplary hybrid CXCR3 ligand (SEQ ID NO:18).

Figure 7 provides the amino acid sequence of an exemplary hybrid CXCR3 ligand (SEQ ID NO:19).

Figure 8 provides the amino acid sequence of an exemplary hybrid CXCR3 ligand (SEQ ID NO:20).

### DEFINITIONS

The term "polypeptide" refers to a polymer of amino acids and does not refer to a specific length of the product; thus, peptides, oligopeptides, and proteins are included within the definition of polypeptide. This term also does not refer to or exclude post-translational modifications of the polypeptide, for example, glycosylations, acetylations, phosphorylations and the like. Included within the term "polypeptide" are, for example, polypeptides containing one or more analogs of an amino acid (including, for example, unnatural amino acids, non-coded amino acids, etc.), polypeptides with substituted linkages, as well as other modifications known in the art, both naturally occurring and non-naturally occurring. The term "polypeptide" includes fusion proteins, including, but not limited to, fusion proteins with a heterologous amino acid sequence, fusions with heterologous and homologous leader sequences, with or without N-terminal methionine residues; immunologically tagged proteins; and the like.

The terms "polynucleotide" and "nucleic acid molecule" are used interchangeably herein to refer to polymeric forms of nucleotides of any length. The polynucleotides may contain deoxyribonucleotides, ribonucleotides, and/or their analogs. Nucleotides may have any three-dimensional structure, and may perform any function, known or unknown. The term "polynucleotide" includes single-, double-stranded and triple helical molecules. "Oligonucleotide" generally refers to polynucleotides of between about 5 and about 100 nucleotides of single- or double-stranded DNA. However, for the purposes of this disclosure, there is no upper limit to the length of an oligonucleotide. Oligonucleotides are also known as oligomers or oligos and may be isolated from genes, or chemically synthesized by methods known in the art. The term "polynucleotide" includes double-stranded DNA found, inter alia, in linear DNA molecules (e.g., restriction fragments), viruses, plasmids, and chromosomes.

The following are non-limiting embodiments of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes, and primers. A nucleic acid molecule may also comprise modified nucleic acid molecules, such as methylated nucleic acid molecules and nucleic acid molecule analogs. Analogs of purines and pyrimidines are known in the art. Nucleic acids may be naturally occurring, *e.g*. DNA or RNA, or may be synthetic analogs, as known in the art. Such analogs may be preferred for use as probes because of superior stability under assay conditions. Modifications in the native structure, including alterations in the backbone, sugars or heterocyclic bases, have been shown to increase intracellular stability and binding affinity. Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include 3'-O'-5'-S-phosphorothioate, 3'-S-5'-O- phosphorothioate, 3'-CH₂-5'-O-phosphonate and 3'-NH-5'-O-phosphoroamidate. Peptide nucleic acids replace the entire ribose phosphodiester backbone with a peptide linkage.

A polynucleotide or polypeptide has a certain percent "sequence identity" to another polynucleotide or polypeptide, meaning that, when aligned, that percentage of bases or amino acids are the same when comparing the two sequences. Sequence similarity can be determined in a number of different manners. To determine sequence identity, sequences can be aligned using the methods and computer programs, including BLAST, available over the world wide web at ncbi.nlm.nih.gov/BLAST. See, e.g., Altschul et al. (1990), J. Mol. Biol. 215:403-10. Another alignment algorithm is FASTA, available in the Genetics Computing Group (GCG) package, from Madison, Wisconsin, USA, a wholly owned subsidiary of Oxford Molecular Group, Inc. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Of particular interest are alignment programs that permit gaps in the sequence. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. See J. Mol. Biol. 48: 443-453 (1970)

The term "host cell" includes an individual cell or cell culture which can be or has been a recipient of any recombinant vector(s) or isolated polynucleotide of the invention. Host cells include progeny of a single host cell, and the progeny may not necessarily be completely identical (in morphology or in total DNA complement) to the original parent cell due to natural, accidental, or deliberate mutation and/or change. A host cell includes cells transfected or infected *in vivo* or *in vitro* with a recombinant vector or a polynucleotide of the invention. A host cell which comprises a recombinant vector of the invention is a "recombinant host cell".

The terms "DNA regulatory sequences", and "regulatory elements", used interchangeably herein, refer to transcriptional and translational control sequences, such as promoters, enhancers, polyadenylation signals, terminators, protein degradation signals, and the like, that provide for and/or regulate expression of a coding sequence and/or production of an encoded polypeptide in a host cell.

The term "transformation" is used interchangeably with "genetic modification" and refers to a permanent or transient genetic change induced in a cell following introducton of new DNA (i.e., DNA exogenous to the cell). Genetic change ("modification") can be accomplished either by incorporation of the new DNA into the genome of the host cell, or by transient or stable maintenance of the new DNA as an episomal element. Where the cell is a mammalian cell, a permanent genetic change is generally achieved by introduction of the DNA into the genome of the cell.

"Operably linked" refers to a juxtaposition wherein the components so described are in a relationship permitting them to function in their intended manner. For instance, a promoter is operably linked to a coding sequence if the promoter effects its transcription or expression.

By "construct" is meant a recombinant nucleic acid, generally recombinant DNA, that has been generated for the purpose of the expression of a specific nucleotide sequence(s), or is to be used in the construction of other recombinant nucleotide sequences.

The term "binds specifically," in the context of antibody binding, refers to high avidity and/or high affinity binding of an antibody to a specific polypeptide i.e., epitope of a polypeptide, e.g., a synthetic CXCR3 ligand (e.g. a hybrid CXCR3 ligand or a consensus CXCR3 ligand). For example, antibody binding to an epitope on a specific a synthetic CXCR3 ligand polypeptide or fragment thereof is stronger than binding of the same antibody to any other epitope, particularly those which may be present in molecules in association with, or in the same sample, as the specific polypeptide of interest, e.g., binds more strongly to a specific synthetic CXCR3 ligand (e.g. a hybrid CXCR3 ligand or a consensus CXCR3 ligand) than to a different synthetic CXCR3 ligand (e.g. a hybrid CXCR3 ligand or a consensus CXCR3 ligand) epitope so that by adjusting binding conditions the antibody binds almost exclusively to the specific synthetic CXCR3 ligand (e.g. a hybrid CXCR3 ligand or a consensus CXCR3 ligand) epitope and not to any other synthetic CXCR3 ligand (e.g. a hybrid CXCR3 ligand or a consensus CXCR3 ligand) epitope, and not to any other synthetic CXCR3 ligand (e.g. a hybrid CXCR3 ligand or a consensus CXCR3 ligand) polypeptide (or fragment) or any other polypeptide which does not comprise the epitope. Antibodies which bind specifically to a polypeptide may be capable of binding other polypeptides at a weak, yet detectable, level (e.g., 10% or less of the binding shown to the polypeptide of interest). Such weak binding, or background binding, is readily discernible from the specific antibody binding to a subject polypeptide, e.g. by use of appropriate controls. In general, specific antibodies bind to a given polypeptide with a binding affinity of 10⁻⁷ M or more, e.g., 10⁻⁸ M or more (e.g., 10⁻⁹ M, 10⁻¹⁰ M, 10⁻¹¹ M, etc.). In general, an antibody with a binding affinity of 10⁻⁶ M or less is not useful in that it will not bind an antigen at a detectable level using conventional methodology currently used.

As used herein, the terms "treatment," "treating," and the like, refer to obtaining a desired pharmacologic and/or physiologic effect. The effect may be prophylactic in terms of completely or partially preventing a disease or symptom thereof and/or may be therapeutic in terms of a partial or complete cure for a disease and/or adverse affect attributable to the disease. "Treatment", as used herein, covers any treatment of a disease in a mammal, particularly in a human, and includes: (a) increasing survival time; (b) decreasing the risk of death due to the disease; (c) preventing the disease from occurring in a subject which may be predisposed to the disease but has not yet been diagnosed as having it; (d) inhibiting the disease, i.e., arresting its development (e.g., reducing the rate of disease progression); and (e) relieving the disease, i.e., causing regression of the disease.

The terms "individual," "host," "subject," and "patient," used interchangeably herein, refer to a mammal, e.g., a human.

The term "therapeutically effective amount" is meant an amount of a therapeutic agent, or a rate of delivery of a therapeutic agent, effective to facilitate a desired therapeutic effect. The precise desired therapeutic effect will vary according to the condition to be treated, the formulation to be administered, and a variety of other factors that are appreciated by those of ordinary skill in the art.

A "fibrotic condition," "fibrotic disease," and "fibrotic disorder" are used interchangeably to refer to a condition, disease or disorder that is amenable to treatment by administration of a compound having anti-fibrotic activity. Fibrotic disorders include, but are not limited to, pulmonary fibrosis, including idiopathic pulmonary fibrosis (IPF) and pulmonary fibrosis from a know etiology, liver fibrosis, and renal fibrosis. Other exemplary fibrotic conditions include musculoskeletal fibrosis, cardiac fibrosis, post-surgical adhesions, scleroderma, glaucoma, and skin lesions such as keloids.

The terms "cancer," "neoplasm," and "tumor," are used interchangeably herein to refer to cells which exhibit relatively autonomous growth, so that they exhibit an aberrant growth phenotype characterized by a significant loss of control of cell proliferation. Cancerous cells can be benign or malignant.

The term "dosing event" as used herein refers to administration of an antiviral agent to a patient in need thereof, which event may encompass one or more releases of an antiviral agent from a drug dispensing device. Thus, the term "dosing event," as used herein, includes, but is not limited to, installation of a continuous delivery device (e.g., a pump or other controlled release injectable system); and a single subcutaneous injection followed by installation of a continuous delivery system.

"Patterned" or "temporal" as used in the context of drug delivery is meant delivery of drug in a pattern, generally a substantially regular pattern, over a pre-selected period of time (*e.g*., other than a period associated with, for example a bolus injection). "Patterned" or "temporal" drug delivery is meant to encompass delivery of drug at an increasing, decreasing, substantially constant, or pulsatile, rate or range of rates (*e.g*., amount of drug per unit time, or volume of drug formulation for a unit time), and further encompasses delivery that is continuous or substantially continuous, or chronic.

The term "controlled drug delivery device" is meant to encompass any device wherein the release (e.g., rate, timing of release) of a drug or other desired substance contained therein is controlled by or determined by the device itself and not substantially influenced by the environment of use, or releasing at a rate that is reproducible within the environment of use.

By "substantially continuous" as used in, for example, the context of "substantially continuous infusion" or "substantially continuous delivery" is meant to refer to delivery of drug in a manner that is substantially uninterrupted for a pre-selected period of drug delivery, where the quantity of drug received by the patient during any 8 hour interval in the pre-selected period never falls to zero. Furthermore, "substantially continuous" drug delivery can also encompass delivery of drug at a substantially constant, pre-selected rate or range of rates (*e.g*., amount of drug per unit time, or volume of drug formulation for a unit time) that is substantially uninterrupted for a pre-selected period of drug delivery.

A "specific pirfenidone analog," and all grammatical variants thereof, refers to, and is limited to, each and every pirfenidone analog shown in Table 1.

The term "chemotherapeutic agent" or "chemotherapeutic" (or "chemotherapy", in the case of treatment with a chemotherapeutic agent) is meant to encompass any non-proteinaceous (i.e., non-peptidic) chemical compound useful in the treatment of cancer. Examples of chemotherapeutic agents include alkylating agents such as thiotepa and cyclophosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); a camptothecin (including synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, foremustine, lomustine, nimustine, ranimustine; antibiotics such as the enediyne antibiotics (e.g. calicheamicin, especially calicheamicin gamma1I and calicheamicin phiI1, see, e.g., Agnew, Chem. Intl. Ed. Engl., 33:183-186 (1994); dynemicin, including dynemicin A; bisphosphonates, such as clodronate; an esperamicin; as well as neocarzinostatin chromophore and related chromoprotein enediyne antibiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubincin (Adramycin™) (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as demopterin, methotrexate, pteropterin, trimetrexate; purine analogs such as fludarabine, 6-mercaptopurine, thiamiprine, thioguanine; pyrimidine analogues such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replinisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; losoxantrone; podophyllinic acid; 2-ethylhydrazide; procarbazine; PSK®; razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethane; vindesine; dacarbazine; mannomustine; mitobronitol; mitolactol; pipobroman; gacytosine; arabinoside ("Ara-C"); cyclophosphamide; thiopeta; taxoids, e.g. paclitaxel (TAXOL®, Bristol Meyers Squibb Oncology, Princeton, NJ) and docetaxel (TAXOTERE®, Rhone-Poulenc Rorer, Antony, France); chlorambucil; gemcitabine (Gemzar™); 6-thioguanine; mercaptopurine; methotrexate; platinum analogs such as cisplatin and carboplatin; vinblastine; platinum; etoposide (VP-16); ifosfamide; mitroxantrone; vancristine; vinorelbine (Navelbine™); novantrone; teniposide; edatrexate; daunomycin; arninopterin; xeoloda; ibandronate; CPT-11; topoisomerase inhibitor RFS 2000; difluromethylornithine (DMFO); retinoids such as retinoic acid; capecitabine; and pharmaceutically acceptable salts, acids or derivatives of any of the above. Also included in the definition of "chemotherapeutic agent" are anti-hormonal agents that act to regulate or inhibit hormone action on tumors such as anti-estrogens and selective estrogen receptor modulators (SERMs), including, for example, tamoxifen (including Nolvadex™), raloxifene, droloxifene, 4-hydroxytamoxifen, trioxifene, keoxifene, LY117018, onapristone, and toremifene (Fareston™); inhibitors of the enzyme aromatase, which regulates estrogen production in the adrenal glands, such as, for example, 4(5)-imidazoles, aminoglutethimide, megestrol acetate (Megace™), exemestane, formestane, fadrozole, vorozole (Rivisor™), letrozole (Femara™), and anastrozole (Arimidex™); and anti-androgens such as flutamide, nilutamide, bicalutamide, leuprolide, and goserelin; and pharmaceutically acceptable salts, acids or derivatives of any of the above.

The term "antineoplastic" agent, drug or compound is meant to refer to any agent, including any chemotherapeutic agent, biological response modifier (including without limitation (i) proteinaceous, i.e. peptidic, molecules capable of elaborating or altering biological responses and (ii) non-proteinaceous, i.e. non-peptidic, molecules capable of elaborating or altering biological responses), cytotoxic agent, or cytostatic agent, that reduces proliferation of a neoplastic cell.

The term "biological response modifier" refers to any proteinaceous (i.e., peptidic) molecule or any non-proteinaceous (i.e., non-peptidic) molecule capable of elaborating or altering a biological response relevant to the treatment of cancer. Examples of biological response modifiers include antagonists of tumor-associated antigens, such as anti-tumor antigen antibodies, antagonists of cellular receptors capable of inducing cell proliferation, agonists of cellular receptors capable of inducing apoptosis, such as Apo-2 ligands, Type I interferon receptor agonists, such as interferon-α molecules and interferon-β molecules, Type II interferon receptor agonists, such as interferon-γ molecules, Type III interferon receptor agonists, such as IL-28A, IL-28B, and IL-29, antagonists of inflammatory cytokines, including tumor necrosis factor (TNF) antagonists, such as anti-TNF antibodies (e.g. REMICADE™anti-TNF monoclonal antibody) and soluble TNF receptor (e.g. ENBREL™TNF receptor-Ig immunoadhesin), growth factor cytokines, such as hematopoietic cytokines, including erythropoietins, such as EPOGEN™ epoetin-alfa, granulocyte colony stimulating factors (G-CSFs), such as NEUPOGEN™ filgrastim, granulocyte-macrophage colony stimulating factors (GM-CSFs), and thrombopoietins, lymphocyte growth factor cytokines, such as interleukin-2, and antagonists of growth factor cytokines, including antagonists of angiogenic factors, e.g. vascular endothelial cell growth factor (VEGF) antagonists, such as AVASTIN™ bevacizumab (anti-VEGF monoclonal antibody).

As used herein, the term "a Type I interferon receptor agonist" refers to any naturally occurring or non-naturally occurring ligand of human Type I interferon receptor, which binds to and causes signal transduction via the receptor. Type I interferon receptor agonists include interferons, including naturally-occurring interferons, modified interferons, synthetic interferons, pegylated interferons, fusion proteins comprising an interferon and a heterologous protein, shuffled interferons; antibody specific for an interferon receptor; non-peptide chemical agonists; and the like.

As used herein, the term "a Type II interferon receptor agonist" refers to any naturally-occurring or non-naturally-occurring ligand of a human Type II interferon receptor which binds to and causes signal transduction via the receptor. Type II interferon receptor agonists include interferons, including naturally-occurring interferons, modified interferons, synthetic interferons, pegylated interferons, fusion proteins comprising an interferon and a heterologous protein, shuffled interferons; antibody specific for an interferon receptor; non-peptide chemical agonists; and the like.

As used herein, the term "a Type III interferon receptor agonist" refers to any naturally occurring or non-naturally occurring ligand of humanIL-28 receptor α ("IL-28R"), the amino acid sequence of which is described by Sheppard, et al., infra., that binds to and causes signal transduction via the receptor.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a synthetic CXCR3 ligand" includes a plurality of such ligands and reference to "the formulation" includes reference to one or more formulations and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION OF THE INVENTION

The present invention provides synthetic CXCR3 polypeptide ligands, e.g., hybrid CXCR3 polypeptide ligands and consensus CXCR3 polypeptide ligands, as well as compositions and formulations comprising the synthetic CXCR3 polypeptide ligands. A subject synthetic CXCR3 ligand is useful for treating a variety of disorders, as discussed below.

### SYNTHETIC CXCR3 LIGANDS

The present invention provides synthetic CXCR3 polypeptide ligands. In some embodiments, a synthetic CXCR3 ligand is a consensus CXCR3 ligand. In other embodiments, a synthetic CXCR3 ligand is a hybrid CXCR3 ligand. Thus, as used herein, the term "synthetic CXCR3 ligand" includes "consensus CXCR3 ligand" and "hybrid CXCR3 ligand." The present invention further provides compositions comprising a subject synthetic CXCR3 ligand, including pharmaceutical compositions. A subject synthetic CXCR3 ligand is useful in the treatment of various disorders, as described herein; and in experimental applications, as described more fully below.

A subject synthetic CXCR3 ligand, including a consensus CXCR3 ligand and hybrid CXCR3 ligand, has a length of from about 70 amino acids to about 125 amino acids, e.g., from about 70 amino acids to about 73 amino acids, from about 73 amino acids to about 75 amino acids, from about 75 amino acids to about 77 amino acids, from about 77 amino acids to about 80 amino acids, from about 80 amino acids to about 90 amino acids, from about 90 amino acids to about 100 amino acids, from about 100 amino acids to about 102 amino acids, from about 102 amino acids to about 105 amino acids, from about 105 amino acids to about 110 amino acids, from about 110 amino acids to about 115 amino acids, from about 115 amino acids to about 120 amino acids, or from about 120 amino acids to about 125 amino acids. In some embodiments, e.g., where a subject CXCR3 ligand is a fusion protein, a subject synthetic CXCR3 ligand comprises more than 125 amino acids.

In some embodiments, a subject synthetic CXCR3 ligand includes a signal sequence. In other embodiments, a subject synthetic CXCR3 ligand lacks a signal sequence.

A subject synthetic CXCR3 ligand, including a consensus CXCR3 ligand and hybrid CXCR3 ligand, binds a CXCR3 receptor on a cell surface. Typically, a subject synthetic CXCR3 ligand is a CXCR3 agonist. In some embodiments, a subject hybrid CXCR3 ligand has one or more of the following activities: antiproliferative activity, anti-bacterial activity, anti-angiogenic activity, and anti-fibrotic activity. Whether a subject synthetic CXCR3 ligand functions as a CXCR3 agonist is readily determined using known assays. For example, assays for CXCR3 agonist activity are discussed in U.S. Patent No. 6,184,358; and U.S. Patent No. 6,491,906.

### Hybrid CXCR3 ligands

The present invention provides a hybrid CXCR3 ligand comprising a polypeptide of from about 70 to about 125 amino acids, optionally having an additional methionine attached to the ordinarily first amino acid at the N-terminus, the amino acid sequence of the polypeptide comprising, in sequence, discrete sub-sequences corresponding in amino acid identity and number to sub-sequences of different, naturally occurring CXCR3 ligands (e.g., IP-10, I-TAC, and Mig), where the amino acid sequence of the hybrid CXCR3 polypeptide differs from the amino acid sequence of naturally occurring CXCR3 ligands (e.g., TP-10, I-TAC, and Mig).

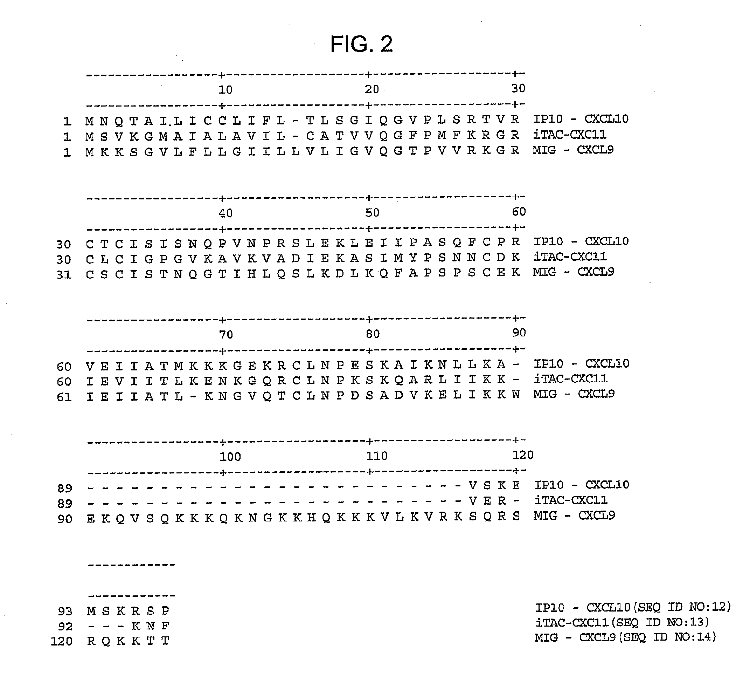

A subject hybrid CXCR3 ligand comprises two, three, four, five, six, seven, eight, nine, or ten, or more discrete sub-sequences corresponding in amino acid identity and number to sub-sequences of different, naturally occurring CXCR3 ligands (e.g., IP-10, I-TAC, and Mig). In some embodiments, a subject hybrid CXCR3 ligand comprises two, three, four, five, six, seven, eight, nine, or ten, or more discrete sub-sequences corresponding in amino acid identity and number to sub-sequences of naturally-occurring IP-10, TAC, and Mig. The discrete subsequence are from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 75 to about 80, from about 80 to about 85, or from about 85 to about 90 contiguous amino acids of a naturally-occurring CXCR3 ligand. In some embodiments, the discrete sub-sequences are chosen from the amino acid sequences depicted in Figure 2.

In some embodiments, a subject hybrid CXCR3 ligand comprises, in order from N-terminus to C-terminus, from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 75 to about 80, from about 80 to about 85, or from about 85 to about 90 contiguous amino acids of a first CXCR3-binding chemokine selected from naturally-occurring IP-10, I-TAC, and Mig; and from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 3S to about 40, from about 40 to about 45, from about 45 to about 50, from about 50 to about 55, from about 55 to about 60, from about 60 to about 65, from about 65 to about 70, from about 75 to about 80, from about 80 to about 85, or from about 85 to about 90 contiguous amino acids of a second CXCR3-binding chemokine selected from naturally-occurring IP-10, I-TAC, and Mig, where the first and second chemokines are different.

In some embodiments, a subject hybrid CXCR3 ligand further comprises from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 75 to about 80, from about 80 to about 85, or from about 85 to about 90 contiguous amino acids of a third CXCR3-binding chemokine selected from naturally-occurring IP-10, I-TAC, and Mig, where the third chemokine is different from the second chemokine, and in some embodiments is different from the first and second chemokines.

In some embodiments, a subject hybrid CXCR3 ligand further comprises from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 75 to about 80, from about 80 to about 85, or from about 85 to about 90 contiguous amino acids of a fourth CXCR3-binding chemokine selected from naturally-occurring IP-10, I-TAC, and Mig, where the fourth chemokine is different from the third chemokine.

In some embodiments, a subject hybrid CXCR3 ligand further comprises from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 75 to about 80, from about 80 to about 85, or from about 85 to about 90 contiguous amino acids of a fifth CXCR3-binding chemokine selected from naturally-occurring IP-10, I-TAC, and Mig, where the fifth chemokine is different from the fourth chemokine.

The following are non-limiting examples.

Example 1. In some embodiments, a subject hybrid CXCR3 ligand comprises, in order from N-terminus to C-terminus, from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of IP-10; and from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of I-TAC.

Example 2. In some embodiments, a subject hybrid CXCR3 ligand comprises, in order from N-terminus to C-terminus, from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of I-TAC; and from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of IP-10.

Example 3. In some embodiments, a subject hybrid CXCR3 ligand comprises, in order from N-terminus to C-terminus, from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of IP-10; and from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 70 to about 75, from about 75 to about 80, or from about 80 to about 90 contiguous amino acids of Mig.

Example 4. In some embodiments, a subject hybrid CXCR3 ligand comprises, in order from N-terminus to C-terminus, from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 70 to about 75, from about 75 to about 80, or from about 80 to about 90 contiguous amino acids of Mig; and from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of IP-10.

Example 5. In some embodiments, a subject hybrid CXCR3 ligand comprises, in order from N-terminus to C-terminus, from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 70 to about 75, from about 75 to about 80, or from about 80 to about 90 contiguous amino acids of Mig; and from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of I-TAC.

Example 6. In some embodiments, a subject hybrid CXCR3 ligand comprises, in order from N-terminus to C-terminus, from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of I-TAC; and from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 70 to about 75, from about 75 to about 80, or from about 80 to about 90 contiguous amino acids of Mig.

In some embodiments, a polypeptide of one of Examples 1-6 further comprises an additional N-terminal methionine residue.

In some embodiments, a polypeptide of one of Examples 1-6 further comprises, at the N-terminus or at the C-terminus, from about 2 to about 70, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, or from about 65 to about 70 contiguous amino acids of I-TAC or IP-10, or from about 2 to about 90, e.g., from about 2 to about 5, from about 5 to about 7, from about 7 to about 10, from about 10 to about 15, from about 15 to about 20, from about 20 to about 25, from about 25 to about 30, from about 30 to about 35, from about 35 to about 40, from about 40 to about 45, from about 45 to about 50, from about 50 to about 55, from about 55 to about 60, from about 60 to about 65, from about 65 to about 70, from about 70 to about 75, from about 75 to about 80, or from about 80 to about 90 contiguous amino acids of Mig. In some of these embodiments, the polypeptide further comprises an additional N-terminal methionine.

The following examples of hybrid CXCR3 ligands are provided for illustration only, and are not meant to be limiting.

In one exemplary embodiment, a hybrid CXCR3 ligand comprises, in order from N-terminus to C-terminus, amino acids 1-25 of IP-10; and amino acids 36-73 of I-TAC. In some embodiments, this polypeptide further comprises an additional N-terminal methionine residue.

In another exemplary embodiment, a hybrid CXCR3 ligand comprises, in order from N-terminus to C-terminus, amino acids 1-35 of I-TAC; and amino acids 36-77 of IP-10. In some embodiments, this polypeptide further comprises an additional N-terminal methionine residue.

In another exemplary embodiment, a hybrid CXCR3 ligand comprises, in order from N-terminus to C-terminus, amino acids 1-35 of IP-10; and amino acids 36-102 of Mig. In some embodiments, this polypeptide further comprises an additional N-terminal methionine residue.

In another exemplary embodiment, a hybrid CXCR3 ligand comprises, in order from N-terminus to C-terminus, amino acids 1-35 of Mig; and amino acids 36-77 of IP-10. In some embodiments, this polypeptide further comprises an additional N-terminal methionine residue.

In another exemplary embodiment, a hybrid CXCR3 ligand comprises, in order from N-terminus to C-terminus, amino acids 1-35 of I-TAC; and amino acids 36-102 of Mig. In some embodiments, this polypeptide further comprises an additional N-terminal methionine residue.

In another exemplary embodiment, a hybrid CXCR3 ligand comprises, in order from N-terminus to C-terminus, amino acids 1-35 of Mig; and amino acids 36-73 of I-TAC. In some embodiments, this polypeptide further comprises an additional N-terminal methionine residue.

In another exemplary embodiment, a hybrid CXCR3 ligand comprises, in order from N-terminus to C-terminus, amino acids 1-20 of IP-10; amino acids 21-60 of I-TAC; and amino acids 61-102 of Mig. In some embodiments, this polypeptide further comprises an additional N-terminal methionine residue.

In another exemplary embodiment, a hybrid CXCR3 ligand comprises, in order from N-terminus to C-terminus, amino acids 1-20 of I-TAC; amino acids 21-60 of IP-10; and amino acids 61-102 of Mig. In some embodiments, this polypeptide further comprises an additional N-terminal methionine residue.

In another exemplary embodiment, a hybrid CXCR3 ligand comprises, in order from N-terminus to C-terminus, amino acids 1-20 of IP-10; amino acids 21-60 of Mig; and amino acids 61-73 of I-TAC. In some embodiments, this polypeptide further comprises an additional N-terminal methionine residue.

In another exemplary-embodiment, a hybrid CXCR3 ligand comprises, in order from N-terminus to C-terminus, amino acids 1-20 of Mig; amino acids 21-60 of IP-10; and amino acids 61-73 of I-TAC. In some embodiments, this polypeptide further comprises an additional N-terminal methionine residue.

In another exemplary embodiment, a hybrid CXCR3 ligand comprises, in order from N-terminus to C-terminus, amino acids 1-20 of Mig; amino acids 21-60 of I-TAC; and amino acids 61-77 of IP-10. In some embodiments, this polypeptide further comprises an additional N-terminal methionine residue.

In another exemplary embodiment, a hybrid CXCR3 ligand comprises, in order from N-terminus to C-terminus, amino acids 1-20 of I-TAC; amino acids 21-60 of Mig; and amino acids 61-77 of IP-10. In some embodiments, this polypeptide further comprises an additional N-terminal methionine residue.

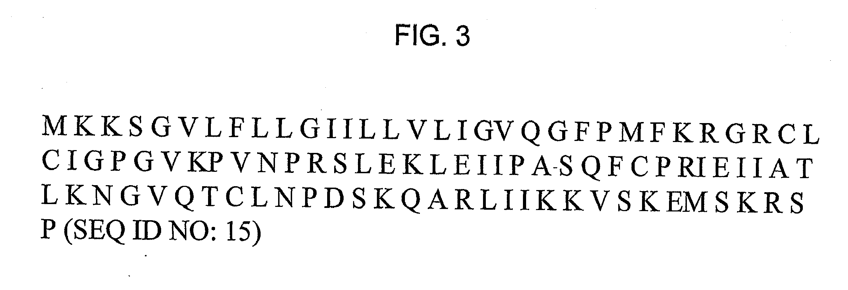

In a particular embodiment, a subject hybrid CXCR3 ligand comprises the amino acid sequence depicted in Figure 3, and set forth in SEQ ID NO:15. In this embodiment, a subject CXCR3 ligand comprises, in order from N-terminus to C-terminus, amino acids 1-20 of MIG; amino acids 21-38 of iTAC, amino acids 39-59 of IP10, amino acids 61-79 of MIG, amino acids 80-88 of iTAC, and amino acids 89-98 of IP10. In some embodiments, this polypeptide further comprises an additional N-terminal methionine residue.

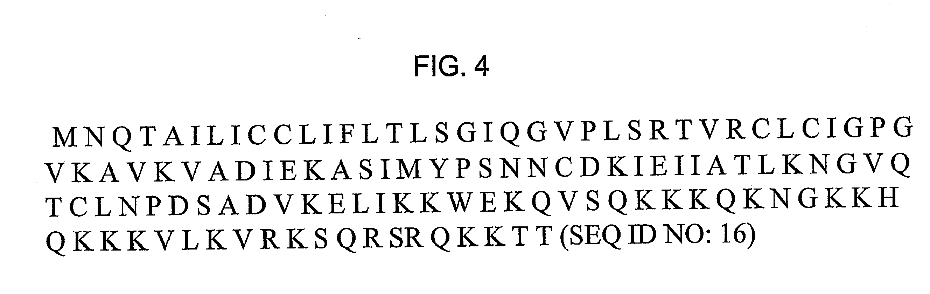

In another particular embodiment, a subject hybrid CXCR3 ligand comprises the amino acid sequence depicted in Figure 4, and set forth in SEQ ID NO:16. In this embodiment, a subject CXCR3 ligand comprises, in order from N-terminus to C-terminus, amino acids 1-30 of IP10; amino acids 32-61 of iTAC; and amino acids 63-125 of MIG. In some embodiments, this polypeptide further comprises an additional N-terminal methionine residue.

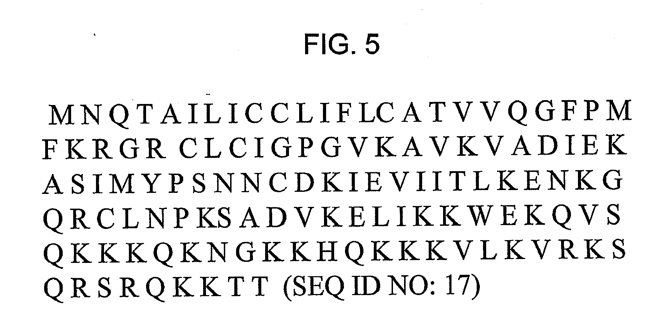

In another particular embodiment, a subject hybrid CXCR3 ligand comprises the amino acid sequence depicted in Figure 5, and set forth in SEQ ID NO:17. In this embodiment, a subject CXCR3 ligand comprises, in order from N-terminus to C-terminus, amino acids 1-14 of IP10; amino acids 15-79 of iTAC; and amino acids 80-125 of MIG. In some embodiments, this polypeptide further comprises an additional N-terminal methionine residue.

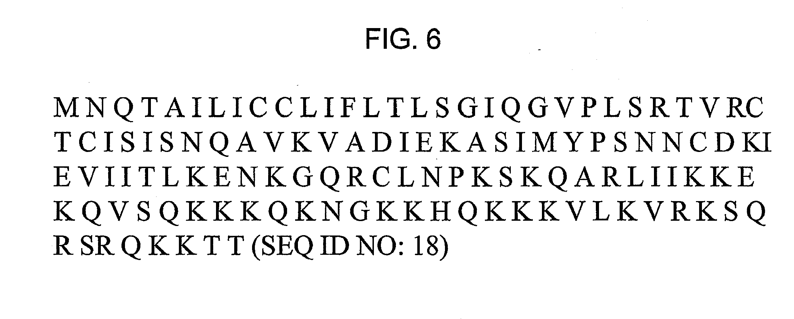

In another particular embodiment, a subject hybrid CXCR3 ligand comprises the amino acid sequence depicted in Figure 6, and set forth in SEQ ID NO:18. In this embodiment, a subject CXCR3 ligand comprises, in order from N-terminus to C-terminus, amino acids 1-38 of IP10; amino acids 39-88 of iTAC; and amino acids 90-125 of MIG. In some embodiments, this polypeptide further comprises an additional N-terminal methionine residue.

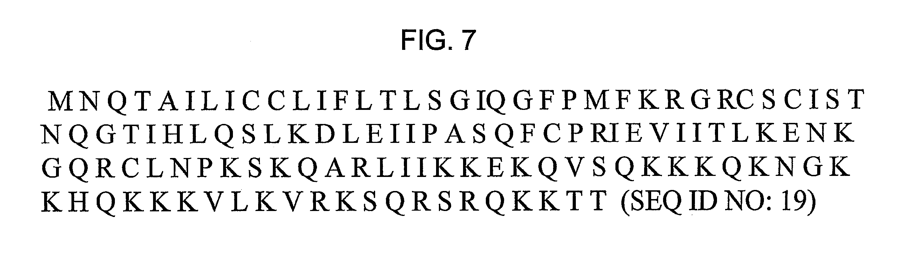

In another particular embodiment, a subject hybrid CXCR3 ligand comprises the amino acid sequence depicted in Figure 7, and set forth in SEQ ID NO:19. In this embodiment, a subject CXCR3 ligand comprises, in order from N-terminus to C-terminus, amino acids 1-21 of IP10; amino aacids 22-30 of iTAC; amino acids 32-49 of MIG; amino acids 49-59 of IP10; amino acids 60-88 of iTAC; and amino acids 90-125 of MIG. In some embodiments, this polypeptide further comprises an additional N-terminal methionine residue.

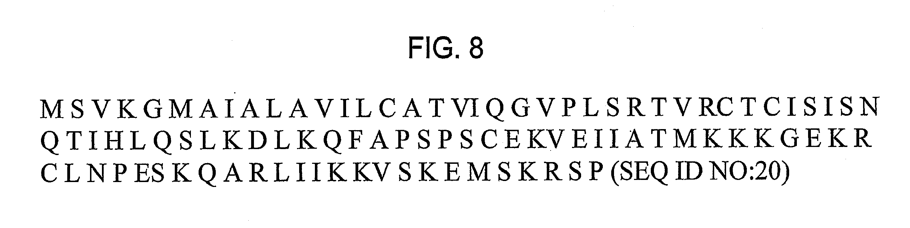

In another particular embodiment, a subject hybrid CXCR3 ligand comprises the amino acid sequence depicted in Figure 8, and set forth in SEQ ID NO:20. In this embodiment, a subject CXCR3 ligand comprises, in order from N-terminus to C-terminus, amino acids 1-18 of iTAC; amino acids 19-38 of IP10; amino acids 40-60 of MIG; amino acids 60-80 of IP10; amino acids 81-88 of iTAC; and amino acids 89-98 of IP10. In some embodiments, this polypeptide further comprises an additional N-terminal methionine residue.

### Consensus CXCR3 ligands

The present invention provides consensus CXCR3 ligand polypeptides, and compositions comprising the polypeptides. As used herein, the term "consensus CXCR3 ligand" refers to a non-naturally-occurring polypeptide, typically from about 70 amino acids to about 125 amino acids in length, optionally having an additional methionine attached to the ordinarily first amino acid at the N-terminus, which predominantly includes those amino acid residues that are common to IP-10, Mig, and I-TAC, and which includes, at one or more of those positions where there is no amino acid common to IP-10, Mig, and I-TAC, an amino acid which predominantly occurs at that position and in no event includes any amino acid residue which is not extant in that position in at least one naturally-occurring IP-10, Mig, or I-TAC amino acid sequence.

In some embodiments, a consensus CXCR3 ligand predominantly includes those amino acid residues that are common to IP-10, Mig, and I-TAC, and which includes, at one or more of those positions where there is no amino acid common to IP-10, Mig, and I-TAC, but where there is an amino acid common to two of IP-10, Mig, and I-TAC, the residue that is common to two of IP-10, Mig, and I-TAC.

In some embodiments, a subject consensus CXCR3 ligand predominantly includes those amino acid residues that are common to IP-10, Mig, and I-TAC, and which includes, at one or more of those positions where there is no amino acid common to IP-10, Mig, and I-TAC, the residue found in IP-10 at that position in from 2 to 30, e.g., from about 2 to about 5, from about 5 to about 7, from about 7 to about 10, from about 10 to about 15, from about 15 to about 18, from about 18 to about 20, from about 20 to about 22, from about 22 to about 25, from about 25 to about 27, or from about 27 to about 30, consecutive positions at which there is no amino acid common to IP-10, Mig, and I-TAC.

In some embodiments, a subject consensus CXCR3 ligand predominantly includes those amino acid residues that are common to IP-10, Mig, and I-TAC, and which includes, at one or more of those positions where there is no amino acid common to IP-10, Mig, and I-TAC, the residue found in I-TAC at that position in from 2 to 30, e.g., from about 2 to about 5, from about 5 to about 7, from about 7 to about 10, from about 10 to about 15, from about 15 to about 18, from about 18 to about 20, from about 20 to about 22, from about 22 to about 25, from about 25 to about 27, or from about 27 to about 30, consecutive positions at which there is no amino acid common to IP-10, Mig, and I-TAC.

In some embodiments, a subject consensus CXCR3 ligand predominantly includes those amino acid residues that are common to IP-10, Mig, and I-TAC, and which includes, at one or more of those positions where there is no amino acid common to IP-10, Mig, and I-TAC, the residue found in Mig at that position in from 2 to 30, e.g., from about 2 to about 5, from about 5 to about 7, from about 7 to about 10, from about 10 to about 15, from about 15 to about 18, from about 18 to about 20, from about 20 to about 22, from about 22 to about 25, from about 25 to about 27, or from about 27 to about 30, consecutive positions at which there is no amino acid common to IP-10, Mig, and I-TAC.

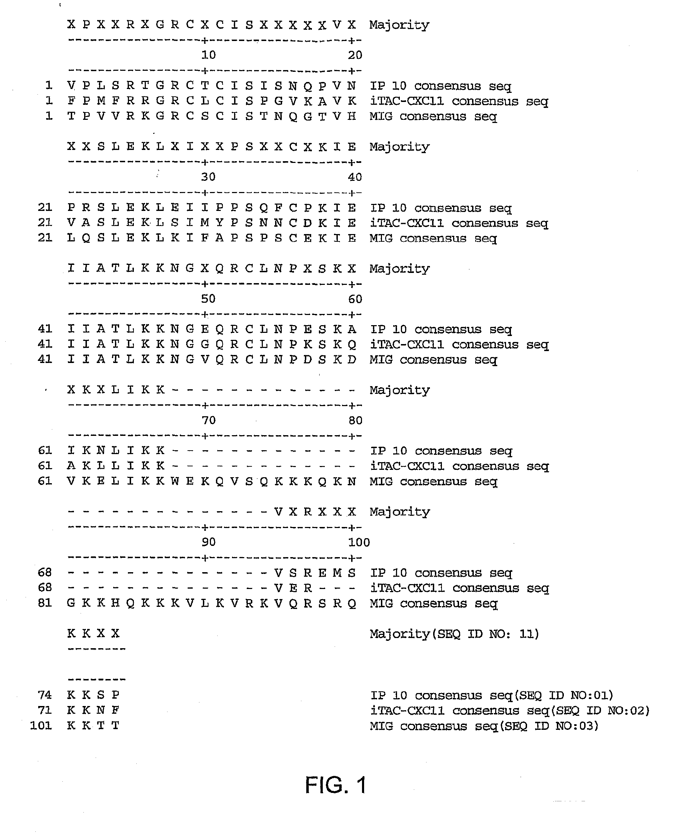

In some embodiments, a consensus CXCR3 ligand predominantly includes those amino acid residues that are common to IP-10, Mig, and I-TAC, and which includes, at one or more of those positions where there is no amino acid common to IP-10, Mig, and I-TAC, an amino acid that occurs in IP-10. In some of these embodiments, for every residue for which there is no amino acid common to IP-10, Mig, and I-TAC, the residue found in IP-10 is used in the consensus sequence. Where, for every residue for which there is no amino acid common to IP-10, Mig, and I-TAC, the residue found in IP-10 is used in the consensus sequence, the protein is referred to as "consensus 1." In some embodiments, a consensus 1 polypeptide comprises the amino acid sequence shown in Figure 1 and designated "IP-10 consensus sequence" (SEQ ID NO:01).

In some embodiments, a consensus CXCR3 ligand predominantly includes those amino acid residues that are common to IP-10, Mig, and I-TAC, and which includes, at one or more of those positions where there is no amino acid common to IP-10, Mig, and I-TAC, an amino acid that occurs in I-TAC. In some of these embodiments, for every residue for which there is no amino acid common to IP-10, Mig, and I-TAC, the residue found in I-TAC is used in the consensus sequence. Where, for every residue for which there is no amino acid common to IP-10, Mig, and I-TAC, the residue found in I-TAC is used in the consensus sequence, the protein is referred to as "consensus 2." In some embodiments, a consensus 2 polypeptide comprises the amino acid sequence shown in Figure 1 and designated "I-TAC consensus sequence" (SEQ ID NO:02).

In some embodiments, a consensus CXCR3 ligand predominantly includes those amino acid residues that are common to IP-10, Mig, and I-TAC, and which includes, at one or more of those positions where there is no amino acid common to IP-10, Mig, and I-TAC, an amino acid that occurs in Mig. In some of these embodiments, for every residue for which there is no amino acid common to IP-10, Mig, and I-TAC, the residue found in Mig is used in the consensus sequence. In these embodiments, where a gap exists in the "majority" sequence, the amino acid found in Mig is used. Where, for every residue for which there is no amino acid common to IP-10, Mig, and I-TAC, the residue found in Mig is used in the consensus sequence, the protein is referred to as "consensus 3." In some embodiments, a consensus 3 polypeptide comprises the amino acid sequence shown in Figure 1 and designated "Mig consensus sequence" (SEQ ID NO:03).

The amino acid sequence of human IP-10 is found in GenBank Accession Nos. P02778, NP_001556, and 1312356A. Amino acids 1-21 are a signal sequence, and mature IP-10 is amino acids 22-98 of a sequence found under GenBank Accession Nos. P02778, NP_001556, and 1312356A.

The amino acid sequence of human Mig is found under GenBank Accession Nos. NP_002407 and Q07325. Amino acids 1-22 are a signal sequence, and mature Mig is amino acids 23-125 of a sequence found under GenBank Accession Nos. NP_002407 and Q07325.

The amino acid sequence of human I-TAC is found under GenBank Accession Nos. Q14625 and AAD38867. Amino acids 1-21 are a signal sequence, and mature I-TAC is amino acids 22-94.

In particular embodiments, a subject consensus CXCR3 ligand has one of the amino acid sequences as set forth in Figure 1.

### Modifications

In some embodiments, a subject synthetic CXCR3 ligand (e.g., a hybrid CXCR3 ligand; a consensus CXCR3 ligand) includes one or more modifications. Modifications of interest that may or may not alter the primary amino acid sequence include chemical derivatization of polypeptides, e.g., acetylation, or carboxylation; changes in amino acid sequence that introduce or remove a glycosylation site; changes in amino acid sequence that make the protein susceptible to PEGylation (addition of a polyethylene glycol moiety); and the like. In one embodiment, the invention contemplates the use of synthetic CXCR3 ligand variants with one or more non-naturally occurring glycosylation and/or pegylation sites that are engineered to provide glycosyl- and/or PEG-derivatized polypeptides with reduced serum clearance. Thus, the invention includes PEGylated synthetic CXCR3 ligands. Also included are modifications of glycosylation, e.g., those made by modifying the glycosylation patterns of a polypeptide during its synthesis and processing or in further processing steps; e.g., by exposing the polypeptide to enzymes that affect glycosylation, such as mammalian glycosylating or deglycosylating enzymes. Also embraced are sequences that have phosphorylated amino acid residues, e.g., phosphotyrosine, phosphoserine, or phosphothreonine. In some embodiments, e.g., where the host cell used for production of a subject synthetic CXCR3 ligands is a bacterial host cell, the synthetic CXCR3 ligand is modified to comprise an N-terminal methionine.

In some embodiments, a subject synthetic CXCR3 ligand polypeptide is a fusion protein comprising a synthetic CXCR3 ligand polypeptide and a heterologous polypeptide (e.g., a fusion partner). Suitable fusion partners include peptides and polypeptides that confer enhanced stability *in vivo* (e.g., enhanced serum half-life); provide ease of purification, e.g., (His)ₙ, e.g., 6His, and the like; provide for secretion of the fusion protein from a cell; provide an epitope tag, e.g., GST, hemagglutinin (HA; e.g., CYPYDVPDYA; SEQ ID NO:4), FLAG (e.g., DYKDDDDK; SEQ ID NO:5), c-myc (e.g., CEQKLISEEDL; SEQ ID NO:6), and the like; provide a detectable signal, e.g., an enzyme that generates a detectable product (e.g., (β-galactosidase, luciferase), or a protein that is itself detectable, e.g., a green fluorescent protein, etc.; provides for multimerization, e.g., a multimerization domain such as an Fc portion of an immunoglobulin; and the like.

A fusion protein may comprise an amino acid sequence that provides for secretion of the fusion protein from the cell. Those skilled in the art are aware of such secretion signal sequences. Secretion signals that are suitable for use in bacteria include, but are not limited to, the secretion signal of Braun's lipoprotein of *E. coli*, *S. marcescens*, *E. amylosora*, *M. morganii*, and *P. mirabilis*, the TraT protein of *E. coli* and Salmonella; the penicillinase (PenP) protein of *B. licheniformis* and *B. cereus* and *S. aureus*; pullulanase proteins of *Klebsiella pneumoniae* and *Klebsiella aerogenese*; *E. coli* lipoproteins 1pp-28, Pal, Rp1A, Rp1B, OsmB, NIpB, and Orl17; chitobiase protein of *V. harseyi*; the β-1,4-endoglucanase protein of *Pseudomonas solanacearum*, the Pal and Pcp proteins of *H. influenzae*; the OprI protein of *P*. *aeruginosa*; the MalX and AmiA proteins of *S*. *pneumonia*; the 34 kda antigen and TpmA protein of *Treponema pallidum*; the P37 protein of *Mycoplasma hyorhinis*; the neutral protease of *Bacillus amyloliquefaciens*; and the 17 kda antigen of *Rickettsia rickettsii.* Secretion signal sequences suitable for use in yeast are known in the art, and can be used. See, e.g., U.S. Patent No. 5,712,113.

In some embodiments, a subject synthetic CXCR3 ligand is a fusion polypeptide comprising a subject synthetic CXCR3 polypeptide; and a fusion partner, wherein a protease cleavage site is positioned between the synthetic CXCR3 polypeptide and the fusion partner.

Proteolytic cleavage sites are known to those skilled in the art; a wide variety are known and have been described amply in the literature, including, e.g., Handbook of Proteolytic Enzymes (1998) AJ Barrett, ND Rawlings, and JF Woessner, eds., Academic Press. Proteolytic cleavage sites include, but are not limited to, an enterokinase cleavage site: (Asp)₄Lys (SEQ ID NO:07); a factor Xa cleavage site: Ile-Glu-Gly-Arg (SEQ ID NO:08); a thrombin cleavage site, e.g., Leu-Val-Pro-Arg-Gly-Ser (SEQ ID NO:09); a renin cleavage site, e.g., His-Pro-Phe-His-Leu-Val-Ile-His (SEQ ID NO:10); a collagenase cleavage site, e.g., X-Gly-Pro (where X is any amino acid); a trypsin cleavage site, e.g., Arg-Lys; a viral protease cleavage site, such as a viral 2A or 3C protease cleavage site, including, but not limited to, a protease 2A cleavage site from a picornavirus (see, e.g., Sommergruber et al. (1994) Virol. 198:741-745), a Hepatitis A virus 3C cleavage site (see, e.g., Schultheiss et al. (1995) J. Virol. 69:1727-1733), human rhinovirus 2A protease cleavage site (see, e.g., Wang et al. (1997) Biochem. Biophys. Res. Comm. 235:562-566), and a picornavirus 3 protease cleavage site (see, e.g., Walker et al. (1994) Biotechnol. 12:601-605.

### Preparation of a subject synthetic CXCR3 ligand

A subject synthetic CXCR3 ligand is conveniently prepared using any known method, including chemical synthesis methods, production by standard recombinant techniques, and combinations thereof. For example, a subject synthetic CXCR3 ligand can be synthesized using an automated solid-phase tert-butyloxycarbonyl and benzyl protection strategy. A subject synthetic CXCR3 ligand can be synthesized by native chemical ligation, e.g., fragments of from about 15 to about 40 amino acids in length (e.g., fragments of from about 15 to about 20, from about 20 to about 25, from about 25 to about 30, from about 30 to about 35, or from about 35 to about 40 amino acids in length) can be synthesized using standard methods of chemical synthesis, and the fragments ligated, using a process as described in Dawson, et al. (1994) Science 266:776-779. The purity of synthesized polypeptides may be assessed by reverse-phase HPLC and isoelectric focusing. The primary structures of the ligands may be verified by Edman sequencing methods.

In many embodiments, an expression vector comprising a nucleotide sequence that encodes a subject synthetic CXCR3 ligand is prepared, using conventional methods, and is introduced into a host cell. The expression vector provides for production of the subject synthetic CXCR3 ligand in the host cell.

Thus, the present invention provides a method for producing a synthetic CXCR3 ligand, the method comprising culturing a host cell, which host cell comprises an expression vector that includes a nucleotide sequence that encodes a subject CXCR3 ligand, under conditions that favor production of the synthetic CXCR3 ligand by the host cell; and isolating the synthetic CXCR3 ligand from the culture (e.g., from a host cell lysate and/or from the culture medium). The method may be carried out using a eukaryotic cell or a prokaryotic cell.

The polypeptides may be expressed in prokaryotes or eukaryotes in accordance with conventional ways, depending upon the purpose for expression. For large scale production of the protein, a unicellular organism, such as *E. coli*, *B. subtilis*, *S. cerevisiae*, insect cells in combination with baculovirus vectors, or cells of a higher organism such as vertebrates, particularly mammals, e.g. COS 7 cells, CHO cells, HEK293 cells, and the like, may be used as the expression host cells. In some situations, it is desirable to express the gene in eukaryotic cells, where the protein will benefit from native folding and post-translational modifications. The polypeptide can then be isolated from cell culture supernatant or from cell lysates using affinity chromatography methods or anion exchange/size exclusion chromatography methods, as described above.

With the availability of the protein or fragments thereof in large amounts, by employing an expression host, the protein may be isolated and purified in accordance with conventional ways. A lysate may be prepared of the expression host and the lysate purified using high performance liquid chromatography (HPLC), exclusion chromatography, gel electrophoresis, affinity chromatography, or other purification technique.

A subject synthetic CXCR3 ligand may also be isolated and purified in accordance with conventional methods of recombinant synthesis. A lysate may be prepared of the expression host and the lysate purified using HPLC, exclusion chromatography, gel electrophoresis, affinity chromatography, or other purification technique. For the most part, the compositions which are used will comprise at least 20% by weight of the desired product, more usually at least about 75% by weight, preferably at least about 95% by weight, and for therapeutic purposes, usually at least about 99.5% by weight, in relation to contaminants related to the method of preparation of the product and its purification. Usually, the percentages will be based upon total protein.

The present invention provides compositions comprising a subject CXCR3 ligand. A CXCR3 ligand will in many embodiments be pure, e.g., at least about 90% pure (free from non-CXCR3 polypeptides and/or other macromolecules), at least about 95% pure, at least about 98% pure, or at least about 99% pure, or greater than 99% pure.

A subject CXCR3 ligand composition comprises, in addition to a CXCR3 ligand, one or more of a buffer, a salt, a pH adjuster, a solubilizing agent, a chelating agent, a detergent, a non-ionic detergent, a protease inhibitor, an adjuvant, etc.

In some embodiments, a subject composition comprises a subject synthetic CXCR3 ligand; and pharmaceutically acceptable excipient(s). A wide variety of pharmaceutically acceptable excipients are known in the art and need not be discussed in detail herein. Pharmaceutically acceptable excipients have been amply described in a variety of publications, including, for example, A. Gennaro (2000) "Remington: The Science and Practice of Pharmacy", 20th edition, Lippincott, Williams, & Wilkins; Pharmaceutical Dosage Forms and Drug Delivery Systems (1999) H.C. Ansel et al., eds 7th ed., Lippincott, Williams, & Wilkins; and Handbook of Pharmaceutical Excipients (2000) A.H. Kibbe et al., eds., 3rd ed. Amer. Pharmaceutical Assoc.

### POLYNUCLEOTIDES, VECTORS, AND HOST CELLS

The present invention further provides a polynucleotide comprising a nucleotide sequence that encodes a subject synthetic CXCR3 ligand, vectors comprising a subject polynucleotide, and host cells comprising a subject polynucleotide or vector. A subject polynucleotide is useful for generating a subject expression vector and genetically modified host cells, which are useful for producing a subject ligand. The present invention further provides compositions comprising a subject polynucleotide.

The subject invention provides nucleic acid compositions encoding a subject synthetic CXCR3 ligand; as well as the complement of such nucleic acids. By nucleic acid composition is meant a composition comprising a sequence of a nucleic acid molecule having an open reading frame that encodes a synthetic CXCR3 ligand of the subject invention, and is capable, under appropriate conditions, of being expressed such that a synthetic CXCR3 ligand is produced. Also encompassed in this term are nucleic acids that are homologous or substantially similar or identical to the nucleic acids encoding a subject synthetic CXCR3 ligand.

Thus, the subject invention provides nucleic acids comprising a nucleotide sequence encoding a subject synthetic CXCR3 ligand, and nucleic acids having substantial nucleotide sequence identity to such nucleic acids (e.g., homologs). In many embodiments, a subject nucleic acid comprises a nucleotide sequence that encodes a subject synthetic CXCR3 ligand and that has at least about 75%, at least about 80%, at least about 85%, at least about 90%, at least about 95%, at least about 98%, or at least about 99%, or more, nucleotide sequence identity with a nucleotide sequence encoding a subject synthetic CXCR3 ligand (e.g., with the CXCR3 coding sequence), or the complementary sequence thereof.

Sequence similarity is calculated based on a reference sequence, which may be a subset of a larger sequence, such as a conserved motif, coding region, flanking region, *etc.* A reference sequence will usually be at least about 18 nt long, more usually at least about 30 nt long, and may extend to the complete sequence that is being compared. Algorithms for sequence analysis are known in the art, such as BLAST, described in Altschul et al. (1990), J. Mol. Biol. 215:403-10 (using default settings, i.e. parameters *w*=4 and *T*=17).

Also provided are nucleic acids that hybridize to the above-described nucleic acids under stringent conditions. An example of stringent hybridization conditions is hybridization at 50°C or higher and 0.1×SSC (15 mM sodium chloride/1.5 mM sodium citrate). Another example of stringent hybridization conditions is overnight incubation at 42°C in a solution: 50% formamide, 5 × SSC (150 mM NaCl, 15 mM trisodium citrate), 50 mM sodium phosphate (pH7.6), 5 × Denhardt's solution, 10% dextran sulfate, and 20 µg/ml denatured, sheared salmon sperm DNA, followed by washing the filters in 0.1 × SSC at about 65°C. Stringent hybridization conditions are hybridization conditions that are at least as stringent as the above representative conditions. Other stringent hybridization conditions are known in the art and may also be employed to identify nucleic acids of this particular embodiment of the invention.

As used herein, the term "stringent hybridization conditions" are those typically used by one of skill in the art to establish at least a 90% sequence identity between complementary pieces of DNA or DNA and RNA. In some embodiments, a nucleic acid molecule of the invention is capable of hybridizing to the CXCR3 coding sequence of a nucleic acid molecule comprising a nucleotide sequence that encodes a CXCR3 ligand as set forth in any one of SEQ ID NOs: 01, 02, 03, 15, 16, 17, 18, 19, and 20, or the complement of the coding region of such a nucleic acid sequence, under stringent hybridization conditions that comprise: prehybridization of filters containing nucleic acid for 8 hours to overnight at 65° C in a solution comprising 6X single strength citrate (SSC) (1X SSC is 0.15 M NaCl, 0.015 M Na citrate; pH 7.0), 5X Denhardt's solution, 0.05% sodium pyrophosphate and 100 µg/ml herring sperm DNA; hybridization for 18-20 hours at 65° C in a solution containing 6X SSC, 1X Denhardt's solution, 100 µg/ml yeast tRNA and 0.05% sodium pyrophosphate; and washing of filters at 65° C for 1 h in a solution containing 0.2X SSC and 0.1% SDS (sodium dodecyl sulfate).

In particular embodiments, a subject nucleic acid comprises a nucleotide sequence that encodes a CXCR3 ligand comprising an amino acid sequence as set forth in any of SEQ ID NOs:01, 02, 03, 15, 16, 17, 18, 19, and 20; or the complement of such a nucleic acid.

Nucleic acids encoding the proteins and polypeptides of the subject invention are in many embodiments DNA, including cDNA. The terms "synthetic CXCR3 ligand nucleic acid," "hybrid CXCR3 ligand nucleic acid," and "consensus CXCR3 ligand nucleic acid" as used herein, refer to the open reading frame encoding specific-subject synthetic CXCR3 proteins and polypeptides, hybrid CXCR3 proteins and polypeptides, consensus CXCR3 proteins and polypeptides, as well as adjacent 5' and 3' non-coding nucleotide sequences involved in the regulation of expression, e.g., from about 100 bp up to about 20 kb beyond the coding region, but possibly further in either direction. The nucleic acid may be introduced into an appropriate vector for extrachromosomal maintenance or for integration into a host genome, as described in greater detail below.

The nucleic acid compositions of the subject invention may encode all or a part of the subject synthetic CXCR3 ligands. Double or single stranded fragments may be obtained from the DNA sequence by chemically synthesizing oligonucleotides in accordance with conventional methods, by restriction enzyme digestion, by polymerase chain reaction (PCR) amplification, *etc*.

The subject nucleic acid molecules are generally propagated by placing the molecule in a vector. Viral and non-viral vectors are used, including plasmids. The choice of plasmid will depend on the type of cell in which propagation is desired and the purpose of propagation. Certain vectors are useful for amplifying and making large amounts of the desired DNA sequence.

The present invention further provides recombinant vectors ("constructs") comprising a subject polynucleotide. Recombinant vectors include vectors used for propagation of a polynucleotide of the invention, and expression vectors. Recombinant vectors are useful for propagation of the subject polynucleotides (cloning vectors). A subject recombinant expression vector is useful for effecting expression of a subject polynucleotide in a cell, e.g., for production of a subject synthetic CXCR3 ligand. The choice of appropriate vector is well within the skill of the art. Many such vectors are available commercially.

Expression vectors are suitable for expression in cells in culture. These vectors will generally include regulatory sequences ("control sequences" or "control regions") which are necessary to effect the expression of a subject polynucleotide to which they are operably linked. Still other vectors are suitable for transfer and expression in cells in a whole organism or person.

Expression vectors generally have convenient restriction sites located near the promoter sequence to provide for the insertion of nucleic acid sequences encoding heterologous proteins. A selectable marker operative in the expression host may be present. Expression vectors may be used for the production of fusion proteins, where the exogenous fusion peptide provides additional functionality, i.e. increased protein synthesis, stability, reactivity with defined antisera, an enzyme marker, e.g. β-galactosidase, luciferase, horse radish peroxidase, etc.

Expression cassettes may be prepared that comprise a transcription initiation region, a promoter region, a subject polynucleotide, and a transcriptional termination region. After introduction of the DNA, the cells containing the construct may be selected by means of a selectable marker, the cells expanded and then used for expression.

The expression cassettes may be introduced into a variety of vectors, *e.g.* plasmid, BAC, HAC, YAC, bacteriophage such as lambda, P1, M13, *etc*., animal or plant viruses, and the like, where the vectors are normally characterized by the ability to provide selection of cells comprising the expression vectors. The vectors may provide for extrachromosomal maintenance, particularly as plasmids or viruses, or for integration into the host chromosome. Where extrachromosomal maintenance is desired, an origin sequence is provided for the replication of the plasmid, which may be low- or high copy-number. A wide variety of markers are available for selection, particularly those which protect against toxins, more particularly against antibiotics. The particular marker that is chosen is selected in accordance with the nature of the host, where in some cases, complementation may be employed with auxotrophic hosts. Introduction of the DNA construct into a host cell may use any convenient method, *e.g*. conjugation, bacterial transformation, calcium-precipitated DNA, electroporation, fusion, transfection, infection with viral vectors, biolistics, *etc*.

The present invention further provides genetically modified host cells, which may be isolated host cells, comprising a subject polynucleotide, or, in some embodiments, a subject expression vector. Suitable host cells include prokaryotes such as *E. coli*, *B*. *subtilis*, eukaryotes, including insect cells in combination with baculovirus vectors, yeast cells, such as *Saccharomyces cerevisiae*, or cells of a higher organism such as vertebrates, including amphibians (e.g., *Xenopus laevis* oocytes), and mammals, particularly mammals, e.g. COS cells, CHO cells, HEK293 cells, MA-10 cells, and the like, may be used as the expression host cells. Host cells can be used for the purposes of propagating a subject polynucleotide, for production of a synthetic CXCR3 ligand polypeptide.

### ANTIBODY COMPOSITIONS

Also provided are antibodies that bind specifically to a subject synthetic CXCR3 ligand polypeptide. Suitable antibodies are obtained by immunizing a host animal with peptides comprising all or a portion of the subject protein. Suitable host animals include mouse, rat sheep, goat, hamster, rabbit, *etc.* In many embodiments, a subject antibody is isolated; and in many embodiments a subject antibody is purified.

Also provided are compositions comprising a subject antibody. A subject antibody composition comprises, in addition to a subject antibody, one or more of a buffer, a salt, a pH adjuster, a solubilizing agent, a chelating agent, a detergent, a non-ionic detergent, a protease inhibitor, etc.

The immunogen may comprise the complete protein, or fragments and derivatives thereof. Typical immunogens comprise all or a part of the protein, where these residues contain the post-translation modifications found on the native target protein. Immunogens are produced in a variety of ways known in the art, *e.g*., expression of cloned genes using conventional recombinant methods, chemical synthesis of synthetic CXCR3 ligand polypeptides, etc.

For preparation of polyclonal antibodies, the first step is immunization of the host animal with the target protein, where the target protein will preferably be in substantially pure form, comprising less than about 1% contaminant. The immunogen may comprise the complete target protein, fragments or derivatives thereof. To increase the immune response of the host animal, the target protein may be combined with an adjuvant, where suitable adjuvants include alum, dextran, sulfate, large polymeric anions, oil and water emulsions, e.g. Freund's adjuvant, Freund's complete adjuvant, and the like. The target protein may also be conjugated to synthetic carrier proteins or synthetic antigens. A variety of hosts may be immunized to produce the polyclonal antibodies. Such hosts include rabbits, guinea pigs, rodents, e.g. mice, rats, sheep, goats, and the like. The target protein is administered to the host, usually intradermally, with an initial dosage followed by one or more, usually at least two, additional booster dosages. Following immunization, the blood from the host will be collected, followed by separation of the serum from the blood cells. The Ig present in the resultant antiserum may be further fractionated using known methods, such as ammonium salt fractionation, DEAE chromatography, and the like.

Monoclonal antibodies are produced by conventional techniques. Generally, the spleen and/or lymph nodes of an immunized host animal provide a source of plasma cells. The plasma cells are immortalized by fusion with myeloma cells to produce hybridoma cells. Culture supernatant from individual hybridomas is screened using standard techniques to identify those producing antibodies with the desired specificity. Suitable animals for production of monoclonal antibodies to the human protein include mouse, rat, hamster, *etc.* To raise antibodies against the mouse protein, the animal will generally be a hamster, guinea pig, rabbit, etc. The antibody may be purified from the hybridoma cell supernatants or ascites fluid by conventional techniques, *e.g*. affinity chromatography using protein bound to an insoluble support, protein A sepharose, *etc.*

The antibody may be produced as a single chain, instead of the normal multimeric structure. Single chain antibodies are described in Jost et al. (1994) J. Biol. Chem. 269:26267-73, and others. DNA sequences encoding the variable region of the heavy chain and the variable region of the light chain are ligated to a spacer encoding at least about 4 amino acids of small neutral amino acids, including glycine and/or serine. The protein encoded by this fusion allows assembly of a functional variable region that retains the specificity and affinity of the original antibody.

Also of interest in certain embodiments are humanized antibodies. Methods of humanizing antibodies are known in the art. The humanized antibody may be the product of an animal having transgenic human immunoglobulin constant region genes (see for example International Patent Applications WO 90/10077 and WO 90/04036). Alternatively, the antibody of interest may be engineered by recombinant DNA techniques to substitute the CH1, CH2, CH3, hinge domains, and/or the framework domain with the corresponding human sequence (see WO 92/02190).

The use of Ig cDNA for construction of chimeric immunoglobulin genes is known in the art (Liu et al. (1987) Proc. Natl. Acad. Sci. USA 84:3439 and (1987) J. Immunol. 139:3521). mRNA is isolated from a hybridoma or other cell producing the antibody and used to produce cDNA. The cDNA of interest may be amplified by the polymerase chain reaction using specific primers (U.S. Patent nos. 4,683,195 and 4,683,202). Alternatively, a library is made and screened to isolate the sequence of interest. The DNA sequence encoding the variable region of the antibody is then fused to human constant region sequences. The sequences of human constant regions genes may be found in Kabat et al. (1991) Sequences of Proteins of Immunological Interest, N.I.H. publication no. 91-3242. Human C region genes are readily available from known clones. The choice of isotype will be guided by the desired effector functions, such as complement fixation, or activity in antibody-dependent cellular cytotoxicity. Preferred isotypes are IgG1, IgG3 and IgG4. Either of the human light chain constant regions, kappa or lambda, may be used. The chimeric, humanized antibody is then expressed by conventional methods.

Antibody fragments, such as Fv, F(ab')₂ and Fab may be prepared by cleavage of the intact protein, *e.g*. by protease or chemical cleavage. Alternatively, a truncated gene is designed. For example, a chimeric gene encoding a portion of the F(ab')₂ fragment would include DNA sequences encoding the CH1 domain and hinge region of the H chain, followed by a translational stop codon to yield the truncated molecule.

Consensus sequences of H and L J regions may be used to design oligonucleotides for use as primers to introduce useful restriction sites into the J region for subsequent linkage of V region segments to human C region segments. C region cDNA can be modified by site directed mutagenesis to place a restriction site at the analogous position in the human sequence.

Expression vectors include plasmids, retroviruses, YACs, EBV derived episodes, and the like. A convenient vector is one that encodes a functionally complete human CH or CL immunoglobulin sequence, with appropriate restriction sites engineered so that any VH or VL sequence can be easily inserted and expressed. In such vectors, splicing usually occurs between the splice donor site in the inserted J region and the splice acceptor site preceding the human C region, and also at the splice regions that occur within the human CH exons. Polyadenylation and transcription termination occur at native chromosomal sites downstream of the coding regions. The resulting chimeric antibody may be joined to any strong promoter, including retroviral LTRs, *e.g*. SV-40 early promoter, (Okayama et al. (1983) Mol. Cell. Bio. 3:280), Rous sarcoma virus LTR (Gorman et al. (1982) P.N.A.S. 79:6777), and moloney murine leukemia virus LTR (Grosschedl et al. (1985) Cell 41:885); native Ig promoters, *etc*.

### METHODS OF TREATING FIBROTIC DISORDERS

The present invention provides methods for treating a fibrotic disorder in an individual having a fibrotic disorder. The method generally involves administering an effective amount of a subject synthetic CXCR3 ligand. The methods provide for treatment of fibrotic diseases, including those affecting the lung such as idiopathic pulmonary fibrosis, pulmonary fibrosis from a known etiology, liver fibrosis or cirrhosis, cardiac and renal fibrosis. The etiology may be due to any acute or chronic insult including toxic, metabolic, genetic and infectious agents.

Fibrosis is generally characterized by the pathologic or excessive accumulation of collagenous connective tissue. Fibrotic disorders include, but are not limited to, collagen disease, interstitial lung disease, human fibrotic lung disease (e.g., obliterative bronchiolitis, idiopathic pulmonary fibrosis, pulmonary fibrosis from a known etiology, tumor stroma in lung disease, systemic sclerosis affecting the lungs, Hermansky-Pudlak syndrome, coal worker's pneumoconiosis, asbestosis, silicosis, chronic pulmonary hypertension, AIDS-associated pulmonary hypertension, sarcoidosis, and the like), fibrotic vascular disease, arterial sclerosis, atherosclerosis, varicose veins, coronary infarcts, cerebral infarcts, myocardial fibrosis, musculoskeletal fibrosis, post-surgical adhesions, human kidney disease (e.g., nephritic syndrome, Alport's syndrome, HIV-associated-nephropathy, polycystic kidney disease, Fabry's disease, diabetic nephropathy, chronic glomerulonephritis, nephritis associated with systemic lupus, and the like), cutis keloid formation, progressive systemic sclerosis (PSS), primary sclerosing cholangitis (PSC), liver fibrosis, liver cirrhosis, renal fibrosis, pulmonary fibrosis, cystic fibrosis, chronic graft versus host disease, scleroderma (local and systemic), Grave's opthalmopathy, diabetic retinopathy, glaucoma, Peyronie's disease, penis fibrosis, urethrostenosis after the test using a cystoscope, inner accretion after surgery, scarring, myelofibrosis, idiopathic retroperitoneal fibrosis, peritoneal fibrosis from a known etiology, drug-induced ergotism, fibrosis incident to benign or malignant cancer, fibrosis incident to microbial infection (e.g., viral, bacterial, parasitic, fungal, etc.), Alzheimer's disease, fibrosis incident to inflammatory bowel disease (including stricture formation in Crohn's disease and microscopic colitis), fibrosis induced by chemical or environmental insult (e.g., cancer chemotherapy, pesticides, radiation (e.g., cancer radiotherapy), and the like), and the like.

In some embodiments, an effective amount of a synthetic CXCR3 ligand is an amount that, when administered to an individual having a fibrotic disorder, is effective to reduce fibrosis or reduce the rate of progression of fibrosis by at least about 10%, at least about 15%, at least about 20%, at least about 25%, at least about 30%, at least about 35%, at least about 40%, at least about 45%, or at least about 50%, or more, compared with the degree of fibrosis in the individual prior to treatment or compared to the rate of progression of fibrosis that would have been experienced by the patient in the absence of treatment with the synthetic CXCR3 ligand.

In some embodiments, an effective amount of a synthetic CXCR3 ligand is an amount that, when administered to an individual having a fibrotic disorder, is effective to increase, or to reduce the rate of deterioration of, at least one function of the organ affected by fibrosis (e.g., lung, liver, kidney, etc.) by at least about 10%, at least about 15%, at least about 20%, at least about 25%, at least about 30%, at least about 35%, at least about 40%, at least about 45%, or at least about 50%, or more, compared to the basal level of organ function in the individual prior to treatment or compared to the rate of deterioration in organ function that would have been experienced by the individual in the absence of treatment with the synthetic CXCR3 ligand.

Methods of measuring the extent of fibrosis in a given organ, and methods of measuring the function of any given organ, are well known in the art.

### Combination therapies

In some embodiments, the present invention provides combination therapies for the treatment of a fibrotic disorder. Accordingly, the present invention provides a method of treating a fibrotic disorder, generally involving administering a subject synthetic CXCR3 ligand in combination therapy with a second therapeutic agent. Suitable second therapeutic agents include, but are not limited to, a Type I interferon receptor agonist, a Type III interferon receptor agonist, a Type II interferon receptor agonist, pirfenidone or a pirfenidone analog, a TNF antagonist, a TGF-β antagonist, an endothelin receptor antagonist, a stress-activated protein kinase inhibitor, etc.

In other embodiments, the present invention provides methods of treating a fibrotic disorder that involve administering a synergistic combination of a subject synthetic CXCR3 ligand and a second therapeutic agent. As used herein, a "synergistic combination" of a subject synthetic CXCR3 ligand and a second therapeutic agent is a combined dosage that is more effective in the therapeutic or prophylactic treatment of a fibrotic disorder than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of the second therapeutic agent when administered at the same dosage as a monotherapy.

In some embodiments, the present invention provides methods that involve administering a synergistic combination of a subject synthetic CXCR3 ligand and pirfenidone or specific pirfenidone analog. As used herein, a "synergistic combination" of a subject synthetic CXCR3 ligand and pirfenidone or specific pirfenidone analog is a combined dosage that is more effective in the therapeutic or prophylactic treatment of a fibrotic disorder than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of pirfenidone or a specific pirfenidone analog when administered at the same dosage as a monotherapy.

### METHODS OF TREATING IDIOPATHIC PULMOMARY FIBROSIS

The present invention provides methods of treating idiopathic pulmonary fibrosis (IPF). The methods generally involve administering to an individual having IPF an effective amount of a subject synthetic CXCR3 ligand.

In some embodiments; a diagnosis of IPF is confirmed by the finding of usual interstitial pneumonia (UIP) on histopathological evaluation of lung tissue obtained by surgical biopsy. The criteria for a diagnosis of IPF are known. Ryu et al. (1998) Mayo Clin. Proc. 73:1085-1101.

In other embodiments, a diagnosis of IPF is a definite or probable IPF made by high resolution computer tomography (HRCT). In a diagnosis by HRCT, the presence of the following characteristics is noted: (1) presence of reticular abnormality and/or traction bronchiectasis with basal and peripheral predominance; (2) presence of honeycombing with basal and peripheral predominance; and (3) absence of atypical features such as micronodules, peribronchovascular nodules, consolidation, isolated (non-honeycomb) cysts, ground glass attenuation (or, if present, is less extensive than reticular opacity), and mediastinal adenopathy (or, if present, is not extensive enough to be visible on chest x-ray). A diagnosis of definite IPF is made if characteristics (1), (2), and (3) are met. A diagnosis of probable IPF is made if characteristics (1) and (3) are met.

In some embodiments, an "effective amount" of a synthetic CXCR3 ligand is a dosage that is effective to decrease disease progression by at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, or more, compared with a placebo control or an untreated control.

Disease progression is the occurrence of one or more of the following: (1) a decrease in predicted FVC of 10% or more; (2) an increase in A-a gradient of 5 mm Hg or more; (3) a decrease of 15% of more in single breath DL_{co}. Whether disease progression has occurred is determined by measuring one or more of these parameters on two consecutive occasions 4 to 14 weeks apart, and comparing the value to baseline.

Thus, e.g., where an untreated or placebo-treated individual exhibits a 50% decrease in FVC over a period of time, an individual administered with an effective amount of a subject CXCR3 ligand exhibits a decrease in FVC of 45%, about 42%, about 40%, about 37%, about 35%, about 32%, about 30%, or less, over the same time period.

In some embodiments, an "effective amount" of a synthetic CXCR3 ligand is a dosage that is effective to increase progression-free survival time, e.g., the time from baseline (e.g., a time point from 1 day to 28 days before beginning of treatment) to death or disease progression is increased by at least about 10%, at least about 20%, at least about 25%, at least about 3 0%, at least about 40%, at least about 50%, at least about 60%, at least about 70%, at least about 80%, at least about 90%, at least about 2-fold, at least about 3-fold, at least about 4-fold, at least about 5-fold, or more, compared a-placebo-treated or an untreated control individual. Thus, e.g., in some embodiments an effective amount of a synthetic CXCR3 ligand is a dosage that is effective to increase the progression-free survival time by at least about 1 week, at least about 2 weeks, at least about 3 weeks, at least about 4 weeks, at least about 2 months, at least about 3 months, at least about 4 months, at least about 5 months, at least about 6 months, at least about 8 months, at least about 10 months, at least about 12 months, at least about 18 months, at least about 2 years, at least about 3 years, or longer, compared to a placebo-treated or untreated control.

In some embodiments, an effective amount of a synthetic CXCR3 ligand is a dosage that is effective to increase at least one parameter of lung function, e.g., an effective amount of a synthetic CXCR3 ligand increases at least one parameter of lung function by at least about 10%, at least about 20%, at least about 25%, at least about 30%, at least about 40%, at least about 50%, at least about 60%, at least about 70%, at least about 80%, at least about 90%, at least about 2-fold, at least about 3-fold, at least about 4-fold, at least about 5-fold, or more, compared to an untreated individual or a placebo-treated control individual. In some of these embodiments, a determination of whether a parameter of lung function is increased is made by comparing the baseline value with the value at any time point after the beginning of treatment, e.g., 48 weeks after the beginning of treatment, or between two time points, e.g., about 4 to about 14 weeks apart, after the beginning of treatment.

In some embodiments, an effective amount of a synthetic CXCR3 ligand is a dosage that is effective to increase the FVC by at least about 10% at least about 20%, at least about 25%, at least about 30%, at least about 40%, at least about 50%, at least about 60%, at least about 70%, at least about 80%, at least about 90%, at least about 2-fold, at least about 3-fold, at least about 4-fold, at least about 5-fold, or more compared to baseline on two consecutive occasions 4 to 14 weeks apart.

In some of these embodiments, an effective amount of a synthetic CXCR3 ligand is a dosage that results in a decrease in alveolar:arterial (A-a) gradient of at least about 5 mm Hg, at least about 7 mm Hg, at least about 10 mm Hg, at least about 12 mm Hg, at least about 15 mm Hg, or more, compared to baseline.

In some of these embodiments, an effective amount of a synthetic CXCR3 ligand is a dosage that increases the single breath DL_{co} by at least about 15 %, at least about 20%, at least about 30%, at least about 40%, at least about 50%, at least about 60%, at least about 70%, at least about 80%, at least about 90%, at least about 2-fold, at least about 3-fold, at least about 4-fold, at least about 5-fold, or more, compared to baseline. DL_{co} is the lung diffusing capacity for carbon monoxide, and is expressed as mL CO/mm Hg/second.

Parameters of lung function include, but are not limited to, forced vital capacity (FVC); forced expiratory volume (FEV₁); total lung capacity; partial pressure of arterial oxygen at rest; partial pressure of arterial oxygen at maximal exertion.

Lung function can be measured using any known method, including, but not limited to spirometry.

### Combination therapies

In some embodiments, the present invention provides combination therapies for the treatment of IPF. Accordingly, the present invention provides a method of treating IPF, generally involving administering a subject synthetic CXCR3 ligand in combination therapy with a second therapeutic agent. Suitable second therapeutic agents include, but are not limited to, a Type I interferon receptor agonist, a Type III interferon receptor agonist, a Type II interferon receptor agonist, pirfenidone or a pirfenidone analog, a TNF antagonist, a TGF-β antagonist, an endothelin receptor antagonist, a stress-activated protein kinase inhibitor, etc.

In other embodiments, the present invention provides methods of treating IPF that involve administering a synergistic combination of a subject synthetic CXCR3 ligand and a second therapeutic agent. As used herein, a "synergistic combination" of a subject synthetic CXCR3 ligand and a second therapeutic agent is a combined dosage that is more effective in the therapeutic or prophylactic treatment of IPF than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of the second therapeutic agent when administered at the same dosage as a monotherapy.

In some embodiments, the present invention provides methods that involve administering a synergistic combination of a subject synthetic CXCR3 ligand and pirfenidone or specific pirfenidone analog. As used herein, a "synergistic combination" of a subject synthetic CXCR3 ligand and pirfenidone or specific pirfenidone analog is a combined dosage that is more effective in the therapeutic or prophylactic treatment of IPF than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of pirfenidone or a specific pirfenidone analog when administered at the same dosage as a monotherapy.

### METHODS OF TREATING LIVER FIBROSIS

The present invention provides methods of treating liver fibrosis, including reducing clinical liver fibrosis, reducing the likelihood that liver fibrosis will occur, and reducing a parameter associated with liver fibrosis. The methods generally involve administering a combination of an effective amount of a subject synthetic CXCR3 ligand to an individual in need thereof. Of particular interest in many embodiments is treatment of humans.

Liver fibrosis is a precursor to the complications associated with liver cirrhosis, such as portal hypertension, progressive liver insufficiency, and hepatocellular carcinoma. A reduction in liver fibrosis thus reduces the incidence of such complications. Accordingly, the present invention further provides methods of reducing the likelihood that an individual will develop complications associated with cirrhosis of the liver.

The present methods generally involve administering a therapeutically effective amount of a subject synthetic CXCR3 ligand. As used herein, an "effective amount" of a subject synthetic CXCR3 ligand is an amount that is effective in reducing liver fibrosis or reduce the rate of progression of liver fibrosis; and/or that is effective in reducing the likelihood that an individual will develop liver fibrosis; and/or that is effective in reducing a parameter associated with liver fibrosis; and/or that is effective in reducing a disorder associated with cirrhosis of the liver.

The invention also provides a method for treatment of liver fibrosis in an individual comprising administering to the individual an amount of a subject synthetic CXCR3 ligand that is effective for prophylaxis or therapy of liver fibrosis in the individual, e.g., increasing the probability of survival, reducing the risk of death, ameliorating the disease burden or slowing the progression of disease in the individual.

Whether treatment with a subject synthetic CXCR3 ligand is effective in reducing liver fibrosis is determined by any of a number of well-established techniques for measuring liver fibrosis and liver function. Whether liver fibrosis is reduced is determined by analyzing a liver biopsy sample. An analysis of a liver biopsy comprises assessments of two major components: necroinflammation assessed by "grade" as a measure of the severity and ongoing disease activity, and the lesions of fibrosis and parenchymal or vascular remodeling as assessed by "stage" as being reflective of long-term disease progression. See, e.g., Brunt (2000) Hepatol. 31:241-246; and METAVIR (1994) Hepatology 20:15-20. Based on analysis of the liver biopsy, a score is assigned. A number of standardized scoring systems exist which provide a quantitative assessment of the degree and severity of fibrosis. These include the METAVIR, Knodell, Scheuer, Ludwig, and Ishak scoring systems.

The METAVIR scoring system is based on an analysis of various features of a liver biopsy, including fibrosis (portal fibrosis, centrilobular fibrosis, and cirrhosis); necrosis (piecemeal and lobular necrosis, acidophilic retraction, and ballooning degeneration); inflammation (portal tract inflammation, portal lymphoid aggregates, and distribution of portal inflammation); bile duct changes; and the Knodell index (scores of periportal necrosis, lobular necrosis, portal inflammation, fibrosis, and overall disease activity). The definitions of each stage in the METAVIR system are as follows: score: 0, no fibrosis; score: 1, stellate enlargement of portal tract but without septa formation; score: 2, enlargement of portal tract with rare septa formation; score: 3, numerous septa without cirrhosis; and score: 4, cirrhosis.

Knodell's scoring system, also called the Hepatitis Activity Index, classifies specimens based on scores in four categories of histologic features: I. Periportal and/or bridging necrosis; II. Intralobular degeneration and focal necrosis; III. Portal inflammation; and IV. Fibrosis. In the Knodell staging system, scores are as follows: score: 0, no fibrosis; score: 1, mild fibrosis (fibrous portal expansion); score: 2, moderate fibrosis; score: 3, severe fibrosis (bridging fibrosis); and score: 4, cirrhosis. The higher the score, the more severe the liver tissue damage. Knodell (1981) Hepatol. 1:431.

In the Scheuer scoring system scores are as follows: score: 0, no fibrosis; score: 1, enlarged, fibrotic portal tracts; score: 2, periportal or portal-portal septa, but intact architecture; score: 3, fibrosis with architectural distortion, but no obvious cirrhosis; score: 4, probable or definite cirrhosis. Scheuer (1991) J. Hepatol. 13:372.

The Ishak scoring system is described in Ishak (1995) J. Hepatol. 22:696-699. Stage 0, no fibrosis; stage 1, fibrous expansion of some portal areas, with or without short fibrous septa; stage 2, fibrous expansion of most portal areas, with or without short fibrous septa; stage 3, fibrous expansion of most portal areas with occasional portal to portal (P-P) bridging; stage 4, fibrous expansion of portal areas with marked bridging (P-P) as well as portal-central (P-C); stage 5, marked bridging (P-P and/or P-C) with occasional nodules (incomplete cirrhosis); stage 6, cirrhosis, probable or definite .The benefit of anti-fibrotic therapy can also be measured and assessed by using the Child-Pugh scoring system which comprises a multicomponent point system based upon abnormalities in serum bilirubin level, serum albumin level, prothrombin time, the presence and severity of ascites, and the presence and severity of encephalopathy. Based upon the presence and severity of abnormality of these parameters, patients may be placed in one of three categories of increasing severity of clinical disease: A, B, or C.

In some embodiments, a therapeutically effective amount of a subject synthetic CXCR3 ligand is an amount that effects a change of one unit or more in the fibrosis stage based on pre-and post-therapy liver biopsies: In particular embodiments, a therapeutically effective amount of a subject synthetic CXCR3 ligand reduces liver fibrosis by at least one unit in the METAVIR, the Knodell, the Scheuer, the Ludwig, or the Ishak scoring system.

Secondary, or indirect, indices of liver function can also be used to evaluate the efficacy of treatment with a subject synthetic CXCR3 ligand. Morphometric computerized semi-automated assessment of the quantitative degree of liver fibrosis based upon specific staining of collagen and/or serum markers of liver fibrosis can also be measured as an indication of the efficacy of a subject treatment method. Secondary indices of liver function include, but are not limited to, serum transaminase levels, prothrombin time, bilirubin, platelet count, portal pressure, albumin level, and assessment of the Child-Pugh score.

In another embodiment, an effective amount of a subject synthetic CXCR3 ligand is an amount that is effective to increase an index of liver function by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to the index of liver function in an untreated individual, or in a placebo-treated individual. Those skilled in the art can readily measure such indices of liver function, using standard assay methods, many of which are commercially available, and are used routinely in clinical settings.

Serum markers of liver fibrosis can also be measured as an indication of the efficacy of a subject treatment method. Serum markers of liver fibrosis include, but are not limited to, hyaluronate, N-terminal procollagen III peptide, 7S domain of type IV collagen, C-terminal procollagen I peptide, and laminin. Additional biochemical markers of liver fibrosis include α-2-macroglobulin, haptoglobin, gamma globulin, apolipoprotein A, and gamma glutamyl transpeptidase.

In another embodiment, a therapeutically effective amount of a subject synthetic CXCR3 ligand is an amount that is effective to reduce a serum level of a marker of liver fibrosis by at least about 10%, at least about 20%, at least about 25%, at least about 3 0%, at least about 35%, at least about 40%, at least about 45%, at least about 50%, at least about 55%, at least about 60%, at least about 65%, at least about 70%, at least about 75%, or at least about 80%, or more, compared to the level of the marker in an untreated individual, or in a placebo-treated individual. Those skilled in the art can readily measure such serum markers of liver fibrosis, using standard assay methods, many of which are commercially available, and are used routinely in clinical settings. Methods of measuring serum markers include immunological-based methods, e.g., enzyme-linked immunosorbent assays (ELISA), radioimmunoassays, and the like, using antibody specific for a given serum marker.

Quantitative tests of functional liver reserve can also be used to assess the efficacy of treatment with a subject synthetic CXCR3 ligand. These include: indocyanine green clearance (ICG), galactose elimination capacity (GEC), aminopyrine breath test (ABT), antipyrine clearance, monoethylglycine-xylidide (MEG-X) clearance, and caffeine clearance.

As used herein, a "complication associated with cirrhosis of the liver" refers to a disorder that is a sequellae of decompensated liver disease, i.e., or occurs subsequently to and as a result of development of liver fibrosis, and includes, but it not limited to, development of ascites, variceal bleeding, portal hypertension, jaundice, progressive liver insufficiency, encephalopathy, hepatocellular carcinoma, liver failure requiring liver transplantation, and liver-related mortality.

In another embodiment, a therapeutically effective amount of a subject synthetic CXCR3 ligand is an amount that is effective in reducing the incidence (e.g., the likelihood that an individual will develop) of a disorder associated with cirrhosis of the liver by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to an untreated individual, or in a placebo-treated individual.

Whether combination therapy with a subject synthetic CXCR3 ligand is effective in reducing the incidence of a disorder associated with cirrhosis of the liver can readily be determined by those skilled in the art.

Reduction in liver fibrosis increases liver function. Thus, the invention provides methods for increasing liver function, generally involving administering a therapeutically effective amount of a subject synthetic CXCR3 ligand. Liver functions include, but are not limited to, synthesis of proteins such as serum proteins (e.g., albumin, clotting factors, alkaline phosphatase, aminotransferases (e.g., alanine transaminase, aspartate transaminase), 5'-nucleosidase, γ-glutaminyltranspeptidase, etc.), synthesis of bilirubin, synthesis of cholesterol, and synthesis of bile acids; a liver metabolic function, including, but not limited to, carbohydrate metabolism, amino acid and ammonia metabolism, hormone metabolism, and lipid metabolism; detoxification of exogenous drugs; a hemodynamic function, including splanchnic and portal hemodynamics; and the like.

Whether a liver function is increased is readily ascertainable by those skilled in the art, using well-established tests of liver function. Thus, synthesis of markers of liver function such as albumin, alkaline phosphatase, alanine transaminase, aspartate transaminase, bilirubin, and the like, can be assessed by measuring the level of these markers in the serum, using standard immunological and enzymatic assays. Splanchnic circulation and portal hemodynamics can be measured by portal wedge pressure and/or resistance using standard methods. Metabolic functions can be measured by measuring the level of ammonia in the serum.

Whether serum proteins normally secreted by the liver are in the normal range can be determined by measuring the levels of such proteins, using standard immunological and enzymatic assays. Those skilled in the art know the normal ranges for such serum proteins. The following are non-limiting examples. The normal range of alanine transaminase is from about 7 to about 56 units per liter of serum. The normal range of aspartate transaminase is from about 5 to about 40 units per liter of serum. Bilirubin is measured using standard assays. Normal bilirubin levels are usually less than about 1.2 mg/dL. Serum albumin levels are measured using standard assays. Normal levels of serum albumin are in the range of from about 35 to about 55 g/L. Prolongation of prothrombin time is measured using standard assays. Normal prothrombin time is less than about 4 seconds longer than control.

In another embodiment, a therapeutically effective amount of a subject synthetic CXCR3 ligand is an amount that is effective to increase liver function by at least about 10%, at least about 20%, at least about 30%, at least about 40%, at least about 50%, at least about 60%, at least about 70%, at least about 80%, or more. For example, a therapeutically effective amount of a subject synthetic CXCR3 ligand is an amount that is effective to reduce an elevated level of a serum marker of liver function by at least about 10%, at least about 20%, at least about 30%, at least about 40%, at least about 50%, at least about 60%, at least about 70%, at least about 80%, or more, or to reduce the level of the serum marker of liver function to within a normal range. A therapeutically effective amount of a subject synthetic CXCR3 ligand is also an amount effective to increase a reduced level of a serum marker of liver function by at least about 10%, at least about 20%, at least about 30%, at least about 40%, at least about 50%, at least about 60%, at least about 70%, at least about 80%, or more, or to increase the level of the serum marker of liver function to within a normal range.

### Combination therapies

In some embodiments, the present invention provides combination therapies for the treatment of liver fibrosis. Accordingly, the present invention provides a method of treating liver fibrosis, generally involving administering a subject synthetic CXCR3 ligand in combination therapy with a second therapeutic agent. Suitable second therapeutic agents include, but are not limited to, a Type I interferon receptor agonist, a Type III interferon receptor agonist, a Type II interferon receptor agonist, pirfenidone or a pirfenidone analog, a TNF antagonist, a TGF-β antagonist, an endothelin receptor antagonist, a stress-activated protein kinase inhibitor, etc.

In other embodiments, the present invention provides methods of treating liver fibrosis that involve administering a synergistic combination of a subject synthetic CXCR3 ligand and a second therapeutic agent. As used herein, a "synergistic combination" of a subject synthetic CXCR3 ligand and a second therapeutic agent is a combined dosage that is more effective in the therapeutic or prophylactic treatment of liver fibrosis than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of the second therapeutic agent when administered at the same dosage as a monotherapy.

In some embodiments, the present invention provides methods that involve administering a synergistic combination of a subject synthetic CXCR3 ligand and pirfenidone or specific pirfenidone analog. As used herein, a "synergistic combination" of a subject synthetic CXCR3 ligand and pirfenidone or specific pirfenidone analog is a combined dosage that is more effective in the therapeutic or prophylactic treatment of liver fibrosis than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of pirfenidone or a specific pirfenidone analog when administered at the same dosage as a monotherapy.

### METHODS OF TREATING RENAL FIBROSIS

Renal fibrosis is characterized by the excessive accumulation of extracellular matrix (ECM) components. Overproduction of transforming growth factor-beta (TGF-β) is believed to underlie tissue fibrosis caused by excess deposition of ECM, resulting in disease. TGF-β's fibrogenic action results from simultaneous stimulation of matrix protein synthesis, inhibition of matrix degradation and enhanced integrin expression that facilitates ECM assembly.

The present invention provides methods of treating renal fibrosis. The methods generally involve administering to an individual having renal fibrosis an effective amount of a subject synthetic CXCR3 ligand. As used herein, an "effective amount" of a subject synthetic CXCR3 ligand that is effective in reducing renal fibrosis; and/or that is effective in reducing the likelihood that an individual will develop renal fibrosis; and/or that is effective in reducing a parameter associated with renal fibrosis; and/or that is effective in reducing a disorder associated with fibrosis of the kidney.

In one embodiment, an effective amount of a subject synthetic CXCR3 ligand is an amount that is sufficient to reduce renal fibrosis, or reduce the rate of progression of renal fibrosis, by at least about 10%, at least about 15%, at least about 20%, at least about 25%, at least about 30%, at least about 35%, at least about 40%, at least about 45%, at least about 50%, compared to the degree of renal fibrosis in the individual prior to treatment, or compared to the rate of progression of renal fibrosis that would have been experienced by the patient in the absence of treatment.

Whether fibrosis is reduced in the kidney is determined using any known method. For example, histochemical analysis of kidney biopsy samples for the extent of ECM deposition and/or fibrosis is performed. Other methods are known in the art. See, e.g., Masseroli et al. (1998) Lab. Invest. 78:511-522; U.S. Patent No. 6,214,542.

In some embodiments, an effective amount of a subject synthetic CXCR3 ligand is an amount that is effective to increase kidney function by at least about 10%, at least about 15%, at least about 20%, at least about 25%, at least about 30%, at least about 35%, at least about 40%, at least about 45%, at least about 50%, compared to the basal level of kidney function in the individual prior to treatment.

In some embodiments, an effective amount of a subject synthetic CXCR3 ligand is an amount that is effective to slow the decline in kidney function by at least about 10%, at least about 15%, at least about 20%, at least about 25%, at least about 30%, at least about 35%, at least about 40%, at least about 45%, at least about 50%, compared to the decline in kidney function that would occur in the absence of treatment.

Kidney function can be measured using any known assay, including, but not limited to, plasma creatinine level (where normal levels are generally in a range of from about 0.6 to about 1.2 mg/dL); creatinine clearance (where the normal range for creatinine clearance is from about 97 to about 137 mL/minute in men, and from about 88 to about 128 mL/minute in women); the glomerular filtration rate (either calculated or obtained from inulin clearance or other methods), blood urea nitrogen (where the normal range is from about 7 to about 20 mg/dL); and urine protein levels.

In other embodiments, the present invention provides methods that involve administering a synergistic combination of a subject synthetic CXCR3 ligand and a second therapeutic agent. As used herein, a "synergistic combination" of a subject synthetic CXCR3 ligand and a second therapeutic agent is a combined dosage that is more effective in the therapeutic or prophylactic treatment of renal fibrosis than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of the second therapeutic agent when administered at the same dosage as a monotherapy.

The invention also provides a method for treatment of renal fibrosis in an individual comprising administering to the individual a subject synthetic CXCR3 ligand in an amount that is effective for prophylaxis or therapy of renal fibrosis in the individual, e.g., increasing the time to doubling of serum creatinine levels, increasing the time to end-stage renal disease requiring renal replacement therapy (e.g., dialysis or transplant), increasing the probability of survival, reducing the risk of death, ameliorating the disease burden or slowing the progression of disease in the individual.

### Combination therapies

In some embodiments, the present invention provides combination therapies for the treatment of renal fibrosis. Accordingly, the present invention provides a method of treating liver fibrosis, generally involving administering a subject synthetic CXCR3 ligand in combination therapy with a second therapeutic agent. Suitable second therapeutic agents include, but are not limited to, a Type I interferon receptor agonist, a Type III interferon receptor agonist, a Type II interferon receptor agonist, pirfenidone or a pirfenidone analog, a TNF antagonist, a TGF-β antagonist, an endothelin receptor antagonist, a stress-activated protein kinase inhibitor, etc.

In other embodiments, the present invention provides methods of treating renal fibrosis that involve administering a synergistic combination of a subject synthetic CXCR3 ligand and a second therapeutic agent. As used herein, a "synergistic combination" of a subject synthetic CXCR3 ligand and a second therapeutic agent is a combined dosage that is more effective in the therapeutic or prophylactic treatment of renal fibrosis than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of the second therapeutic agent when administered at the same dosage as a monotherapy.

In some embodiments, the present invention provides methods that involve administering a synergistic combination of a subject synthetic CXCR3 ligand and pirfenidone or specific pirfenidone analog. As used herein, a "synergistic combination" of a subject synthetic CXCR3 ligand and pirfenidone or specific pirfenidone analog is a combined dosage that is more effective in the therapeutic or prophylactic treatment of renal fibrosis than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of pirfenidone or a specific pirfenidone analog when administered at the same dosage as a monotherapy.

### METHODS OF TREATING CANCER

The present invention provides methods of treating cancer. The methods generally involve administering an effective amount of a subject synthetic CXCR3 ligand to an individual in need thereof.

The methods are effective to reduce a tumor load by at least about 5%, at least about 10%, at least about 20%, at least about 25%, at least about 50%, at least about 75%, at least about 85%, or at least about 90%, up to total eradication of the tumor, when compared to a suitable control. Thus, in these embodiments, an "effective amount" of a subject synthetic CXCR3 ligand is an amount that is sufficient to reduce tumor load by at least about 5%, at least about 10%, at least about 20%, at least about 25%, at least about 50%, at least about 75%, at least about 85%, or at least about 90%, up to total eradication of the tumor, when compared to a suitable control. In an experimental animal system, a suitable control may be a genetically identical animal not treated with the synthetic CXCR3 ligand. In non-experimental systems, a suitable control may be the tumor load present before administering the synthetic CXCR3 ligand. Other suitable controls may be a placebo control.

Whether a tumor load has been decreased can be determined using any known method, including, but not limited to, measuring solid tumor mass; counting the number of tumor cells using cytological assays; fluorescence-activated cell sorting (e.g., using antibody specific for a tumor-associated antigen) to determine the number of cells bearing a given tumor antigen; computed tomography scanning, magnetic resonance imaging, and/or x-ray imaging of the tumor to estimate and/or monitor tumor size; measuring the amount of tumor-associated antigen in a biological sample, e.g., blood; and the like.

The methods are effective to reduce the growth rate of a tumor by at least about 5%, at least about 10%, at least about 20%, at least about 25%, at least about 50%, at least about 75%, at least about 85%, or at least about 90%, up to total inhibition of growth of the tumor, when compared to a suitable control. Thus, in these embodiments, "effective amounts" of a synthetic CXCR3 ligand is an amount that is sufficient to reduce tumor growth rate by at least about 5%, at least about 10%, at least about 20%, at least about 25%, at least about 50%, at least about 75%, at least about 85%, or at least about 90%, up to total inhibition of tumor growth, when compared to a suitable control. In an experimental animal system, a suitable control may be a genetically identical animal not treated with the synthetic CXCR3 ligand. In non-experimental systems, a suitable control may be the tumor load present before administering the synthetic CXCR3 ligand. Other suitable controls may be a placebo control.

Whether growth of a tumor is inhibited can be determined using any known method, including, but not limited to, an *in vitro* proliferation assay; a ³H-thymidine uptake assay; and the like.

The methods are useful for treating a wide variety of cancers, including carcinomas, sarcomas, leukemias, and lymphomas.

Carcinomas that can be treated using a subject method include, but are not limited to, esophageal carcinoma, hepatocellular carcinoma, basal cell carcinoma (a form of skin cancer), squamous cell carcinoma (various tissues), bladder carcinoma, including transitional cell carcinoma (a malignant neoplasm of the bladder), bronchogenic carcinoma, colon carcinoma, colorectal carcinoma, gastric carcinoma, lung carcinoma, including small cell carcinoma and non-small cell carcinoma of the lung, adrenocortical carcinoma, thyroid carcinoma, pancreatic carcinoma, breast carcinoma, ovarian carcinoma, prostate carcinoma, adenocarcinoma, sweat gland carcinoma, sebaceous gland carcinoma, papillary carcinoma; papillary adenocarcinoma, cystadenocarcinoma, medullary carcinoma, renal cell carcinoma, ductal carcinoma in situ or bile duct carcinoma, choriocarcinoma, seminoma, embryonal carcinoma, Wilm's tumor, cervical carcinoma, uterine carcinoma, testicular carcinoma, osteogenic carcinoma, epithelieal carcinoma, and nasopharyngeal carcinoma, etc.

Sarcomas that can be treated using a subject method include, but are not limited to, fibrosarcoma, myxosarcoma, liposarcoma, chondrosarcoma, chordoma, osteogenic sarcoma, osteosarcoma, angiosarcoma, endotheliosarcoma, lymphangiosarcoma, lymphangioendotheliosarcoma; synovioma, mesothelioma, Ewing's sarcoma, leiomyosarcoma, rhabdomyosarcoma, and other soft tissue sarcomas.

Other solid tumors that can be treated using a subject method include, but are not limited to, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

Leukemias that can be treated using a subject method include, but are not limited to, a) chronic myeloproliferative syndromes (neoplastic disorders of multipotential hematopoietic stem cells); b) acute myelogenous leukemias (neoplastic transformation of a multipotential hematopoietic stem cell or a hematopoietic cell of restricted lineage potential; c) chronic lymphocytic leukemias (CLL; clonal proliferation of immunologically immature and functionally incompetent small lymphocytes), including B-cell CLL, T-cell CLL prolymphocytic leukemia, and hairy cell leukemia; and d) acute lymphoblastic leukemias (characterized by accumulation of lymphoblasts). Lymphomas that can be treated using a subject method include, but are not limited to, B-cell lymphomas (e.g., Burkitt's lymphoma); Hodgkin's lymphoma; and the like.

### Combination therapies

In some embodiments, the present invention provides combination therapies for the treatment of cancer. Accordingly, the present invention provides a method of treating cancer, generally involving administering a subject synthetic CXCR3 ligand in combination therapy with at least a second therapeutic agent. Suitable second therapeutic agents include, but are not limited to, a Type I interferon receptor agonist; a Type II interferon receptor agonist; a Type III interferon receptor agonist; pirfenidone or a pirfenidone analog; and an antiproliferative agent selected from an alkylating agent, a nitrosourea, an antimetabolite, an anti-tumor antibody, a steroid hormone, a vinca alkyloid, a biological response modifier, and a taxane.

In other embodiments, the present invention provides methods of treating cancer that involve administering a synergistic combination of a subject synthetic CXCR3 ligand and a second therapeutic agent. As used herein, a "synergistic combination" of a subject synthetic CXCR3 ligand and a second therapeutic agent is a combined dosage that is more effective in the therapeutic or prophylactic treatment of cancer than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of the second therapeutic agent when administered at the same dosage as a monotherapy.

In some embodiments, a synthetic CXCR3 ligand is administered as an adjuvant therapy to a standard cancer therapy. Standard cancer therapies include surgery (e.g., surgical removal of cancerous tissue), radiation therapy, bone marrow transplantation, chemotherapeutic treatment, biological response modifier treatment, and certain combinations of the foregoing.

Radiation therapy includes, but is not limited to, x-rays or gamma rays that are delivered from either an externally applied source such as a beam, or by implantation of small radioactive sources.

Chemotherapeutic agents are non-peptidic (i.e., non-proteinaceous) compounds that reduce proliferation of cancer cells, and encompass cytotoxic agents and cytostatic agents. Non-limiting examples of chemotherapeutic agents include alkylating agents, nitrosoureas, antimetabolites, antitumor antibiotics, plant (vinca) alkaloids, and steroid hormones.

Agents that act to reduce cellular proliferation are known in the art and widely used. Such agents include alkylating agents, such as nitrogen mustards, nitrosoureas, ethylenimine derivatives, alkyl sulfonates, and triazenes, including, but not limited to, mechlorethamine, cyclophosphamide (Cytoxan™), melphalan (L-sarcolysin), carmustine (BCNU), lomustine (CCNU), semustine (methyl-CCNU), streptozocin, chlorozotocin, uracil mustard, chlormethine, ifosfamide, chlorambucil, pipobroman, triethylenemelamine, triethylenethiophosphoramine, busulfan, dacarbazine, and temozolomide.

Antimetabolite agents include folic acid analogs, pyrimidine analogs, purine analogs, and adenosine deaminase inhibitors, including, but not limited to, cytarabine (CYTOSAR-U), cytosine arabinoside, fluorouracil (5-FU), floxuridine (FudR), 6-thioguanine, 6-mercaptopurine (6-MP), pentostatin, 5-fluorouracil (5-FU), methotrexate, 10-propargyl-5,8-dideazafolate (PDDF, CB3717), 5,8-dideazatetrahydrofolic acid (DDATHF), leucovorin, fludarabine phosphate, pentostatine, and gemcitabine.

Suitable natural products and their derivatives, (e.g., vinca alkaloids, antitumor antibiotics, enzymes, lymphokines, and epipodophyllotoxins), include, but are not limited to, Ara-C, paclitaxel (Taxol®), docetaxel (Taxotere®), deoxycoformycin, mitomycin-C, L-asparaginase, azathioprine; brequinar; alkaloids, *e.g*. vincristine, vinblastine, vinorelbine, vindesine, *etc*.; podophyllotoxins, *e.g*. etoposide, teniposide, *etc*.; antibiotics, *e.g*. anthracycline, daunorubicin hydrochloride (daunomycin, rubidomycin, cerubidine), idarubicin, doxorubicin, epirubicin and morpholino derivatives, *etc*.; phenoxizone biscyclopeptides, *e.g*. dactinomycin; basic glycopeptides, *e.g*. bleomycin; anthraquinone glycosides, *e.g*. plicamycin (mithramycin); anthracenediones, *e.g*. mitoxantrone; azirinopyrrolo indolediones, *e.g*. mitomycin; macrocyclic immunosuppressants, *e.g*. cyclosporine, FK-506 (tacrolimus, prograf), rapamycin, *etc*.; and the like.

Other anti-proliferative cytotoxic agents are navelbene, CPT-11, anastrazole, letrazole, capecitabine, reloxafine, cyclophosphamide, ifosamide, and droloxafine.

Microtubule affecting agents that have antiproliferative activity are also suitable for use and include, but are not limited to, allocolchicine (NSC 406042), Halichondrin B (NSC 609395), colchicine (NSC 757), colchicine derivatives (e.g., NSC 33410), dolstatin 10 (NSC 376128), maytansine (NSC 153858), rhizoxin (NSC 332598), paclitaxel (Taxol®), Taxol® derivatives, docetaxel (Taxotere®), thiocolchicine (NSC 361792), trityl cysterin, vinblastine sulfate, vincristine sulfate, natural and synthetic epothilones including but not limited to, eopthilone A, epothilone B, discodermolide; estramustine, nocodazole, and the like.

Hormone modulators and steroids (including synthetic analogs) that are suitable for use include, but are not limited to, adrenocorticosteroids, *e.g*. prednisone, dexamethasone, *etc*.; estrogens and pregestins, *e.g*. hydroxyprogesterone caproate, medroxyprogesterone acetate, megestrol acetate, estradiol, clomiphene, tamoxifen; *etc*.; and adrenocortical suppressants, *e.g*. aminoglutethimide; 17α-ethinylestradiol; diethylstilbestrol, testosterone, fluoxymesterone, dromostanolone propionate, testolactone, methylprednisolone, methyl-testosterone, prednisolone, triamcinolone, chlorotrianisene, hydroxyprogesterone, aminoglutethimide, estramustine, medroxyprogesterone acetate, leuprolide, Flutamide (Drogenil), Toremifene (Fareston), and Zoladex®. Estrogens stimulate proliferation and differentiation, therefore compounds that bind to the estrogen receptor are used to block this activity. Corticosteroids may inhibit T cell proliferation.

Other chemotherapeutic agents include metal complexes, *e.g*. cisplatin (cis-DDP), carboplatin, *etc*.; ureas, *e.g*. hydroxyurea; and hydrazines, *e.g*. N-methylhydrazine; epidophyllotoxin; a topoisomerase inhibitor; procarbazine; mitoxantrone; leucovorin; tegafur; etc. Other anti-proliferative agents of interest include immunosuppressants, *e.g*. mycophenolic acid, thalidomide, desoxyspergualin, azasporine, leflunomide, mizoribine, azaspirane (SKF 105685); Iressa® (ZD 1839, 4-(3-chloro-4-fluorophenylamino)-7-methoxy-6-(3-(4-morpholinyl)propoxy)quinazoline); etc.

"Taxanes" include paclitaxel, as well as any active taxane derivative or pro-drug. "Paclitaxel" (which should be understood herein to include analogues, formulations, and derivatives such as, for example, docetaxel, Taxol®, Taxotere® (a formulation of docetaxel), 10-desacetyl analogs of paclitaxel and 3'N-desbenzoyl-3'N-t-butoxycarbonyl analogs of paclitaxel) may be readily prepared utilizing techniques known to those skilled in the art (see also WO 94/07882, WO 94/07881, WO 94/07880, WO 94/07876, WO 93/23555, WO 93/10076; U.S. Pat. Nos. 5,294,637; 5,283,253; 5,279,949; 5,274,137; 5,202,448; 5,200,534; 5,229,529; and EP 590,267), or obtained from a variety of commercial sources, including for example, Sigma Chemical Co., St. Louis, Mo. (T7402 from *Taxus brevifolia*; or T-1912 from *Taxus yannanensis*).

Paclitaxel should be understood to refer to not only the common chemically available form of paclitaxel, but analogs and derivatives (e.g., Taxotere® docetaxel, as noted above) and paclitaxel conjugates (e.g., paclitaxel-PEG, paclitaxel-dextran, or paclitaxel-xylose).

Also included within the term "taxane" are a variety of known derivatives, including both hydrophilic derivatives, and hydrophobic derivatives. Taxane derivatives include, but not limited to, galactose and mannose derivatives described in International Patent Application No. WO 99/18113; piperazino and other derivatives described in WO 99/14209; taxane derivatives described in WO 99/09021, WO 98/22451, and U.S. Patent No. 5,869,680; 6-thio derivatives described in WO 98/28288; sulfenamide derivatives described in U.S. Patent No. 5,821,263; and taxol derivative described in U.S. Patent No. 5,415,869. It further includes prodrugs of paclitaxel including, but not limited to, those described in WO 98/58927; WO 98/13059; and U.S. Patent No. 5,824,701.

Biological response modifiers suitable for use in connection with the methods of the invention include, but are not limited to, (1) inhibitors of tyrosine kinase (RTK) activity; (2) inhibitors of serine/threonine kinase activity; (3) tumor-associated antigen antagonists, such as antibodies that bind specifically to a tumor antigen; (4) apoptosis receptor agonists; (5) interleukin-2; (6) IFN-α; (7) IFN-γ; (8) colony-stimulating factors; (9) inhibitors of angiogenesis; and (10) antagonists of tumor necrosis factor.

### METHODS OF TREATING ANGIOGENIC DISORDERS

The present invention provides methods for treating angiogenic disorders. The methods generally involve administering an effective amount of a subject synthetic CXCR3 ligand to an individual in need thereof.

In a subject method of treating an angiogenic disorder, an "effective amount" of a subject synthetic CXCR3 ligand is an amount that is angiostatic, e.g., an amount that reduces angiogenesis by at least about 10%, at least about 20%, at least about 25%, at least about 30%, at least about 40%, at least about 50%, at least about 60%, at least about 70%, at least about 80%, or at least about 90%, or more, compared with the level of angiogenesis in the absence of treatment with the synthetic CXCR3 ligand.

Many systems are available for assessing angiogenesis. For example, as angiogenesis is required for solid tumor growth, the inhibition of tumor growth in an animal model may be used as an index of the inhibition of angiogenesis. Angiogenesis may also be assessed in terms of models of wound-healing, in cutaneous or organ wound repair; and in chronic inflammation, e.g., in diseases such as rheumatoid-arthritis, atherosclerosis and idiopathic pulmonary fibrosis (IPF). It may also be assessed by counting vessels in tissue sections, e.g., following staining for marker molecules, e.g., CD3H, Factor VIII, or PECAM-1.

Whether angiogenesis is reduced can be determined using any method known in the art, including, e.g., stimulation of neovascularization into implants impregnated with relaxin; stimulation of blood vessel growth in the cornea or anterior eye chamber; stimulation of endothelial cell proliferation, migration or tube formation *in vitro*; and the chick chorioallantoic membrane assay; the hamster cheek pouch assay; the polyvinyl alcohol sponge disk assay. Such assays are well known in the art and have been described in numerous publications, including, e.g., Auerbach et al. ((1991) Pharmac. Ther. 51:1-11), and references cited therein.

A system in widespread use for assessing angiogenesis is the corneal micropocket assay of neovascularization, as may be practiced using rat corneas. This *in vivo* model is widely accepted as being generally predictive of clinical usefulness. See, e.g., O'Reilly et. al. (1994) Cell 79:315-328, Li et. al. (1991) Invest. Ophthalmol. Vis. Sci. 32(11):2898-905; and Miller et. al. (1994) Am. J. Pathol. 145(3):574-84.

The subject method is useful for treating angiogenic disorders, e.g., any disease characterized by pathological neovacularization. Such disorders include, but are not limited to, solid tumors, hemangiomas, rheumatoid arthritis, atherosclerosis and idiopathic pulmonary fibrosis (IPF); but also include BPH, vascular restenosis, arteriovenous malformations (AVM), retinopathies, including diabetic retinopathy, meningioma, hemangiomas, thyroid hyperplasias (including Grave's disease), neovascular glaucoma, neovascularization associated with corneal injury, neovascularization associated with corneal transplantation, neovascularization associated with corneal graft, psoriasis, angiofibroma, hemophilic joints, hypertrophic scars, osler-weber syndrome, age-related macular degeneration, pyogenic granuloma retrolental fibroplasia, scleroderma, trachoma, vascular adhesions, synovitis, dermatitis, and endometriosis.

### Combination therapies

In some embodiments, the present invention provides combination therapies for the treatment of angiogenic disorders. Accordingly, the present invention provides a method of treating an angiogenic disorder, generally involving administering a subject synthetic CXCR3 ligand in combination therapy with at least a second therapeutic agent. Suitable second therapeutic agents include, but are not limited to, a Type I interferon receptor agonist, a Type II interferon receptor agonist, a Type III interferon receptor agonist, and pirfenidone or a pirfenidone analog.

In other embodiments, the present invention provides methods of treating an angiogenic disorder that involve administering a synergistic combination of a subject synthetic CXCR3 ligand and a second therapeutic agent. As used herein, a "synergistic combination" of a subject synthetic CXCR3 ligand and a second therapeutic agent is a combined dosage that is more effective in the therapeutic or prophylactic treatment of an angiogenic disorder than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of the second therapeutic agent when administered at the same dosage as a monotherapy.

### METHODS OF TREATING BACTERIAL INFECTIONS

The present invention provides methods of treating a bacterial infection. The methods generally involve administering an effective amount of a subject synthetic CXCR3 ligand to an individual in need thereof. Individuals in need thereof include individuals diagnosed as having a bacterial infection, e.g., an infection with pathological bacteria (including opportunistic infections).

Bacterial infections that can be treated using the methods of the invention include infections of gram positive and gram negative, aerobic and anaerobic organisms, including, but not limited to, Staphylococcus, Lactobacillus, Streptococcus, Sarcina, Escherichia, Enterobacter, Klebsiella, Pseudomonas, Acinetobacter, Proteus, Campylobacter, Citrobacter, Nisseria, Baccillus, Bacteroides, Peptococcus, Clostridium, Salmonella, Shigella, Serratia, Haemophilus, Brucella and other organisms.

All clinically important members of the family *Enterobacteriaceae*, including, but not limited to: all clinically important strains of *Escherichia*, with *E. coli* being of particular interest; all clinically important strains of Klebsiella, with *K. pneumoniae* being of particular interest; all clinically important strains of Shigella, with *S. dysenteriae* being of particular interest; all clinically important strains of Salmonella, including *S. abortus-equi*, *S*. *typhi*, *S. typhimurium*, *S. newport*, *S. paratyphi-A*, *S. paratyphi-B*, *S. potsdam*, and *S. pollurum*; all clinically important strains of Serratia, most notably *S*. *marcescens*; all clinically important strains of Yersinia, most notably *Y. pestis*; all clinically important strains of *Enterobacter*, most notably *E*. *cloacae*; all clinically important Enterococci, most notably *E*. *faecalis* and *E*. *faecium*; all clinically important Haemophilus strains, most notably *H*. *influenzae* all clinically important Mycobacteria, most notably *M. tuberculosis*, *M. avium-intracellulare*, *M. bovis*, and *M leprae*; *Neisseria gonorrhoeae* and *N. meningitidis*; all clinically important Pseudomonads, with *P. aeuruginosa* being of particular interest; all clinically important Staphylococci, with *S. aureus* and *S*. *epidermidis* being of particular interest; all clinically important *Streptococci*, with *S. pneumoniae* being of particular interest; and *Vibrio cholera.*

A subject method of treating a bacterial infection involves administering an effective amount of a subject synthetic CXCR3 ligand. An effective amount of a subject synthetic CXCR3 ligand is an amount that is effective, in one or more dosages, to reduce a symptom or parameter associated with the bacterial infection by at least about 10%, at least about 20%, at least about 25%, at least about 30%, at least about 40%, at least about 50%, at least about 60%, at least about 70%, at least about 80%, at least about 90%, or more, compared with no treatment with the synthetic CXCR3 ligand.

Parameters associated with a bacterial infection includes number of bacteria in the infected individual, or in a particular tissue, fluid, or organ of the infected individual. Thus, in some embodiments, an effective amount of a subject synthetic CXCR3 ligand is an amount that, when administered in one or more dosages, is effective to reduce the number of bacteria in an individual or a tissue, fluid, or organ of the individual by at least about 10%, at least about 20%, at least about 25%, at least about 30%, at least about 40%, at least about 50%, at least about 60%, at least about 70%, at least about 80%, at least about 90%, or more, compared with no treatment with the synthetic CXCR3 ligand.

Symptoms associated with a bacterial infection include, but are not limited to, chills, fever, vomiting, elevated white blood cell count, and the like. The actual amount of compound administered and the route of administration will depend upon the particular disease or bacteria as well as other factors such as the size, age, health status, and sex of the individual being treated and is determined by routine analysis.

Whether a subject treatment method is effective in reducing a parameter or symptom associated with a bacterial infection can be determined using any known method. For example, bacterial count is determined using standard assays. For example, the number of bacteria in a biological sample from an individual is determined by culturing the sample on medium designed to allow growth of a given pathogenic bacterium, and, after allowing a period of time for growth, counting the number of bacteria. The number of bacteria in a fluid sample can be determined by counting the number of bacteria as visualized under a microscope.

### Combination therapies

In some embodiments, the present invention provides combination therapies for the treatment of bacterial infections. Accordingly, the present invention provides a method of treating a bacterial infection, generally involving administering a subject synthetic CXCR3 ligand in combination therapy with at least a second therapeutic agent. Suitable second therapeutic agents include, but are not limited to, a Type II interferon receptor antagonist, and a standard antibiotic agent, e.g., a β-lactam, a macrolide, a lincosamide, an aminoglycoside, a tetracycline, a polypeptide antibiotic, a sulfonamide, and a glycopeptide, e.g., vancomycin, daptomycin, teicoplanin, and oritavancin.

In other embodiments, the present invention provides methods of treating a bacterial infection that involve administering a synergistic combination of a subject synthetic CXCR3 ligand and a second therapeutic agent. As used herein, a "synergistic combination" of a subject synthetic CXCR3 ligand and a second therapeutic agent is a combined dosage that is more effective in the therapeutic or prophylactic treatment of a bacterial infection than the incremental improvement in treatment outcome that could be predicted or expected from a merely additive combination of (i) the therapeutic or prophylactic benefit of a subject synthetic CXCR3 ligand when administered at that same dosage as a monotherapy and (ii) the therapeutic or prophylactic benefit of the second therapeutic agent when administered at the same dosage as a monotherapy.

### EXPERIMENTAL USES

A subject synthetic CXCR3 ligand is useful as a reagent in an *in vitro* or *in vivo* model system to study the biology of CXCR3; and to analyze effects of CXCR3 agonists and antagonists.

### Animal model of tumorigenesis

In some embodiments, a synthetic CXCR3 ligand is utilized to inhibit tumor growth in a non-human animal model of tumorigenesis. Any non-human animal model of tumorigenesis is suitable for use. An exemplary model is discussed in U.S. Patent No. 6,491,906. The model provides for an assessment of tumorigenesis, spontaneous metastasis and experimental lung colonization. A human non-small cell lung carcinoma (NSCLC) cell line is used. Either intact NSCLC tumors or cell lines may be used. Tumor growth is assessed by tumor size and mass, while spontaneous metastasis and lung colonization (experimental metastasis) is determined by histopathologic analysis of the lungs. In this system, a synthetic CXCR3 ligand can be used as a positive control for tumor growth inhibition activity. In addition, the system can be used to screen for agonists or antagonists of synthetic CXCR3 ligand activity.

The human NSCLC/SCID mouse model particularly involves the use of SCID mice of between the ages of 4 to 6 weeks. SCID mice should only be used if their serum Ig is <1 µg/ml. Human NSCLC/SCID mice chimera receive 20 µl of anti-asialo GM1 (aASGM1; Wako Chemicals, Dallas Tex.) by tail vein 24 hours prior to tumor implantation. This therapy removes host-derived NK cells.

Using intact human NSCLC, 1 mm³ specimens (grossly devoid of necrosis and weighed) are placed subcutaneously into the bilateral flank regions of a cohort group of SCID mice. Using the NSCLC cell lines (Calu-6, A549, Calu-1, and Calu-3), semiconfluent grown tumor cells are harvested and a cohort group of SCID are given 10⁶ cells and 5x10⁵ cells in 100 µl of PBS injected into bilateral flank regions and tail vein, respectively. At least one group of SCID mice form the treatment group and are administered a subject synthetic CXCR3 ligand. All mice are monitored daily for both evidence of illness and measurement of tumor size by digital engineers calipers.

Animals are sacrificed on a weekly basis for 16 weeks or sooner if the tumor size reaches 3 cm or the animals appear ill. Animals that appear ill are sacrificed, necropsy performed, and excluded from the study if their illness is for reasons other than tumor burden. At time of sacrifice, tumors in the subcutaneous location are measured and weighed. The experimental lung tumor colonization or spontaneous lung metastasis is then determined.

The administration of a subject synthetic CXCR3 ligand will have a significant attenuating effect on tumor growth within SCID mice. Thus, the tumor growth inhibition activity of synthetic CXCR3 ligand can be used as a positive control for comparison to the tumor growth inhibition activity of a candidate anticancer compound. Alternatively, the system can be used to evaluate the activity of a candidate agonist or antagonist of the tumor growth inhibition activity of synthetic CXCR3 ligand.

### Endothelial cell chemotaxis assay

A subject synthetic CXCR3 ligand can also be used in the evaluation of candidate agents for endothelial cell chemotactic activity. An endothelial cell chemotaxis assay is performed in 48-well, blind well chemotaxis chambers (Nucleopore Corp., Maryland). Nucleopore chemotaxis membranes (5 micron pore size) are prepared by soaking them sequentially in 3% acetic acid overnight and for 2 hours in 0.1 mg/ml gelatin. Membranes are rinsed in sterile water, dried under sterile air, and stored at room temperature for up to 1 month. Bovine adrenal gland capillary endothelial cells (BCE), maintained in gelatin-coated flasks in DME with 10% FBS are used as the target cells. Twenty four hours before use, BCE are starved in DME with 0.1% BSA. Twenty five microliters of cells, suspended at a concentration of 1 x 10⁶ cells per ml in DME with 0.1% BSA are dispensed into each of the bottom wells. A chemotaxis membrane is positioned atop the bottom wells, chambers are sealed, inverted, and incubated for 2 hours to allow cells to adhere to the membrane. Chambers are then reinverted, 50 ml test media is dispensed into the top wells and reincubated for an additional 2 hours. Membranes are then fixed and stained with Diff-Quick staining kit (American Scientific Products) to enumerate membrane-bound cells, and cells that had migrated through the membrane to the opposite surface are counted.

The presence of a subject synthetic CXCR3 ligand in the test media will induce cell migration across the chamber membrane. Thus, a subject synthetic CXCR3 ligand can be used as a positive control in the system. Alternatively, the system can be used to evaluate candidate agonists or antagonists of the chemotactic activity of a synthetic CXCR3 ligand.

### In vivo angiogenesis assay

In addition, a synthetic CXCR3 ligand can be used in the evaluation of the anti-angiogenic activity of a candidate agent in an *in vivo* model of angiogenesis. The well-characterized corneal micropocket model in the rat is suitable for use. For example, 5 mg total protein of a test sample is combined with a equal volume of sterile Hydron casting solution, and 5 ml aliquots are pipetted onto the surface of 1 mm Teflon rods glued to the surface of a glass petri dish. Pellets are air-dried in a laminar flow hood (1 hour) and refrigerated overnight. Prior to implantation pellets are rehydrated with a drop of lactated ringers solution.

Animals are anesthetized with metofane and injected with sodium pentobarbital intraperitoneally. A retrobulbar injection of 0.1 ml of 2% lidocaine is made before intracorneal implantation of the Hydron pellet into a surgically created intracorneal pocket approximately 1.5 mm from the limbus. The animals are examined daily with a stereomicroscope. Seven days after implantation, animals are re-anesthetized and perfused sequentially with lactated Ringers solution followed by colloidal carbon. Corneas are harvested, flattened and photographed.

Positive neovascularization responses are recorded only if sustained directional ingrowth of capillary sprouts and hairpin loops towards the implant are observed. Negative responses are recorded when either no growth was observed or when only an occasional sprout or hairpin loop displaying no evidence of sustained growth was detected.

The presence of a synthetic CXCR3 ligand will inhibit the activity of angiogenic agents in the test sample. Thus, a synthetic CXCR3 ligand can be used as a positive control for evaluation of candidate angiogenesis inhibitors in this system. Alternatively, the system can be used to evaluate the activity of a candidate agonist or antagonist of the anti-angiogenic activity of a synthetic CXCR3 ligand.

### Type I interferon receptor agonists

As discussed above, in some embodiments a Type I interferon receptor agonist is administered in combination therapy with a synthetic CXCR3 ligand. Type I interferon receptor agonists include an IFN-α; an IFN-β; an IFN-tau; an IFN-ω; antibody agonists specific for a Type I interferon receptor; and any other agonist of Type I interferon receptor, including non-polypeptide agonists.

### Interferon-alpha (IFN-α)

The term "interferon-alpha" as used herein refers to a family of related polypeptides that inhibit viral replication and cellular proliferation and modulate immune response. The term "IFN-α" includes IFN-α polypeptides that are naturally occurring; non-naturally-occurring IFN-α polypeptides; and analogs of naturally occurring or non-naturally occurring IFN-α that retain antiviral activity of a parent naturally-occurring or non-naturally occurring IFN-α.

Suitable alpha interferons include, but are not limited to, naturally-occurring IFN-α (including, but not limited to, naturally occurring IFN-α2a, IFN-α2b); recombinant interferon alpha-2b such as Intron®A interferon available from Schering Corporation, Kenilworth, N.J.; recombinant interferon alpha-2a such as Roferon® interferon available from Hoffmann-La Roche, Nutley, N. J.; recombinant interferon alpha-2C such as Berofor® alpha 2 interferon available from Boehringer Ingelheim Pharmaceutical, Inc., Ridgefield, Conn.; interferon alpha-n1, a purified blend of natural alpha interferons such as Sumiferon available from Sumitomo, Japan or as Wellferon® interferon alpha-n1 (INS) available from the Glaxo-Wellcome Ltd., London, Great Britain; and interferon alpha-n3 a mixture of natural alpha interferons made by Interferon Sciences and available from the Purdue Frederick Co., Norwalk, Conn., under the Alferon® Tradename.

The term "IFN-α," as used herein, also encompasses hybrid IFN-α. As used herein, the term "hybrid IFN-α" refers to a non-naturally-occurring polypeptide, which includes those amino acid residues that are common to all naturally-occurring human leukocyte IFN-α subtype sequences and which includes, at one or more of those positions where there is no amino acid common to all subtypes, an amino acid which predominantly occurs at that position, provided that at any such position where there is no amino acid common to all subtypes, the polypeptide excludes any amino acid residue which is not present in at least one naturally-occurring subtype. Amino acid residues that are common to all naturally-occurring human leukocyte IFN-α subtype sequences ("common amino acid residues"), and amino acid residues that occur predominantly at non-common residues ("hybrid amino acid residues") are known in the art.

The term "IFN-α" also encompasses hybrid IFN-α. Hybrid IFN-α (also referred to as "CIFN" and "IFN-con" and "hybrid interferon") encompasses but is not limited to the amino acid sequences designated IFN-con₁, IFN-con₂ and IFN-con₃ which are disclosed in U.S. Pat. Nos. 4,695,623 and 4,897,471; and hybrid interferon as defined by determination of a hybrid sequence of naturally occurring interferon alphas (e.g., Infergen®, InterMune, Inc., Brisbane, Calif.). IFN-con₁ is the hybrid interferon agent in the Infergen® alfacon-1 product. The Infergen® hybrid interferon product is referred to herein by its brand name (Infergen®) or by its generic name (interferon alfacon-1). DNA sequences encoding IFN-con may be synthesized as described in the aforementioned patents or other standard methods. Use of CIFN is of particular interest.

Also suitable for use in the present invention are fusion polypeptides comprising an IFN-α and a heterologous polypeptide. Suitable IFN-α fusion polypeptides include, but are not limited to, Albuferon-alpha™ (a fusion product of human albumin and IFN-α; Human Genome Sciences; see, e.g., Osborn et al. (2002) J. Pharmacol. Exp. Therap. 303:540-548). Also suitable for use in the present invention are gene-shuffled forms of IFN-α. See., e.g., Masci et al. (2003) Curr. Oncol. Rep. 5:108-113.

IFN-α polypeptides can be produced by any known method. DNA sequences encoding IFN-con may be synthesized as described in the above-mentioned patents or other standard methods. In many embodiments, IFN-α polypeptides are the products of expression of manufactured DNA sequences transformed or transfected into bacterial hosts, e.g., *E. coli*, or in eukaryotic host cells (e.g., yeast; mammalian cells, such as CHO cells; and the like). In these embodiments, the IFN-α is "recombinant IFN-α." Where the host cell is a bacterial host cell, the IFN-α is modified to comprise an N-terminal methionine. IFN-α produced in *E. coli* is generally purified by procedures known to those skilled in the art and generally described in Klein et al. ((1988) J. Chromatog. 454:205-215) for IFN-con₁.

Bacterially produced IFN-α may comprise a mixture of isoforms with respect to the N-terminal amino acid residue. For example, purified IFN-con may comprise a mixture of isoforms with respect to the N-terminal methionine status. For example, in some embodiments, an IFN-con comprises a mixture of N-terminal methionyl IFN-con, des-methionyl IFN-con with an unblocked N-terminus, and des-methionyl IFN-con with a blocked N-terminus. As one non-limiting example, purified IFN-con₁ comprises a mixture of methionyl IFN-con1 des-methionyl IFN-con₁ and des-methionyl IFN-con₁ with a blocked N-terminus. Klein et al. ((1990) Arch. Biochemistry & Biophys. 276:531-537). Alternatively, IFN-con may comprise a specific, isolated isoform. Isoforms of IFN-con are separated from each other by techniques such as isoelectric focusing which are known to those skilled in the art.

It is to be understood that IFN-α as described herein may comprise one or more modified amino acid residues, e.g., glycosylations, chemical modifications, and the like.

### PEGylated IFN-α

The term "IFN-α" also encompasses derivatives of IFN-α that are derivatized (e.g., are chemically modified) to alter certain properties such as serum half-life. As such, the term "IFN-α" includes glycosylated IFN-α; IFN-α derivatized with polyethylene glycol ("PEGylated IFN-α"); and the like. PEGylated IFN-α, and methods for making same, is discussed in, e.g., U.S. Patent Nos. 5,382,657; 5,981,709; and 5,951,974. PEGylated IFN-α encompasses conjugates of PEG and any of the above-described IFN-α molecules, including, but not limited to, PEG conjugated to interferon alpha-2a (Roferon, Hoffman La-Roche, Nutley, N.J.), interferon alpha 2b (Intron, Schering-Plough, Madison, N.J.), interferon alpha-2c (Berofor Alpha, Boehringer Ingelheim, Ingelheim, Germany); and hybrid interferon as defined by determination of a hybrid sequence of naturally occurring interferon alphas (Infergen®, InterMune, Inc., Brisbane, Calif.).

Any of the above-mentioned IFN-α polypeptides can be modified with one or more polyethylene glycol moieties, i.e., PEGylated. The PEG molecule of a PEGylated IFN-α polypeptide is conjugated to one or more amino acid side chains of the IFN-α polypeptide. In some embodiments, the PEGylated IFN-α contains a PEG moiety on only one amino acid. In other embodiments, the PEGylated IFN-α contains a PEG moiety on two or more amino acids, e.g., the IFN-α contains a PEG moiety attached to two, three, four, five, six, seven, eight, nine, or ten different amino acid residues.

IFN-α may be coupled directly to PEG (i.e., without a linking group) through an amino group, a sulfhydryl group, a hydroxyl group, or a carboxyl group.

In some embodiments, the PEGylated IFN-α is PEGylated at or near the amino terminus (N-terminus) of the IFN-α polypeptide, e.g., the PEG moiety is conjugated to the IFN-α polypeptide at one or more amino acid residues from amino acid 1 through amino acid 4, or from amino acid 5 through about 10.

In other embodiments, the PEGylated IFN-α is PEGylated at one or more amino acid residues from about 10 to about 28.

In other embodiments, the PEGylated IFN-α is PEGylated at or near the carboxyl terminus (C-terminus) of the IFN-α polypeptide, e.g., at one or more residues from amino acids 156-166, or from amino acids 150 to 155.

In other embodiments, the PEGylated IFN-α is PEGylated at one or more amino acid residues at one or more residues from amino acids 100-114.

Selection of the attachment site of polyethylene glycol on the IFN-α is determined by the role of each of the sites within the receptor-binding and/or active site domains of the protein, as would be known to the skilled artisan. In general, amino acids at which PEGylation is to be avoided include amino acid residues from amino acid 30 or amino acid 40; and amino acid residues from amino acid 113 to amino acid 149.

In some embodiments, PEG is attached to IFN-α via a linking group. The linking group is any biocompatible linking group, where "biocompatible" indicates that the compound or group is non-toxic and may be utilized *in vitro* or *in vivo* without causing injury, sickness, disease, or death. PEG can be bonded to the linking group, for example, via an ether bond, an ester bond, a thiol bond or an amide bond. Suitable biocompatible linking groups include, but are not limited to, an ester group, an amide group, an imide group, a carbamate group, a carboxyl group, a hydroxyl group, a carbohydrate, a succinimide group (including, for example, succinimidyl succinate (SS), succinimidyl propionate (SPA), succinimidyl carboxymethylate (SCM), succinimidyl succinamide (SSA) or N-hydroxy succinimide (NHS)), an epoxide group, an oxycarbonylimidazole group (including, for example, carbonyldimidazole (CDI)), a nitro phenyl group (including, for example, nitrophenyl carbonate (NPC) or trichlorophenyl carbonate (TPC)), a trysylate group, an aldehyde group, an isocyanate group, a vinylsulfone group, a tyrosine group, a cysteine group, a histidine group or a primary amine.

Methods for making succinimidyl propionate (SPA) and succinimidyl butanoate (SBA) ester-activated PEGs are described in U.S. Pat. No. 5,672,662 (Harris, et al.) and WO 97/03106.

Methods for attaching a PEG to an IFN-α polypeptide are known in the art, and any known method can be used. See, for example, by Park et al, Anticancer Res., 1:373-376 (1981); Zaplipsky and Lee, Polyethylene Glycol Chemistry: Biotechnical and Biomedical Applications, J. M. Harris, ed., Plenum Press, NY, Chapter 21 (1992); and U.S. Patent No. 5,985,265; U.S. Pat. No. 5,672,662 (Harris, et al.) and WO 97/03106.

Pegylated IFN-α, and methods for making same, are discussed in, e.g., U.S. Patent Nos. 5,382,657; 5,981,709; 5,985,265; and 5,951,974. Pegylated IFN-α encompasses conjugates of PEG and any of the above-described IFN-α molecules, including, but not limited to, PEG conjugated to interferon alpha-2a (Roferon, Hoffman LaRoche, Nutley, N.J.), where PEGylated Roferon is known as PEGASYS® (Hoffman LaRoche); interferon alpha 2b (Intron, Schering-Plough, Madison, N.J.), where PEGylated Intron is known as PEG-INTRON® (Schering-Plough); interferon alpha-2c (Berofor Alpha, Boehringer Ingelheim, Ingelheim, Germany); and hybrid interferon (CIFN) as defined by determination of a hybrid sequence of naturally occurring interferon alphas (Infergen, Amgen, Thousand Oaks, Calif.), where PEGylated Infergen is referred to as PEG-INFERGEN®.

Generally, the PEG moiety is linked to a surface-exposed lysine ("lys") residue. Whether a lysine is surface exposed can be determined using any known method. Generally, analysis of hydrophilicity (e.g., Kyte-Doolittle and Hoppe-Woods analysis) and/or predicted surface-forming regions (e.g., Emini surface-forming probability analysis) is carried out using appropriate computer programs, which are well known to those skilled in the art. Suitable computer programs include PeptideStructure, and the like. Alternatively, NMR investigations can identify the surface accessible residues by virtue of the chemical shift of the protons of a specific functional group in the spectrum. In other cases, the inaccessibility or accessibility of residues to solvents or environment can be assessed by fluorescence. In yet other cases, the surface exposure of accessible lysines can be ascertained by the chemical reactivity to water soluble reagents e.g., Trinitrobenzene sulfonate or TNBS, and like measurements.

In many embodiments, the PEG is a monomethoxy PEG molecule that reacts with primary amine groups on the IFN-α polypeptide. Methods of modifying polypeptides with monomethoxy PEG via reductive alkylation are known in the art. See, e.g., Chamow et al. (1994) Bioconj. Chem. 5:133-140.

In many embodiments, the PEG is a monomethoxy PEG molecule that reacts with primary amine groups on the IFN-α polypeptide. Methods of modifying polypeptides with monomethoxy PEG via reductive alkylation are known in the art. See, e.g., Chamow et al. (1994) Bioconj. Chem. 5:133-140.

In one non-limiting example, PEG is linked to IFN-α via an SPA linking group. SPA esters of PEG, and methods for making same, are described in U.S. Patent No. 5,672,662. SPA linkages provide for linkage to free amine groups on the IFN-α polypeptide.

For example, a PEG molecule is covalently attached via a linkage that comprises an amide bond between a propionyl group of the PEG moiety and the epsilon amino group of a surface-exposed lysine residue in the IFN-α polypeptide. Such a bond can be formed, e.g., by condensation of an α-methoxy, omega propanoic acid activated ester of PEG (mPEGspa).

In some embodiments, the PEGylated IFN-α is a monoPEGylated IFN-α. In other embodiments, the monoPEGylated IFN-α is an IFN-α polypeptide covalently linked to a single PEG moiety via a lysine residue or the N-terminal amino acid residue of the IFN-α polypeptide. In other embodiments, the monoPEGylated IFN-α is an IFN-α polypeptide covalently linked to a single PEG moiety via an amide bond between either the epsilon-amino group of a lysine residue or the alpha-amino group of the IFN-α polypeptide and an activated carboxyl group of the PEG moiety. In other embodiments, the monoPEGylated IFN-α is an IFN-α polypeptide covalently linked to a single, linear PEG moiety. In other embodiments, the monoPEGylated IFN-α is an IFN-α polypeptide covalently linked to a single, linear 30 kD PEG moiety. In other embodiments, the monoPEGylated IFN-α is an IFN-α polypeptide covalently linked to a single, linear 30 kD PEG moiety via an amide bond between the epsilon-amino group of a lysine residue or the alpha-amino group of the IFN-α polypeptide and an activated carboxyl group of the PEG moiety. In other embodiments, the monoPEGylated IFN-α is an IFN-α polypeptide covalently linked to a single, linear 30 kD PEG via an amide bond between the epsilon-amino group of a lysine residue or the alpha-amino group of the IFN-α polypeptide and an activated propionyl group of the PEG moiety. In other embodiments, the monoPEGylated IFN-α is an IFN-α polypeptide covalently linked to a single, linear monomethoxy-PEG (mPEG). In other embodiments, the monoPEGylated IFN-α is the product of a condensation reaction between an IFN-α polypeptide and a linear, succinimidyl propionate ester-activated 30 kD mPEG. In any of the foregoing methods using a PEGylated IFN-α, the IFN-α polypeptide can be a hybrid interferon (CIFN) polypeptide. In any of the foregoing methods using a PEGylated IFN-α, the IFN-α polypeptide can be a CIFN polypeptide that is interferon alfacon-1.

In some embodiments, the PEGylated IFN-α comprises CIFN PEGylated at the epsilon amino group of a lysine residue.

As one non-limiting example, one monopegylated CIFN conjugate preferred for use herein has a linear PEG moiety of about 30 kD attached via a covalent linkage to the CIFN polypeptide, where the covalent linkage is an amide bond between a propionyl group of the PEG moiety and the epsilon amino group of a surface-exposed lysine residue in the CIFN polypeptide, where the surface-exposed lysine residue is chosen from lys³¹, lys⁵⁰, lys⁷¹, lys⁸⁴, lys¹²¹, lys¹²², lys¹³⁴, lys¹³⁵, and lys¹⁶⁵, and the amide bond is formed by condensation of an α-methoxy, omega propanoic acid activated ester of PEG.

### Polyethylene glycol

polyethylene glycol suitable for conjugation to an IFN-α polypeptide is soluble in water at room temperature, and has the general formula R(O-CH₂-CH₂)ₙO-R, where R is hydrogen or a protective group such as an alkyl or an alkanol group, and where n is an integer from 1 to 1000. Where R is a protective group, it generally has from 1 to 8 carbons.

In many embodiments, PEG has at least one hydroxyl group, e.g., a terminal hydroxyl group, which hydroxyl group is modified to generate a functional group that is reactive with an amino group, e.g., an epsilon amino group of a lysine residue, a free amino group at the N-terminus of a polypeptide, or any other amino group such as an amino group of asparagine, glutamine, arginine, or histidine.

In other embodiments, PEG is derivatized so that it is reactive with free carboxyl groups in the IFN-α polypeptide, e.g., the free carboxyl group at the carboxyl terminus of the IFN-α polypeptide. Suitable derivatives of PEG that are reactive with the free carboxyl group at the carboxyl-terminus of IFN-α include, but are not limited to PEG-amine, and hydrazine derivatives of PEG (e.g., PEG-NH-NH₂).

In other embodiments, PEG is derivatized such that it comprises a terminal thiocarboxylic acid group, -COSH, which selectively reacts with amino groups to generate amide derivatives. Because of the reactive nature of the thio acid, selectivity of certain amino groups over others is achieved. For example, -SH exhibits sufficient leaving group ability in reaction with N-terminal amino group at appropriate pH conditions such that the ε-amino groups in lysine residues are protonated and remain non-nucleophilic. On the other hand, reactions under suitable pH conditions may make some of the accessible lysine residues to react with selectivity.

In other embodiments, the PEG comprises a reactive ester such as an N-hydroxy succinimidate at the end of the PEG chain. Such an N-hydroxysuccinimidate-containing PEG molecule reacts with select amino groups at particular pH conditions such as neutral 6.5-7.5. For example, the N-terminal amino groups may be selectively modified under neutral pH conditions. However, if the reactivity of the reagent were extreme, accessible-NH₂ groups of lysine may also react.

The PEG can be conjugated directly to the IFN-α polypeptide, or through a linker. In some embodiments, a linker is added to the IFN-α polypeptide, forming a linker-modified IFN-α polypeptide. Such linkers provide various functionalities, e.g., reactive groups such sulfhydryl, amino, or carboxyl groups to couple a PEG reagent to the linker-modified IFN-α polypeptide.

In some embodiments, the PEG conjugated to the IFN-α polypeptide is linear. In other embodiments, the PEG conjugated to the IFN-α polypeptide is branched. Branched PEG derivatives such as those described in U.S. Pat. No. 5,643,575, "star-PEG's" and multi-armed PEG's such as those described in Shearwater Polymers, Inc. catalog "Polyethylene Glycol Derivatives 1997-1998." Star PEGs are described in the art including, e.g., in U.S. Patent No. 6,046,305.

PEG having a molecular weight in a range of from about 2 kDa to about 100 kDa, is generally used, where the term "about," in the context of PEG, indicates that in preparations of polyethylene glycol, some molecules will weigh more, some less, than the stated molecular weight. For example, PEG suitable for conjugation to IFN-α has a molecular weight of from about 2 kDa to about 5 kDa, from about 5 kDa to about 10 kDa, from about 10 kDa to about 15 kDa, from about 15 kDa to about 20 kDa, from about 20 kDa to about 25 kDa, from about 25 kDa to about 30 kDa, from about 30 kDa to about 40 kDa, from about 40 kDa to about 50 kDa, from about 50 kDa to about 60 kDa, from about 60 kDa to about 70 kDa, from about 70 kDa to about 80 kDa, from about 80 kDa to about 90 kDa, or from about 90 kDa to about 100 kDa.

### Preparing PEG-IFN-α conjugates

As discussed above, the PEG moiety can be attached, directly or via a linker, to an amino acid residue at or near the N-terminus, internally, or at or near the C-terminus of the IFN-α polypeptide. Conjugation can be carried out in solution or in the solid phase.

### N-terminal linkage

Methods for attaching a PEG moiety to an amino acid residue at or near the N-terminus of an IFN-α polypeptide are known in the art. See, e.g., U.S. Patent No. 5,985,265.

In some embodiments, known methods for selectively obtaining an N-terminally chemically modified IFN-α are used. For example, a method of protein modification by reductive alkylation which exploits differential reactivity of different types of primary amino groups (lysine versus the N-terminus) available for derivatization in a particular protein can be used. Under the appropriate reaction conditions, substantially selective derivatization of the protein at the N-terminus with a carbonyl group containing polymer is achieved. The reaction is performed at pH which allows one to take advantage of the pKₐ differences between the ε-amino groups of the lysine residues and that of the α-amino group of the N-terminal residue of the protein. By such selective derivatization attachment of a PEG moiety to the IFN-α is controlled: the conjugation with the polymer takes place predominantly at the N-terminus of the IFN-α and no significant modification of other reactive groups, such as the lysine side chain amino groups, occurs.

### C-terminal linkage

N-terminal-specific coupling procedures such as described in U.S. Patent No. 5,985,265 provide predominantly monoPEGylated products. However, the purification procedures aimed at removing the excess reagents and minor multiply PEGylated products remove the N-terminal blocked polypeptides. In terms of therapy, such processes lead to significant increases in manufacturing costs. For example, examination of the structure of the well-characterized Infergen® Alfacon-1 CIFN polypeptide amino acid sequence reveals that the clipping is approximate 5% at the carboxyl terminus and thus there is only one major C-terminal sequence. Thus, in some embodiments, N-terminally PEGylated IFN-α is not used; instead, the IFN-α polypeptide is C-terminally PEGylated.

An effective synthetic as well as therapeutic approach to obtain mono PEGylated Infergen product is therefore envisioned as follows:

A PEG reagent that is selective for the C-terminal can be prepared with or without spacers. For example, polyethylene glycol modified as methyl ether at one end and having an amino function at the other end may be used as the starting material.

Preparing or obtaining a water-soluble carbodiimide as the condensing agent can be carried out. Coupling IFN-α (e.g., Infergen® Alfacon-1 CIFN or hybrid interferon) with a water-soluble carbodiimide as the condensing reagent is generally carried out in aqueous medium with a suitable buffer system at an optimal pH to effect the amide linkage. A high molecular weight PEG can be added to the protein covalently to increase the molecular weight.

The reagents selected will depend on process optimization studies. A non-limiting example of a suitable reagent is EDAC or 1-ethyl-3- (3-dimethylaminopropyl) carbodiimide. The water solubility of EDAC allows for direct addition to a reaction without the need for prior organic solvent dissolution. Excess reagent and the isourea formed as the by-product of the cross-linking reaction are both water-soluble and may easily be removed by dialysis or gel filtration. A concentrated solution of EDAC in water is prepared to facilitate the addition of a small molar amount to the reaction. The stock solution is prepared and used immediately in view of the water labile nature of the reagent. Most of the synthetic protocols in literature suggest the optimal reaction medium to be in pH range between 4.7 and 6.0. However the condensation reactions do proceed without significant losses in yields up to pH 7.5. Water may be used as solvent. In view of the contemplated use of Infergen, preferably the medium will be 2-(N-morpholino)ethane sulfonic acid buffer pre-titrated to pH between 4.7 and 6.0. However, 0.1M phosphate in the pH 7-7.5 may also be used in view of the fact that the product is in the same buffer. The ratios of PEG amine to the IFN-α molecule is optimized such that the C-terminal carboxyl residue(s) are selectively PEGylated to yield monoPEGylated derivative(s).

Even though the use of PEG amine has been mentioned above by name or structure, such derivatives are meant to be exemplary only, and other groups such as hydrazine derivatives as in PEG-NH-NH₂ which will also condense with the carboxyl group of the IFN-α protein, can also be used. In addition to aqueous phase, the reactions can also be conducted on solid phase. Polyethylene glycol can be selected from list of compounds of molecular weight ranging from 300-40000. The choice of the various polyethylene glycols will also be dictated by the coupling efficiency and the biological performance of the purified derivative in vitro and in vivo i.e., circulation times, anti viral activities etc.

Additionally, suitable spacers can be added to the C-terminal of the protein. The spacers may have reactive groups such as SH, NH₂ or COOH to couple with appropriate PEG reagent to provide the high molecular weight IFN-α derivatives. A combined solid/solution phase methodology can be devised for the preparation of C-terminal pegylated interferons. For example, the C-terminus of IFN-α is extended on a solid phase using a Gly-Gly-Cys-NH₂ spacer and then monopegylated in solution using activated dithiopyridyl-PEG reagent of appropriate molecular weights. Since the coupling at the C-terminus is independent of the blocking at the N-terminus, the envisioned processes and products will be beneficial with respect to cost (a third of the protein is not wasted as in N-terminal PEGylation methods) and contribute to the economy of the therapy to treat chronic hepatitis C infections, liver fibrosis etc.

There may be a more reactive carboxyl group of amino acid residues elsewhere in the molecule to react with the PEG reagent and lead to monoPEGylation at that site or lead to multiple PEGylations in addition to the -COOH group at the C-terminus of the IFN-α. It is envisioned that these reactions will be minimal at best owing to the steric freedom at the C-terminal end of the molecule and the steric hindrance imposed by the carbodiimides and the PEG reagents such as in branched chain molecules. It is therefore the preferred mode of PEG modification for Infergen and similar such proteins, native or expressed in a host system, which may have blocked N-termini to varying degrees to improve efficiencies and maintain higher *in vivo* biological activity.

Another method of achieving C-terminal PEGylation is as follows. Selectivity of C-terminal PEGylation is achieved with a sterically hindered reagent which excludes reactions at carboxyl residues either buried in the helices or internally in IFN-α. For example, one such reagent could be a branched chain PEG ∼40kd in molecular weight and this agent could be synthesized as follows:

OH₃C-(CH₂CH₂O)ₙ-CH₂CH₂NH₂ + Glutamic Acid i.e., HOCO-CH₂CH₂CH(NH2)-COOH is condensed with a suitable agent e.g., dicyclohexyl carbodiimide or water-soluble EDC to provide the branched chain PEG agent OH₃C-(CH₂CH₂O)ₙ-CH₂CH₂NHCOCH(NH₂)CH₂OCH₃-(CH₂CH₂O)ₙ-CH₂CH₂NHCOCH₂.

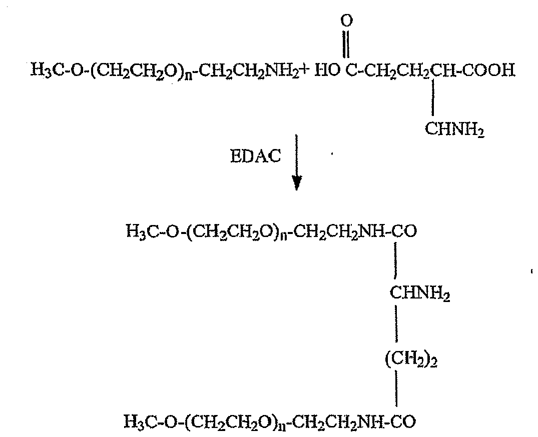

This reagent can be used in excess to couple the amino group with the free and flexible carboxyl group of IFN-α to form the peptide bond.

If desired, PEGylated IFN-α is separated from unPEGylated IFN-α using any known method, including, but not limited to, ion exchange chromatography, size exclusion chromatography, and combinations thereof. For example, where the PEG-IFN-α conjugate is a monoPEGylated IFN-α, the products are first separated by ion exchange chromatography to obtain material having a charge characteristic of monoPEGylated material (other multi-PEGylated material having the same apparent charge may be present), and then the monoPEGylated materials are separated using size exclusion chromatography.

### Mixed populations of IFN-α

In some embodiments, the IFN-α administered is a population of IFN-α polypeptides comprising PEGylated IFN-α polypeptides and non-PEGylated IFN-α polypeptides. Generally, a PEGylated IFN-α species represents from about 0.5% to about 99.5% of the total population of IFNα polypeptide molecules in a population, e.g, a given PEGylated IFN-α species represents about 0.5%, about 1%, about 2%, about 3%, about 4%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 99%, or about 99.5% of the total population of IFN-α polypeptide molecules in a population.

### IFN-β

The term interferon-beta ("IFN-β") includes IFN-β polypeptides that are naturally occurring; non-naturally-occurring IFN-β polypeptides; and analogs and variants of naturally occurring or non-naturally occurring IFN-β that retain antiviral activity of a parent naturally-occurring or non-naturally occurring IFN-β.

Any of a variety of beta interferons can be used in a subject combination therapy. Suitable beta interferons include, but are not limited to, naturally-occurring IFN-β; IFN-β1a, e.g., Avonex® (Biogen, Inc.), and Rebif® (Serono, SA); IFN-β1b (Betaseron®; Berlex); and the like.

The IFN-β formulation may comprise an N-blocked species, wherein the N-terminal amino acid is acylated with an acyl group, such as a formyl group, an acetyl group, a malonyl group, and the like. Also suitable for use is a hybrid IFN-β.

IFN-β polypeptides can be produced by any known method. DNA sequences encoding IFN-β may be synthesized using standard methods. In many embodiments, IFN-β polypeptides are the products of expression of manufactured DNA sequences transformed or transfected into bacterial hosts, e.g., *E. coli,* or in eukaryotic host cells (e.g., yeast; mammalian cells, such as CHO cells; and the like). In these embodiments, the IFN-β is "recombinant IFN-β." Where the host cell is a bacterial host cell, the IFN-β is modified to comprise an N-terminal methionine.

It is to be understood that IFN-β as described herein may comprise one or more modified amino acid residues, e.g., glycosylations, chemical modifications, and the like.

### IFN-tau

The term interferon-tau includes IFN-tau polypeptides that are naturally occurring; non-naturally-occurring IFN-tau polypeptides; and analogs and variants of naturally occurring or non-naturally occurring IFN-tau that retain antiviral activity of a parent naturally-occurring or non-naturally occurring IFN-tau.

Suitable tau interferons include, but are not limited to, naturally-occurring IFN-tau; Tauferon® (Pepgen Corp.); and the like.

IFN-tau may comprise an amino acid sequence as set forth in any one of GenBank Accession Nos. P15696; P56828; P56832; P56829; P56831; Q29429; Q28595; Q28594; S08072; Q08071; Q08070; Q08053; P56830; P28169; P28172; and P28171. The sequence of any known IFN-tau polypeptide may be altered in various ways known in the art to generate targeted changes in sequence. A variant polypeptide will usually be substantially similar to the sequences provided herein, *i.e.* will differ by at least one amino acid, and may differ by at least two but not more than about ten amino acids. The sequence changes may be substitutions, insertions or deletions. Conservative amino acid substitutions typically include substitutions within the following groups: (glycine, alanine); (valine, isoleucine, leucine); (aspartic acid, glutamic acid); (asparagine, glutamine); (serine, threonine); (lysine, arginine); or (phenylalanine, tyrosine).

Modifications of interest that may or may not alter the primary amino acid sequence include chemical derivatization of polypeptides, *e.g*., acetylation, or carboxylation; changes in amino acid sequence that introduce or remove a glycosylation site; changes in amino acid sequence that make the protein susceptible to PEGylation; and the like. Also included are modifications of glycosylation, *e.g*. those made by modifying the glycosylation patterns of a polypeptide during its synthesis and processing or in further processing steps; *e.g*. by exposing the polypeptide to enzymes that affect glycosylation, such as mammalian glycosylating or deglycosylating enzymes. Also embraced are sequences that have phosphorylated amino acid residues, *e.g*. phosphotyrosine, phosphoserine, or phosphothreonine.

The IFN-tau formulation may comprise an N-blocked species, wherein the N-terminal amino acid is acylated with an acyl group, such as a formyl group, an acetyl group, a malonyl group, and the like. Also suitable for use is a hybrid IFN-tau.

IFN-tau polypeptides can be produced by any known method. DNA sequences encoding IFN-tau may be synthesized using standard methods. In many embodiments, IFN-tau polypeptides are the products of expression of manufactured DNA sequences transformed or transfected into bacterial hosts, e.g., *E*. *coli,* or in eukaryotic host cells (e.g., yeast; mammalian cells, such as CHO cells; and the like). In these embodiments, the IFN-tau is "recombinant IFN-tau." Where the host cell is a bacterial host cell, the IFN-tau is modified to comprise an N-terminal methionine.

It is to be understood that IFN-tau as described herein may comprise one or more modified amino acid residues, e.g., glycosylations, chemical modifications, and the like.

### IFN-ω

The term interferon-omega ("IFN-ω") includes IFN-ω polypeptides that are naturally occurring; non-naturally-occurring IFN-ω polypeptides; and analogs and variants of naturally occurring or non-naturally occurring IFN-ω that retain antiviral activity of a parent naturally-occurring or non-naturally occurring IFN-ω.

Any known omega interferon can be used in a subject combination therapy. Suitable IFN-ω include, but are not limited to, naturally-occurring IFN-ω; recombinant IFN-ω, e.g., Biomed 510 (BioMedicines); and the like.

IFN-ω may comprise an amino acid sequence as set forth in GenBank Accession No. NP_002168; or AAA70091. The sequence of any know IFN-ω polypeptide may be altered in various ways known in the art to generate targeted changes in sequence. A variant polypeptide will usually be substantially similar to the sequences provided herein, *i.e*. will differ by at least one amino acid, and may differ by at least two but not more than about ten amino acids. The sequence changes may be substitutions, insertions or deletions. Conservative amino acid substitutions typically include substitutions within the following groups: (glycine, alanine); (valine, isoleucine, leucine); (aspartic acid, glutamic acid); (asparagine, glutamine); (serine, threonine); (lysine, arginine); or (phenylalanine, tyrosine).

Modifications of interest that may or may not alter the primary amino acid sequence include chemical derivatization of polypeptides, *e.g*., acetylation, or carboxylation; changes in amino acid sequence that introduce or remove a glycosylation site; changes in amino acid sequence that make the protein susceptible to PEGylation; and the like. Also included are modifications of glycosylation, *e.g*. those made by modifying the glycosylation patterns of a polypeptide during its synthesis and processing or in further processing steps; *e.g*. by exposing the polypeptide to enzymes that affect glycosylation, such as mammalian glycosylating or deglycosylating enzymes. Also embraced are sequences that have phosphorylated amino acid residues, *e.g*. phosphotyrosine, phosphoserine, or phosphothreonine.

The IFN-ω formulation may comprise an N-blocked species, wherein the N-terminal amino acid is acylated with an acyl group, such as a formyl group, an acetyl group, a malonyl group, and the like. Also suitable for use is a hybrid IFN-ω.

IFN-ω polypeptides can be produced by any known method. DNA sequences encoding IFN-ω may be synthesized using standard methods. In many embodiments, IFN-ω polypeptides are the products of expression of manufactured DNA sequences transformed or transfected into bacterial hosts, e.g.; *E*. *coli,* or in eukaryotic host cells (e.g., yeast; mammalian cells, such as CHO cells; and the like). In these embodiments, the IFN-ω is "recombinant IFN-ω." Where the host cell is a bacterial host cell, the IFN-ω is modified to comprise an N-terminal methionine.

It is to be understood that IFN-ω as described herein may comprise one or more modified amino acid residues, e.g., glycosylations, chemical modifications, and the like.

### Type II Interferon receptor agonists

Type II interferon receptor agonists include any naturally-occurring or non-naturally-occurring ligand of a human Type II interferon receptor which binds to and causes signal transduction via the receptor. Type II interferon receptor agonists include interferons, including naturally-occurring interferons, modified interferons, synthetic interferons, pegylated interferons, fusion proteins comprising an interferon and a heterologous protein, shuffled interferons; antibody specific for an interferon receptor; non-peptide chemical agonists; and the like.

A specific example of a Type II interferon receptor agonist is IFN-γ and variants thereof. While the present invention exemplifies use of an IFN-γ polypeptide, it will be readily apparent that any Type II interferon receptor agonist can be used in a subject method.

### Interferon-Gamma

The nucleic acid sequences encoding IFN-γ polypeptides may be accessed from public databases, e.g., Genbank, journal publications, etc. While various mammalian IFN-γ polypeptides are of interest, for the treatment of human disease, generally the human protein will be used. Human IFN-γ coding sequence may be found in Genbank, accession numbers X13274; V00543; and NM_000619. The corresponding genomic sequence may be found in Genbank, accession numbers J00219; M37265; and V00536. See, for example. Gray et al. (1982) Nature 295:501 (Genbank X13274); and Rinderknecht et al. (1984) J.B.C. 259:6790.

IFN-γ1b (Actimmune®; human interferon) is a single-chain polypeptide of 140 amino acids. It is made recombinantly in *E.coli* and is unglycosylated. Rinderknecht et al. (1984) J. Biol. Chem. 259:6790-6797. Recombinant IFN-γ as discussed in U.S. Patent No. 6,497,871 is also suitable for use herein.

The IFN-γ to be used in the methods of the present invention may be any of natural IFN-γs, recombinant IFN-γs and the derivatives thereof so far as they have an IFN-γ activity, particularly human IFN-γ activity. Human IFN-γ exhibits the antiviral and anti-proliferative properties characteristic of the interferons, as well as a number of other immunomodulatory activities, as is known in the art. Although IFN-γ is based on the sequences as provided above, the production of the protein and proteolytic processing can result in processing variants thereof. The unprocessed sequence provided by Gray et al., *supra*, consists of 166 amino acids (aa). Although the recombinant IFN-γ produced in *E*. *coli* was originally believed to be 146 amino acids, (commencing at amino acid 20) it was subsequently found that native human IFN-γ is cleaved after residue 23, to produce a 143 aa protein, or 144 aa if the terminal methionine is present, as required for expression in bacteria. During purification, the mature protein can additionally be cleaved at the C terminus after reside 162 (referring to the Gray *et al*. sequence), resulting in a protein of 139 amino acids, or 140 amino acids if the initial methionine is present, *e.g*. if required for bacterial expression. The N-terminal methionine is an artifact encoded by the mRNA translational "start" signal AUG that, in the particular case of *E. coli* expression is not processed away. In other microbial systems or eukaryotic expression systems, methionine may be removed.

For use in the subject methods, any of the native IFN-γ peptides, modifications and variants thereof, or a combination of one or more peptides may be used. IFN-γ peptides of interest include fragments, and can be variously truncated at the carboxyl terminus relative to the full sequence. Such fragments continue to exhibit the characteristic properties of human gamma interferon, so long as amino acids 24 to about 149 (numbering from the residues of the unprocessed polypeptide) are present. Extraneous sequences can be substituted for the amino acid sequence following amino acid 155 without loss of activity. See, for example, U.S. Patent No. 5,690,925. Native IFN-γ moieties include molecules variously extending from amino acid residues 24-150; 24-151, 24-152; 24- 153, 24-155; and 24-157. Any of these variants, and other variants known in the art and having IFN-γ activity, may be used in the present methods.

The sequence of the IFN-γ polypeptide may be altered in various ways known in the art to generate targeted changes in sequence. A variant polypeptide will usually be substantially similar to the sequences provided herein, i.e., will differ by at least one amino acid, and may differ by at least two but not more than about ten amino acids. The sequence changes may be substitutions, insertions or deletions. Scanning mutations that systematically introduce alanine, or other residues, may be used to determine key amino acids. Specific amino acid substitutions of interest include conservative and non-conservative changes. Conservative amino acid substitutions typically include substitutions within the following groups: (glycine, alanine); (valine, isoleucine, leucine); (aspartic acid, glutamic acid); (asparagine, glutamine); (serine, threonine); (lysine, arginine); or (phenylalanine, tyrosine).

Modifications of interest that may or may not alter the primary amino acid sequence include chemical derivatization of polypeptides, e.g., acetylation, or carboxylation; changes in amino acid sequence that introduce or remove a glycosylation site; changes in amino acid sequence that make the protein susceptible to PEGylation; and the like. In one embodiment, the invention contemplates the use of IFN-γ variants with one or more non-naturally occurring glycosylation and/or pegylation sites that are engineered to provide glycosyl- and/or PEG-derivatized polypeptides with reduced serum clearance, such as the IFN-γ polypeptide variants described in International Patent Publication No. WO 01/36001 and WO 02/081507. Also included are modifications of glycosylation, e.g., those made by modifying the glycosylation patterns of a polypeptide during its synthesis and processing or in further processing steps; e.g., by exposing the polypeptide to enzymes that affect glycosylation, such as mammalian glycosylating or deglycosylating enzymes. Also embraced are sequences that have phosphorylated amino acid residues, e.g., phosphotyrosine, phosphoserine, or phosphothreonine.

Included for use in the subject invention are IFN-γ polypeptides that have been modified using ordinary chemical techniques so as to improve their resistance to proteolytic degradation, to optimize solubility properties, or to render them more suitable as a therapeutic agent. For examples, the backbone of the peptide may be cyclized to enhance stability (see Friedler et al. (2000) J. Biol. Chem. 275:23783-23789). Analogs may be used that include residues other than naturally occurring L-amino acids, e.g., D-amino acids or non-naturally occurring synthetic amino acids. The protein may be pegylated to enhance stability.

The polypeptides may be prepared by *in vitro* synthesis, using conventional methods as known in the art, by recombinant methods, or may be isolated from cells induced or naturally producing the protein. The particular sequence and the manner of preparation will be determined by convenience, economics, purity required, and the like. If desired, various groups may be introduced into the polypeptide during synthesis or during expression, which allow for linking to other molecules or to a surface. Thus cysteines can be used to make thioethers, histidines for linking to a metal ion complex, carboxyl groups for forming amides or esters, amino groups for forming amides, and the like.

The polypeptides may also be isolated and purified in accordance with conventional methods of recombinant synthesis. A lysate may be prepared of the expression host and the lysate purified using HPLC, exclusion chromatography, gel electrophoresis, affinity chromatography, or other purification technique. For the most part, the compositions which are used will comprise at least 20% by weight of the desired product, more usually at least about 75% by weight, preferably at least about 95% by weight, and for therapeutic purposes, usually at least about 99.5% by weight, in relation to contaminants related to the method of preparation of the product and its purification. Usually, the percentages will be based upon total protein.

### Type III interferon receptor agonists

In any of the above-described methods, the interferon receptor agonist is in some embodiments an agonist of a Type III interferon receptor (e.g., "a Type III interferon agonist"). Type III interferon agonists include an IL-28b polypeptide; and IL-28a polypeptide; and IL-29 polypeptide; antibody specific for a Type III interferon receptor; and any other agonist of Type III interferon receptor, including non-polypeptide agonists.

IL-28A, IL-28B, and IL-29 (referred to herein collectively as "Type III interferons" or "Type III IFNs") are described in Sheppard et al. (2003) Nature 4:63-68. Each polypeptide binds a heterodimeric receptor consisting ofIL-10 receptor β chain and an IL-28 receptor α. Sheppard et al. (2003), supra. The amino acid sequences of IL-28A, IL-28B, and IL-29 are found under GenBank Accession Nos. NP_742150, NP_742151, and NP_742152, respectively.

The amino acid sequence of a Type III IFN polypeptide may be altered in various ways known in the art to generate targeted changes in sequence. A variant polypeptide will usually be substantially similar to the sequences provided herein, *i.e*. will differ by at least one amino acid, and may differ by at least two but not more than about ten amino acids. The sequence changes may be substitutions, insertions or deletions. Scanning mutations that systematically introduce alanine, or other residues, may be used to determine key amino acids. Specific amino acid substitutions of interest include conservative and non-conservative changes. Conservative amino acid substitutions typically include substitutions within the following groups: (glycine, alanine); (valine, isoleucine, leucine); (aspartic acid, glutamic acid); (asparagine, glutamine); (serine, threonine); (lysine, arginine); or (phenylalanine, tyrosine).

Modifications of interest that may or may not alter the primary amino acid sequence include chemical derivatization of polypeptides, *e.g*., acetylation, or carboxylation; changes in amino acid sequence that introduce or remove a glycosylation site; changes in amino acid sequence that make the protein susceptible to PEGylation; and the like. Also included are modifications of glycosylation, *e.g*. those made by modifying the glycosylation patterns of a polypeptide during its synthesis and processing or in further processing steps; *e.g*. by exposing the polypeptide to enzymes that affect glycosylation, such as mammalian glycosylating or deglycosylating enzymes. Also embraced are sequences that have phosphorylated amino acid residues, *e.g*. phosphotyrosine, phosphoserine, or phosphothreonine.

Included for use in the subject invention are polypeptides that have been modified using ordinary chemical techniques so as to improve their resistance to proteolytic degradation, to optimize solubility properties, or to render them more suitable as a therapeutic agent. For examples, the backbone of the peptide may be cyclized to enhance stability (see Friedler et al. (2000) J. Biol. Chem. 275:23783-23789). Analogs may be used that include residues other than naturally occurring L-amino acids, *e.g*. D-amino acids or non-naturally occurring synthetic amino acids. The protein may be pegylated to enhance stability. The polypeptides may be fused to albumin.

The polypeptides may be prepared by *in vitro* synthesis, using conventional methods as known in the art, by recombinant methods, or may be isolated from cells induced or naturally producing the protein. The particular sequence and the manner of preparation will be determined by convenience, economics, purity required, and the like. If desired, various groups may be introduced into the polypeptide during synthesis or during expression, which allow for linking to other molecules or to a surface. Thus cysteines can be used to make thioethers, histidines for linking to a metal ion complex, carboxyl groups for forming amides or esters, amino groups for forming amides, and the like.

### Pirfenidone and Analogs Thereof

Pirfenidone (5-methyl-1-phenyl-2-(1H)-pyridone) and specific pirfenidone analogs are in some embodiments used in a subject combination therapy.

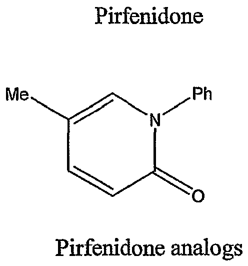

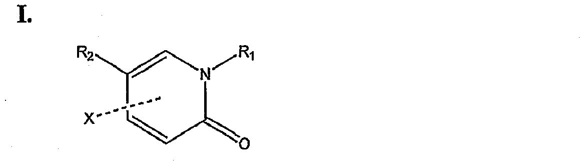

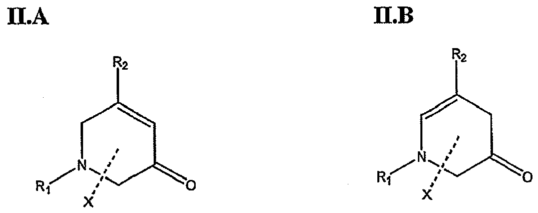

### Descriptions for Substituents R₁, R₂, X

**R₁**: carbocyclic (saturated and unsaturated), heterocyclic (saturated or unsaturated), alkyls (saturated and unsaturated). Examples include phenyl, benzyl, pyrimidyl, naphthyl, indolyl, pyrrolyl, furyl, thienyl, imidazolyl, cyclohexyl, piperidyl, pyrrolidyl, morpholinyl, cyclohexenyl, butadienyl, and the like.

R₁ can further include substitutions on the carbocyclic or heterocyclic moieties with substituents such as halogen, nitro, amino, hydroxyl, alkoxy, carboxyl, cyano, thio, alkyl, aryl, heteroalkyl, heteroaryl and combinations thereof, for example, 4-nitrophenyl, 3-chlorophenyl, 2,5-dinitrophenyl, 4-methoxyphenyl, 5-methyl-pyrrolyl, 2, 5-dichlorocyclohexyl, guanidinyl-cyclohexenyl and the like.

**R₂**: alkyl, carbocylic, aryl, heterocyclic. Examples include: methyl, ethyl, propyl, isopropyl, phenyl, 4-nitrophenyl, thienyl and the like.

**X**: may be any number (from 1 to 3) of substituents on the carbocyclic or heterocyclic ring. The substituents can be the same or different. Substituents can include hydrogen, alkyl, heteroalkyl, aryl, heteroaryl, halo, nitro, carboxyl, hydroxyl, cyano, amino, thio, alkylamino, haloaryl and the like.

The substituents may be optionally further substituted with 1-3 substituents from the group consisting of alkyl, aryl, nitro, alkoxy, hydroxyl and halo groups. Examples include: methyl, 2,3-dimethyl, phenyl, p-tolyl, 4-chlorophenyl, 4-nitrophenyl, 2,5-dichlorophenyl, furyl, thienyl and the like.

Specific Examples include those shown in Table 1:

**Table 1**

| **IA** | **IIB** |
|---|---|
| 5-Methyl-1-(2'-pyridyl)-2-(1H) pyridine, | 6-Methyl-1-phenyl-3-(1H) pyridone, |
| 6-Methyl-1-phenyl-2-(1H) pyridone, | 5-Methyl-1-p-tolyl-3-(1H) pyridone, |
| 5-Methyl-3-phenyl-1-(2'-thienyl)-2-(1H) pyridone, | 5-Methyl-1-(2'-naphthyl)-3-(1H) pyridone, |
| 5-Methyl-1-(2'-naphthyl)-2-(1H) pyridone, | 5-Methyl-1-phenyl-3-(1H) pyridone, |
| 5-Methyl-1-p-tolyl-2-(1H) pyridone, | 5-Methyl-1-(5'-quinolyl)-3-(1H) pyridone, |
| 5-Methyl-1-(1'naphthyl)-2-(1H) pyridone, | 5-Ethyl-1-phenyl-3-(1H) pyridone, |
| 5-Ethyl-1-phenyl-2-(1H) pyridone, | 5-Methyl-1-(4'-methoxyphenyl)-3-(1H) pyridone, |
| 5-Methyl-1-(5'-quinolyl)-2-(1H) pyridone, | 4-Methyl-1-phenyl-3-(1H) pyridone, |
| 5-Methyl-1-(4'-quinolyl)-2-(1H) pyridone, | 5-Methyl-1-(3'-pyridyl)-3-(1H) pyridone, |
| 5-Methyl-1-(4'-pyridyl)-2-(1H) pyridone, | 5-Methyl-1-(2'-Thienyl)-3-(1H) pyridone, |
| 3-Methyl-1-phenyl-2-(1H) pyridone, | 5-Methyl-1-(2'-pyridyl)-3-(1H) pyridone, |
| 5-Methyl-1-(4'-methoxyphenyl)-2-(1H) pyridone, | 5-Methyl-1-(2'-quinolyl)-3-(1H) pyridone, |
| 1-Phenyl-2-(1H) pyridone, | 1-Phenyl-3-(1H) pyridine, |
| 1,3-Diphenyl-2-(1H) pyridone, | 1-(2'-Furyl)-5-methyl-3-(1H) pyridone, |
| 1,3-Diphenyl-5-methyl-2-(1H) pyridone, | 1-(4'-Chlorophenyl)-5-methyl-3-(1H) pyridine. |
| 5-Methyl-1-(3'-trifluoromethylphenyl)-2-(1H)-pyridone, | |
| 3-Ethyl-1-phenyl-2-(1H) pyridone, | |
| 5-Methyl-1-(3'-pyridyl)-2-(1H) pyridone, | |
| 5-Methyl-1-(3-nitrophenyl)-2-(1H) pyridone, | |
| 3-(4'-Chlorophonyl)-5-Methyl-1-phenyl-2-(1H) pyridone, | |
| 5-Methyl-1-(2'-Thienyl)-2-(1H) pyridone, | |
| 5-Methyl-1-(2'-thiazolyl)-2-(1H) pyridone, | |
| 3,6-Dimethyl-1-phenyl-2-(1H) pyridone, | |
| 1-(4'Chlorophenyl)-5-Methyl-2-(1H) pyridone, | |
| 1-(2'-Imidazolyl)-5-Methyl-2-(1H) pyridone, | |
| 1-(4'-Nitrophenyl)-2-(1H) pyridone, | |
| 1-(2'-Furyl)-5-Methyl-2-(1H) pyridone, | |
| 1-Phenyl-3-(4'-chlorophenyl)-2-(1H) pyridine. | |

U.S. Pat. Nos. 3,974,281; 3,839,346; 4,042,699; 4,052,509; 5,310,562; 5,518,729; 5,716,632; and 6,090,822 describe methods for the synthesis and formulation of pirfenidone and specific pirfenidone analogs in pharmaceutical compositions suitable for use in the methods of the present invention.

### TNF antagonists

In some embodiments, a subject method involves administering a subject synthetic CXCR3 ligand and a TNF antagonist. Suitable TNF-α antagonists for use herein include agents that decrease the level of TNF-α synthesis, agents that block or inhibit the binding of TNF-α to a TNF-α receptor (TNFR), and agents that block or inhibit TNFR-mediated signal transduction. Unless otherwise expressly stated, every reference to a "TNF-α antagonist" or "TNF antagonist" herein will be understood to mean a TNF-α antagonist other than (i) pirfenidone and pirfenidone analogs and (ii) SAPK inhibitors.

As used herein, the terms "TNF receptor polypeptide" and "TNFR polypeptide" refer to polypeptides derived from TNFR (from any species) which are capable of binding TNF. Two distinct cell-surface TNFRs have described: Type II TNFR (or p75 TNFR or TNFRII) and Type I TNFR (or p55 TNFR or TNFRI). The mature full-length human p75 TNFR is a glycoprotein having a molecular weight of about 75-80 kilodaltons (kD). The mature full-length human p55 TNFR is a glycoprotein having a molecular weight of about 55-60 kD. Exemplary TNFR polypeptides are derived from TNFR Type I and/or TNFR type II. Soluble TNFR includes p75 TNFR polypeptide; fusions of p75 TNFR with heterologous fusion partners, e.g., the Fc portion of an immunoglobulin.

TNFR polypeptide may be an intact TNFR or a suitable fragment of TNFR. U.S. Pat. No. 5,605,690 provides examples of TNFR polypeptides, including soluble TNFR polypeptides, appropriate for use in the present invention. In many embodiments, the TNFR polypeptide comprises an extracellular domain of TNFR. In some embodiments, the TNFR polypeptide is a fusion polypeptide comprising an extracellular domain of TNFR linked to a constant domain of an immunoglobulin molecule. In other embodiments, the TNFR polypeptide is a fusion polypeptide comprising an extracellular domain of the p75 TNFR linked to a constant domain of an IgG1 molecule. In some embodiments, when administration to humans is contemplated, an Ig used for fusion proteins is human, e.g., human IgG1.

Monovalent and multivalent forms of TNFR polypeptides may be used in the present invention. Multivalent forms of TNFR polypeptides possess more than one TNF binding site. In some embodiments, the TNFR is a bivalent, or dimeric, form of TNFR. For example, as described in U.S. Pat. No. 5,605,690 and in Mohler et al., 1993, J. Immunol., 151:1548-1561, a chimeric antibody polypeptide with TNFR extracellular domains substituted for the variable domains of either or both of the immunoglobulin heavy or light chains would provide a TNFR polypeptide for the present invention. Generally, when such a chimeric TNFR:antibody polypeptide is produced by cells, it forms a bivalent molecule through disulfide linkages between the immunoglobulin domains. Such a chimeric TNFR:antibody polypeptide is referred to as TNFR:Fc.

In one embodiment, a subject method involves administration of an effective amount of the soluble TNFR ENBREL® etanercept. ENBREL® is a dimeric fusion protein consisting of the extracellular ligand-binding portion of the human 75 kilodalton (p75) TNFR linked to the Fc portion of human IgG1. The Fc component of ENBREL® contains the CH2 domain, the CH3 domain and hinge region, but not the CH1 domain of IgG1. ENBREL® is produced in a Chinese hamster ovary (CHO) mammalian cell expression system. It consists of 934 amino acids and has an apparent molecular weight of approximately 150 kilodaltons. Smith et al. (1990) Science 248:1019-1023; Mohler et al. (1993) J. Immunol. 151:1548-1561; U.S. Pat. No. 5,395,760; and U.S. Pat. No. 5,605,690.

Also suitable for use are monoclonal antibodies that bind TNF-α. Monoclonal antibodies include "humanized" mouse monoclonal antibodies; chimeric antibodies; monoclonal antibodies that are at least about 80%, at least about 90%, at least about 95%, or 100% human in amino acid sequence; and the like. See, e.g., WO 90/10077; WO 90/04036; and WO 92/02190. Suitable monoclonal antibodies include antibody fragments, such as Fv, F(ab')₂ and Fab; synthetic antibodies; artificial antibodies; phage display antibodies; and the like.

Examples of suitable monoclonal antibodies include infliximab (REMICADE®, Centocor); and adalimumab (HUMIRA™, Abbott) REMICADE® is a chimeric monoclonal anti-TNF-α antibody that includes about 25% mouse amino acid sequence and about 75% human amino acid sequence. REMICADE®comprises a variable region of a mouse monoclonal anti-TNF-α antibody fused to the constant region of a human IgG1. Elliott et al. (1993) Arthritis Rheum. 36:1681-1690; Elliott et al. (1994) Lancet 344:1105-1110; Baert et al. (1999) Gastroenterology 116:22-28. HUMIRA™ is a human, full-length IgG1 monoclonal antibody that was identified using phage display technology. Piascik (2003) J. Am. Pharm. Assoc. 43:327-328.

Methods to assess TNF antagonist activity are known in the art and exemplified herein. For example, TNF antagonist activity may be assessed with a cell-based competitive binding assay. In such an assay, radiolabeled TNF is mixed with serially diluted TNF antagonist and cells expressing cell membrane bound TNFR. Portions of the suspension are centrifuged to separate free and bound TNF and the amount of radioactivity in the free and bound fractions determined. TNF antagonist activity is assessed by inhibition of TNF binding to the cells in the presence of the TNF antagonist.

As another example, TNF antagonists may be analyzed for the ability to neutralize TNF activity in vitro in a bioassay using cells susceptible to the cytotoxic activity of TNF as target cells. In such an assay, target cells, cultured with TNF, are treated with varying amounts of TNF antagonist and subsequently are examined for cytolysis. TNF antagonist activity is assessed by a decrease in TNF-induced target cell cytolysis in the presence of the TNF antagonist.

### TGF-β Antagonists

In some embodiments, a subject method involves administering a subject synthetic CXCR3 ligand and a TGF-β antagonist. TGF-β antagonists suitable for use in a subject treatment method include agents that decrease the level of TGF-β synthesis, agents that block or inhibit the binding of TGF-β to a TGF-β receptor, and agents that block or inhibit TGF-β receptor-mediated signal transduction. As used herein, the term "TNF-β" includes any TNF-β subtype, including TGF-β1, TGF-β2, and TGF-β3. Suitable TGF-β antagonists include, but are not limited to, antibodies specific for TGF-β (including antibodies specific for a particular TGF-β subtype; and antibodies cross-reactive with two or more TGF-β subtypes); antibodies to TGF-β receptor; soluble TGF-β receptor; decorin; and agents that inhibit TGF-β signaling.

Suitable TGF-β, antagonists include antibodies specific for TNF-β. Antibodies specific for TGF-β are known in the art. See, e.g., U.S. Patent Nos. 5,783,185, 5,772,998, 5,674,843, 5,571,714, 5,462,925, and 5,426,098; WO 97/13844; and U.S. Patent Publication Nos 20030064069 and 20030091566. Non-limiting examples of suitable anti-TGF-β antibodies include CAT-152 (lerdelibumab; Trabio™; Cambridge Antibody Technology), a human anti-TGF-β2 monoclonal antibody; CAT-192 (metelimumab; Cambridge Antibody Technology), a human anti-TGF-β1 monoclonal antibody; and GC-1008 (Genzyme Corp.), a pan-specific human monoclonal antibody to TGF-β1, TGF-β2, and TGF-β3.

Suitable TGF-β antagonists include soluble TGF-β receptors. Soluble TGF-β receptors typically lack most or all of the transmembrane portion of a naturally-occurring TGF-β receptor, such that the protein is not membrane bound, yet retains TGF-β binding. Soluble TGF-β receptors include soluble fusion proteins comprising a portion of a TGF-β receptor fused in-frame to a heterologous (non-TGF-β receptor) protein (a "fusion partner"). Non-limiting examples of fusion partners are immunoglobulin Fc, poly-histidine, and the like. Soluble TGF-β receptors have been described in the art. See, e.g., Wang et al. (1999) Thorax 54:805-812; George et al. (1999) Proc. Natl. Acad. Sci. USA 96:12719-12724; Muraoka et al. (2002) J. Clin. Invest. 109:1551-1559; and Yata et al. (2002) Hepatology 35:1022-1030.

TGF-β antagonists include Gleevec™. Gleevec™ (also known as STI-571, or CGP57148B) has the chemical name 4-[(4-methyl-1-piperazinyl)methyl]-*N*-[4-methyl-3-[[4-(3-pyridinyl)-2-pyrimidinyl]amino-phenyl]benzamide methanesulfonate is commonly known as imatinib mesylate and sold under the trademark Gleevec™. Gleevec™ is a 2-phenylaminopyrimidine that targets the ATP-binding site of the kinase domain of Bcr-Abl tyrosine kinase (see, e.g. Druker et al. (1996) Nature Med. 2, 561; and Buchdunger et al. (1993) Proc. Natl. Acad. Sci. USA 92:2558-2562).

In certain embodiments, the agents are pyrimidine derivatives as described in U.S. Patent No. 5,521,184, the disclosure of which is herein incorporated by reference. In these embodiments, of interest are N-phenyl-2-pyrimidine-amine derivatives of formula (I):

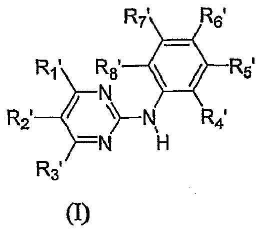

wherein

R_{1'} is 4-pyrazinyl, 1-methyl-1H-pyrrolyl, amino- or amino-lower alkyl-substituted phenyl wherein the amino group in each case is free, alkylated or acylated, 1H-indolyl or 1H-imidazolyl bonded at a five-membered ring carbon atom, or unsubstituted or lower alkyl-substituted pyridyl bonded at a ring carbon atom and unsubstituted or substituted at the nitrogen atom by oxygen, R_{2'} and R_{3'} are each independently of the other hydrogen or lower alkyl, one or two of the radicals R4', R5', R6', R7' and R8' are each nitro, fluoro-substituted lower alkoxy or a radical of formula (II):

-N(R_{9'})-C(=X)-(Y)ₖ-R₁₀ (II)

wherein

R_{9'} is hydrogen or lower alkyl,

X is oxo, thio, imino, N-lower alkyl-imino, hydroximino or O-lower alkyl-hydroximino,

Y is oxygen or the group NH,

*k* is 0 or 1 and

R₁₀ is an aliphatic radical having at least 5 carbon atoms, or an aromatic, aromatic-aliphatic, cycloaliphatic, cycloaliphatic-aliphatic, heterocyclic or heterocyclic-aliphatic radical,

and the remaining radicals R_{4'}, R_{5'}, R_{6'}, R_{7'} and R_{8'} are each independently of the others hydrogen, lower alkyl that is unsubstituted or substituted by free or alkylated amino, piperazinyl, piperidinyl, pyrrolidinyl or by morpholinyl, or lower alkanoyl, trifluoromethyl, free, etherified or esterifed hydroxy, free, alkylated or acylated amino or free or esterified carboxy,

and salts of such compounds having at least one salt-forming group.

In these embodiments:

1-Methyl-1H-pyrrolyl is preferably 1-methyl-1H-pyrrol-2-yl or 1-methyl-1H-pyrrol-3-yl.

Amino- or amino-lower alkyl-substituted phenyl R₁ wherein the amino group in each case is free, alkylated or acylated, is phenyl substituted in any desired position (ortho, meta or para) wherein an alkylated amino group is preferably mono- or di-lower alkylamino, for example dimethylamino, and the lower alkyl moiety of amino-lower alkyl is preferably linear C₁ -C₃ alkyl, such as especially methyl or ethyl.

1H-Indolyl bonded at a carbon atom of the five-membered ring is 1H-indol-2-yl or 1H-indol-3-yl.

Unsubstituted or lower alkyl-substituted pyridyl bonded at a ring carbon atom is lower alkyl-substituted or preferably unsubstituted 2-, or preferably 3- or 4-pyridyl, for example 3-pyridyl, 2-methyl-3-pyridyl, 4-methyl-3-pyridyl or 4-pyridyl. Pyridyl substituted at the nitrogen atom by oxygen is a radical derived from pyridine N-oxide, i.e., N-oxido-pyridyl, e.g. N-oxido-4-pyridyl.

Fluoro-substituted lower alkoxy is lower alkoxy carrying at least one, but preferably several, fluoro substituents, especially trifluoromethoxy or preferably 1,1,2,2-tetrafluoro-ethoxy.

When X is oxo, thio, imino, N-lower alkyl-imino, hydroximino or O-lower alkyl-hydroximino, the group C=X is, in the above order, a radical C=O, C=S, C=N-H, C=N-lower alkyl, C=N-OH or CN-O-lower alkyl, respectively. X is preferably oxo.

k is preferably 0, i.e., the group Y is not present.

Y, if present, is preferably the group NH.

The term "lower" within the scope of this text denotes radicals having up to and including 7, preferably up to and including 4 carbon atoms.

Lower alkyl R1', R2', R3' and R9' is preferably methyl or ethyl.

An aliphatic radical R₁₀ having at least 5 carbon atoms preferably has not more than 22 carbon atoms, generally not more than 10 carbon atoms, and is such a substituted or preferably unsubstituted aliphatic hydrocarbon radical, that is to say such a substituted or preferably unsubstituted alkynyl, alkenyl or preferably alkyl radical, such as C₅ -C₇ alkyl, for example n-pentyl. An aromatic radical R₁₀ has up to 20 carbon atoms and is unsubstituted or substituted, for example in each case unsubstituted or substituted naphthyl, such as especially 2-naphthyl, or preferably phenyl, the substituents preferably being selected from cyano, unsubstituted or hydroxy-, amino- or 4-methyl-piperazinyl-substituted lower alkyl, such as especially methyl, trifluoromethyl, free, etherified or esterified hydroxy, free, alkylated or acylated amino and free or esterified carboxy. In an aromatic-aliphatic radical R₁₀ the aromatic moiety is as defmed above and the aliphatic moiety is preferably lower alkyl, such as especially C₁ -C₂ alkyl, which is substituted or preferably unsubstituted, for example benzyl. A cycloaliphatic radical R₁₀ has especially up to 30, more especially up to 20, and most especially up to 10 carbon atoms, is mono- or poly-cyclic and is substituted or preferably unsubstituted, for example such a cycloalkyl radical, especially such a 5- or 6-membered cycloalkyl radical, such as preferably cyclohexyl. In a cycloaliphatic-aliphatic radical R₁₀ the cycloaliphatic moiety is as defined above and the aliphatic moiety is preferably lower alkyl, such as especially C₁-C₂ alkyl, which is substituted or preferably unsubstituted. A heterocyclic radical R₁₀ contains especially up to 20 carbon atoms and is preferably a saturated or unsaturated monocyclic radical having 5 or 6 ring members and 1-3 hetero atoms which are preferably selected from nitrogen, oxygen and sulfur, especially, for example, thienyl or 2-, 3- or 4-pyridyl, or a bi- or tri-cyclic radical wherein, for example, one or two benzene radicals are annellated (fused) to the mentioned monocyclic radical. In a heterocyclic-aliphatic radical R₁₀ the heterocyclic moiety is as defined above and the aliphatic moiety is preferably lower alkyl, such as especially C₁ -C₂ alkyl, which is substituted or preferably unsubstituted.

Etherified hydroxy is preferably lower alkoxy. Esterified hydroxy is preferably hydroxy esterified by an organic carboxylic acid, such as a lower alkanoic acid, or a mineral acid, such as a hydrohalic acid, for example lower alkanoyloxy or especially halogen, such as iodine, bromine or especially fluorine or chlorine.

Alkylated amino is, for example, lower alkylamino, such as methylamino, or di-lower alkylamino, such as dimethylamino. Acylated amino is, for example, lower alkanoylamino or benzoylamino.

Esterified carboxy is, for example, lower alkoxycarbonyl, such as methoxycarbonyl.

A substituted phenyl radical may carry up to 5 substituents, such as fluorine, but especially in the case of relatively large substituents is generally substituted by only from 1 to 3 substituents. Examples of substituted phenyl that may be given special mention are 4-chlorophenyl, pentafluoro-phenyl, 2-carboxy-phenyl, 2-methoxy-phenyl, 4-fluorophenyl, 4-cyanophenyl and 4-methyl-phenyl.

Salt-forming groups in a compound of formula (I) are groups or radicals having basic or acidic properties. Compounds having at least one basic group or at least one basic radical, for example a free amino group, a pyrazinyl radical or a pyridyl radical, may form acid addition salts, for example with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxybenzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid. When several basic groups are present mono- or poly-acid addition salts may be formed.

Compounds of formula (I) having acidic groups, for example a free carboxy group in the radical R₁₀, may form metal or ammonium salts, such as alkali metal or alkaline earth metal salts, for example sodium, potassium, magnesium or calcium salts, or ammonium salts with ammonia or suitable organic amines, such as tertiary monoamines, for example triethylamine or tri-(2-hydroxyethyl)-amine, or heterocyclic bases, for example N-ethylpiperidine or N,N'-dimethyl-piperazine. Compounds of formula (I) having both acidic and basic groups can form internal salts.

Of particular interest in these embodiments is a pyrimidine derivative in which R_{1'} is 3-pyridyl, R_{2'}, R_{3'}, R_{5'}, R_{6'}, and R_{8'} are each hydrogen, R₄, is methyl, and R_{7'} is a group of formula (II) in which R_{9'} is hydrogen, X is oxo, *k* is 0, and R₁₀ is 4-[(4-methyl-1-piperazinyl)methyl]phenyl. The mesylate salt of this compound having the chemical name 4-[(4-methyl-1-piperazinyl)methyl]-N-[4-methyl-3-[[4-(3-pyridinyl)-2-pyrimidinyl]aminophenyl]benzamide methanesulfonate is now commonly known as imatinib mesylate and sold under the trademark Gleevec™.

### Endothelin Receptor Antagonists

In some embodiments, a subject method involves administering a subject synthetic CXCR3 ligand and an endothelin receptor antagonist. Endothelin receptor antagonists suitable for use in the present invention include agents that decrease the level of endothelin synthesis, agents that block or inhibit the binding of endothelin to an endothelin receptor, and agents that block or inhibit endothelin receptor-mediated signal transduction. As used herein, the term "endothelin antagonist" or "endothelin receptor antagonist" refers to any agent that decreases the level of endothelin synthesis, any agent that blocks or inhibits the binding of endothelin to an endothelin receptor, and any agent that blocks or inhibits endothelin receptor-mediated signal transduction.

In some embodiments, an endothelin receptor antagonist is selective for endothelin A (ETA) receptors. In some embodiments, an endothelin receptor antagonist is selective four endothelin B (ETB) receptors. In other embodiments, an endothelin receptor antagonist is an antagonist of both ETA and ETB receptors.

Specific examples of endothelin antagonists useful in the present invention include, but are not limited to, atrasentan (ABT-627; Abbott Laboratories), Veletri™ (tezosentan; Actelion Pharmaceuticals, Ltd.), sitaxsentan (ICOS-Texas Biotechnology), enrasentan (GlaxoSmithKline), darusentan (LU135252; Myogen) BMS-207940 (Bristol-Myers Squibb), BMS-193884 (Bristol-Myers Squibb), BMS-182874 (Bristol-Myers Squibb), J-104132 (Banyu Pharmaceutical), VML 588/Ro 61-1790 (Vanguard Medica), T-0115 (Tanabe Seiyaku), TAK-044 (Takeda), BQ-788 (Banyu Pharmaceutical), BQ123, YM-598'(Yamanouchi Pharma), PD 145065 (Parke-Davis), A-127722 (Abbott Laboratories), A-192621 (Abbott Laboratories), A-182086 (Abbott Laboratories), TBC3711 (ICOS-Texas Biotechnology), BSF208075 (Myogen), S-0139 (Shionogi), TBC2576 (Texas Biotechnology), TBC3214 (Texas Biotechnology), PD156707 (Parke-Davis), PD180988 (Parke-Davis), ABT-546 (Abbott Laboratories), ABT-627 (Abbott Laboratories), SB247083 (GlaxoSmithKline), SB 209670 (GlaxoSmithKline); and an endothelin receptor antagonists discussed in the art, e.g., Davenport and Battistini (2002) Clinical Science 103:15-35, Wu-Wong et al. (2002) Clinical Science 103:1075-1115, and Luescher and Barton (2000) Circulation 102:2434-2440.

A suitable endothelin receptor antagonist is TRACLEER™ (bosentan; manufactured by Actelion Pharmaceuticals, Ltd.). TRACLEER™ is an orally active dual endothelin receptor antagonist, and blocks the binding of endothelin to both of its receptors endothelin receptor A and endothelin receptor B.

TRACLEER™ belongs to a class of highly substituted.pyrimidine derivatives, with no chiral centers. It is designated chemically as 4-tert-butyl-N-[6-(2-hydroxy-ethoxy)-5-(2-methoxy-phenoxy)-[2,2']-bipyrimidin-4-yl]-benzenesulfonamide monohydrate and has the following structural formula:

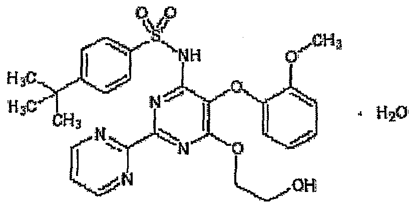

TRACLEER™ treatment is in some embodiments administered at a dose of 62.5 mg bid orally for 4 weeks, followed by a maintenance dose of 125 mg bid orally.

### SAPK inhibitors

A stress activated protein kinase (SAPK) inhibitor suitable for use in a subject combination therapy is an agent other than pirfenidone or a pirfenidone analog. SAPK inhibitors that are suitable for use herein are agents that inhibit enzymatic activity of a SAPK by at least about 10%, at least about 20%, at least about 25%, at least about 30%, at least about 35%, at least about 40%, at least about 50%, at least about 60%, at least about 70%, at least about 80%, or at least about 90%; or more; when compared with the enzymatic activity of the SAPK in the absence of the SAPK inhibitor.

Stress-activated protein kinase inhibitors that are suitable for use in a subject combination therapy include, but are not limited to, a 2-alkyl imidazole as disclosed in U.S. Patent No. 6,548,520; any of the 1,4,5-substituted imidazole compounds disclosed in U.S. Patent No. 6,489,325; 1,4,5-substituted imidazole compounds disclosed in U.S. Patent No. 6,569,871; heteroaryl aminophenyl ketone compounds disclosed in Published U.S. Patent Application No. 2003/0073832; pyridyl imidazole compounds disclosed in U.S. Patent No. 6,288,089; and heteroaryl aminobenzophenones disclosed in U.S. Patent No. 6,432,962. Also suitable for use are compounds disclosed in U.S. Patent No. 6,214,854. Also suitable for use are the heterocyclic compounds discussed in WO 99/61426; and the SAPK inhibitor compounds discussed in U.S. Patent Publication No. 20030149041. A further suitable SAPK inhibitor is BIRB796 (1-(5-tert-butyl-2-p-tolyl-2H-pyrazol-3-yl)-3-[4-(2-morpholin-4-yl-e-thoxy)-naphthalen-1-yl]-urea); see U.S. Patent no. 6,319,921. Another suitable SAPK inhibitor is 2(1H)-quinazolinone. Also suitable for use are pharmaceutically active derivatives, analogs, esters, prodrugs, and salts of any of the aforementioned SAPK inhibitors.

Methods of measuring SAPK activity are known in the art. One non-limiting example of an assay to measure enzymatic activity of a SAPK is as follows. In a final reaction volume of 25 µl, SAPK2a (p38α; 5-10 mU) is incubated with 25 mM Tris pH 7.5, 0.02 mM EGTA, 0.33 mg/ml myelin basic protein, 10 mM magnesium acetate and [γ-³³P-ATP] (specific activity approximately 500 cpm/pmol, concentration as required). The reaction is initiated by the addition of the Mg/ATP mix. After incubation for 40 minutes at room temperature, the reaction is stopped by addition of 5 µl of a 3% phosphoric acid solution. Ten µl of the reaction is then spotted onto a P30 filtermat and washed three times for 5 minutes in 75 mM phosphoric acid and once more in methanol prior to drying and scintillation counting.

### N-Acetylcysteine (NAC)

N-acetylcysteine (NAC) is a stable form of the sulfur amino acid L-cysteine. NAC is an anti-oxidant that scavenges H₂O₂ and other radicals. It is a precursor of glutathione (a major antioxidant), providing cysteine substrate for glutathione synthesis. NAC is commercially available as an over-the-counter nutritional supplement or nutraceutical product. Suitable NAC products for use herein include the NAC nutritional supplement products made by Source Naturals (1000 mg tablets), Biochem (750 mg tablets), Twinlab (600 mg tablets), Nutricology/Allergy Research Group (500 mg tablets), and the like. Such products can be purchased at minimal cost from health food stores and nutritional supplement retailers, such as General Nutrition Center (GNC).

### DOSAGES, FORMULATIONS, AND ROUTES OF ADMINISTRATION

An active agent (e.g., a subject synthetic CXCR3 ligand; at least a second therapeutic agent, such as a Type II interferon receptor agonist, a Type I interferon receptor agonist, pirfenidone or a pirfenidone analog, a TNF antagonist, a TGF-β antagonist, an endothelin receptor agonist, a SAPK inhibitor; etc.) is administered to an individual in need thereof in a formulation (e.g., in separate formulations) with a pharmaceutically acceptable excipient(s). A wide variety of pharmaceutically acceptable excipients are known in the art and need not be discussed in detail herein. Pharmaceutically acceptable excipients have been amply described in a variety of publications, including, for example, A. Gennaro (2000) "Remington: The Science and Practice of Pharmacy", 20th edition, Lippincott, Williams, & Wilkins; Pharmaceutical Dosage Forms and Drug Delivery Systems (1999) H.C. Ansel et al., eds 7th ed., Lippincott, Williams, & Wilkins; and Handbook of Pharmaceutical Excipients (2000) A.H. Kibbe et al., eds., 3rd ed. Amer. Pharmaceutical Assoc.

In the subject methods, the active agent(s) may be administered to the host using any convenient means capable of resulting in the desired therapeutic effect. Thus, the agent can be incorporated into a variety of formulations for therapeutic administration. More particularly, the agents of the present invention can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, solutions, suppositories, injections, inhalants and aerosols.

As such, administration of the agents can be achieved in various ways, including oral, buccal, rectal, parenteral, intraperitoneal, intradermal, intravenous, subcutaneous, intramuscular, intratumoral, transdermal, intratracheal, etc., administration. In some embodiments, two different routes of administration are used. For example, in some embodiments, a subject synthetic-CXCR3 ligand is administered by a route such as intramuscular, subcutaneous, or intravenous, and pirfenidone or pirfenidone analog is administered orally.

Subcutaneous administration of an active agent (e.g., a subject synthetic CXCR3 ligand; an additional therapeutic agent; etc.) is accomplished using standard methods and devices, e.g., needle and syringe, a subcutaneous injection port delivery system, and the like. See, e.g., U.S. Patent Nos. 3,547,119; 4,755,173; 4,531,937; 4,311,137; and 6,017,328. A combination of a subcutaneous injection port and a device for administration of an active agent (e.g., a subject synthetic CXCR3 ligand; an additional therapeutic agent; etc.) to a patient through the port is referred to herein as "a subcutaneous injection port delivery system." In some embodiments, subcutaneous administration is achieved by a combination of devices, e.g., bolus delivery by needle and syringe, followed by delivery using a continuous delivery system.

In some embodiments, an active agent (e.g., a subject synthetic CXCR3 ligand; an additional therapeutic agent; etc.) is delivered by a continuous delivery system. The term "continuous delivery system" is used interchangeably herein with "controlled delivery system" and encompasses continuous (e.g., controlled) delivery devices (e.g., pumps) in combination with catheters, injection devices, and the like, a wide variety of which are known in the art.

Mechanical or electromechanical infusion pumps can also be suitable for use with the present invention. Examples of such devices include those described in, for example, U.S. Pat. Nos. 4,692,147; 4,360,019; 4,487,603; 4,360,019; 4,725,852; 5,820,589; 5,643,207; 6,198,966; and the like. In general, the present methods of drug delivery can be accomplished using any of a variety of refillable, pump systems. Pumps provide consistent, controlled release over time. Typically, the agent (e.g., interferon receptor agonist) is in a liquid formulation in a drug-impermeable reservoir, and is delivered in a continuous fashion to the individual.

In one embodiment, the drug delivery system is an at least partially implantable device. The implantable device can be implanted at any suitable implantation site using methods and devices well known in the art. An implantation site is a site within the body of a subject at which a drug delivery device is introduced and positioned. Implantation sites include, but are not necessarily limited to a subdermal, subcutaneous, intramuscular, or other suitable site within a subject's body. Subcutaneous implantation sites are generally preferred because of convenience in implantation and removal of the drug delivery device.

Drug release devices suitable for use in the invention may be based on any of a variety of modes of operation. For example, the drug release device can be based upon a diffusive system, a convective system, or an erodible system (*e.g.*, an erosion-based system). For example, the drug release device can be an electrochemical pump, osmotic pump, an electroosmotic pump, a vapor pressure pump, or osmotic bursting matrix, e.g., where the drug is incorporated into a polymer and the polymer provides for release of drug formulation concomitant with degradation of a drug-impregnated polymeric material (*e.g.*, a biodegradable, drug-impregnated polymeric material). In other embodiments, the drug release device is based upon an electrodiffusion system, an electrolytic pump, an effervescent pump, a piezoelectric pump, a hydrolytic system, *etc.*

Drug release devices based upon a mechanical or electromechanical infusion pump can also be suitable for use with the present invention. Examples of such devices include those described in, for example, U.S. Pat. Nos. 4,692,147; 4,360,019; 4,487,603; 4,360,019; 4,725,852, and the like. In general, the present methods of drug delivery can be accomplished using any of a variety of refillable, non-exchangeable pump systems. Pumps and other convective systems are generally preferred due to their generally more consistent, controlled release over time. Osmotic pumps are particularly preferred due to their combined advantages of more consistent controlled release and relatively small size (see, *e.g.*, PCT published application no. WO 97/27840 and U.S. Pat. Nos. 5,985,305 and 5,728,396)). Exemplary osmotically-driven devices suitable for use in the invention include, but are not necessarily limited to, those described in U.S. Pat. Nos. 3,760,984; 3,845,770; 3,916,899; 3,923,426; 3,987,790; 3,995,631; 3,916,899; 4,016,880; 4,036,228; 4,111,202; 4,111,203; 4,203,440; 4,203,442; 4,210,139; 4,327,725; 4,627,850; 4,865,845; 5,057,318; 5,059,423; 5,112,614; 5,137,727; 5,234,692; 5,234,693; 5,728,396; and the like.

In some embodiments, the drug delivery device is an implantable device. The drug delivery device can be implanted at any suitable implantation site using methods and devices well known in the art. As noted infra, an implantation site is a site within the body of a subject at which a drug delivery device is introduced and positioned. Implantation sites include, but are not necessarily limited to a subdermal, subcutaneous, intramuscular, or other suitable site within a subject's body.

In some embodiments, an active agent (e.g., a subject synthetic CXCR3 ligand; an additional therapeutic agent; etc.) is delivered using an implantable drug delivery system, e.g., a system that is programmable to provide for administration of the active agent (e.g., a subject synthetic CXCR3 ligand; an additional therapeutic agent; etc.). Exemplary programmable, implantable systems include implantable infusion pumps. Exemplary implantable infusion pumps, or devices useful in connection with such pumps, are described in, for example, U.S. Pat. Nos. 4,350,155; 5,443,450; 5,814,019; 5,976,109; 6,017,328; 6,171,276; 6,241,704; 6,464,687; 6,475,180; and 6,512,954. A further exemplary device that can be adapted for the present invention is the Synchromed infusion pump (Medtronic).

In pharmaceutical dosage forms, an active agent (e.g., a subject synthetic CXCR3 ligand; an additional therapeutic agent; etc.) may be administered in the form of its pharmaceutically acceptable salts, or may also be used alone or in appropriate association, as well as in combination, with other pharmaceutically active compounds. The following methods and excipients are merely exemplary and are in no way limiting.

For oral preparations, an active agent (e.g., a subject synthetic CXCR3 ligand; an additional therapeutic agent; etc.) can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

An active agent (e.g., a subject synthetic CXCR3 ligand; an additional therapeutic agent; etc.) can be formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

Furthermore, an active agent (e.g., a subject synthetic CXCR3 ligand; an additional therapeutic agent; etc.) can be made into suppositories by mixing with a variety of bases such as emulsifying bases or water-soluble bases an active agent (e.g., a subject synthetic CXCR3 ligand; an additional therapeutic agent; etc.) can be administered rectally via a suppository. The suppository can include vehicles such as cocoa butter, carbowaxes and polyethylene glycols, which melt at body temperature, yet are solidified at room temperature.

Unit dosage forms for oral or rectal administration such as syrups, elixirs, and suspensions may be provided wherein each dosage unit, for example, teaspoonful, tablespoonful, tablet or suppository, contains a predetermined amount of the composition containing one or more active agents. Similarly, unit dosage forms for injection or intravenous administration may comprise the active agent(s) in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of an active agent (e.g., a subject synthetic CXCR3 ligand; an additional therapeutic agent; etc.) calculated in an amount sufficient to produce the desired effect in association with a pharmaceutically acceptable diluent, carrier or vehicle. The specifications an active agent (e.g., a subject synthetic CXCR3 ligand; an additional therapeutic agent; etc.) depend on the particular compound employed and the effect to be achieved, and the pharmacodynamics associated with each compound in the host.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

Where the administered agent is a subject synthetic CXCR3 ligand polypeptide, a polynucleotide encoding the subject synthetic CXCR3 ligand may be introduced into tissues or host cells by any number of routes, including viral infection, microinjection, or fusion of vesicles. Jet injection may also be used for intramuscular administration, as described by Furth et al. (1992), Anal Biochem 205:365-368. The DNA may be coated onto gold microparticles, and delivered intradermally by a particle bombardment device, or "gene gun" as described in the literature (see, for example, Tang et al. (1992), Nature 356:152-154), where gold microprojectiles are coated with the therapeutic DNA, then bombarded into skin cells.

In some embodiments, a second therapeutic agent is administered during the entire course of synthetic CXCR3 ligand treatment. Exemplary second therapeutic agents include one or more of pirfenidone or a pirfenidone analog; a Type I interferon receptor agonist; a Type II interferon receptor agonist; one or more antineoplastic agents; a TNF antagonist; a TGF-β antagonist; an endothelin receptor antagonist; and a SAPK inhibitor. In other embodiments, a second therapeutic agent is administered for a period of time that is overlapping with that of the synthetic CXCR3 ligand treatment, e.g., the second therapeutic agent treatment can begin before the synthetic CXCR3 ligand treatment begins and end before the synthetic CXCR3 ligand treatment ends; the second therapeutic agent treatment can begin after the synthetic CXCR3 ligand treatment begins and end after the synthetic CXCR3 ligand treatment ends; the second therapeutic agent treatment can begin after the synthetic CXCR3 ligand treatment begins and end before the synthetic CXCR3 ligand treatment ends; or the second therapeutic agent treatment can begin before the synthetic CXCR3 ligand treatment begins and end after the synthetic CXCR3 ligand treatment ends.

For example, in some embodiments, a second therapeutic agent is administered during the entire course of synthetic CXCR3 ligand treatment. In other embodiments, pirfenidone or a pirfenidone analog is administered for a period of time that is overlapping with that of the synthetic CXCR3 ligand treatment, e.g., the pirfenidone or pirfenidone analog treatment can begin before the synthetic CXCR3 ligand treatment begins and end before the synthetic CXCR3 ligand treatment ends; the pirfenidone or pirfenidone analog treatment can begin after the synthetic CXCR3 ligand treatment begins and end after the synthetic CXCR3 ligand treatment ends; the pirfenidone or pirfenidone analog treatment can begin after the synthetic CXCR3 ligand treatment begins and end before the synthetic CXCR3 ligand treatment ends; or the pirfenidone or pirfenidone analog treatment can begin before the synthetic CXCR3 ligand treatment begins and end after the synthetic CXCR3 ligand treatment ends.

Effective dosages of a subject synthetic CXCR3 ligand range from 0.1 µg to 1000 µg per dose, e.g., from about 0.1 µg to about 0.5 µg per dose, from about 0.5 µg to about 1.0 µg per dose, from about 1.0 µg per dose to about 5.0 µg per dose, from about 5.0 µg to about 10 µg per dose, from about 10 µg to about 20 µg per dose, from about 20 µg per dose to about 30 ug per dose, from about 30 µg per dose to about 40 µg per dose, from about 40 µg per dose to about 50 µg per dose, from about 50 µg per dose to about 60 µg per dose, from about 60 µg per dose to about 70 µg per dose, from about 70 µg to about 80 µg per dose, from about 80 µg per dose to about 100 µ per dose, from about 100 µg to about 150 µg per dose, from about 150 µg to about 200 µg per dose, from about 200 µg per dose to about 250 µg per dose, from about 250 µg to about 300 µg per dose, from about 300 µg to about 400 µg per dose, from about 400 µg to about 500 µg per dose, from about 500 µg to about 600 µg per dose, from about 600 µg to about 700 µg per dose, from about 700 µg to about 800 µg per dose, from about 800 µg to about 900 µg per dose, or from about 900 µg to about a 1000 µg per dose.

In some embodiments, effective dosages of a subject synthetic CXCR3 ligand are expressed as mg/kg body weight. In these embodiments, effective dosages of a subject synthetic CXCR3 ligand are from about 0.1 mg/kg body weight to about 10 mg/kg body weight, e.g., from about 0.1 mg/kg body weight to about 0.5 mg/kg body weight, from about 0.5 mg/kg body weight to about 1.0 mg/kg body weight, from about 1.0 mg/kg body weight to about 2.5 mg/kg body weight, from about 2.5 mg/kg body weight to about 5.0 mg/kg body weight, from about 5.0 mg/kg body weight to about 7.5 mg/kg body weight, or from about 7.5 mg/kg body weight to about 10 mg/kg body weight.

In many embodiments, a subject synthetic CXCR3 ligand is administered for a period of about 1 day to about 7 days, or about 1 week to about 2 weeks, or about 2 weeks to about 3 weeks, or about 3 weeks to about 4 weeks, or about 1 month to about 2 months, or about 3 months to about 4 months, or about 4 months to about 6 months, or about 6 months to about 8 months, or about 8 months to about 12 months, or at least one year, and may be administered over longer periods of time. The interferon receptor agonist can be administered tid, bid, qd, qod, biw, tiw, qw, qow, three times per month, once monthly, substantially continuously, or continuously.

In many embodiments, multiple doses of a subject synthetic CXCR3 ligand are administered. For example, an interferon receptor agonist is administered once per month, twice per month, three times per month, every other week (qow), once per week (qw), twice per week (biw), three times per week (tiw), four times per week, five times per week, six times per week, every other day (qod), daily (qd), twice a day (qid), or three times a day (tid), substantially continuously, or continuously, over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

### Synthetic CXCR3 ligand in combination therapy with pirfenidone

In some embodiments, a subject synthetic CXCR3 ligand is administered in combination therapy with pirdenidone for the treatment of cancer, a fibrotic disorder, or an angiogenic disorder. The methods generally involve administering to an individual in need thereof an effective amount of a synthetic CXCR3 ligand and an effective amount of pirfenidone or pirfenidone analog.

In general, effective dosages of pirfenidone or specific pirfenidone analogs can range from about 0.5 mg/kg/day to about 200 mg/kg/day, or at a fixed dosage of about 400 mg to about 3600 mg per day, or about 50 mg to about 5,000 mg per day, or about 100 mg to about 1,000 mg per day, administered orally, optionally in two or more divided doses per day. Other doses and formulations of pirfendone and pirfenidone analogs suitable for use in a subject method for the treatment of cancer are described in U.S. Pat. Nos. 3,974,281; 3,839,346; 4,042,699; 4,052,509; 5,310,562; 5,518,729; 5,716,632; and 6,090,822.

Those of skill in the art will readily appreciate that dose levels of pirfenidone or pirfenidone analog can vary as a function of the specific compound, the severity of the symptoms and the susceptibility of the subject to side effects. Preferred dosages for a given compound are readily determinable by those of skill in the art by a variety of means.

Pirfenidone (or a pirfenidone analog) can be administered daily, twice a day, or three times a day, or in divided daily doses ranging from 2 to 5 times daily over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

A subject synthetic CXCR3 ligand and pirfenidone (or pirfenidone analog) are generally administered in separate formulations. A subject synthetic CXCR3 ligand and pirfenidone (or pirfenidone analog) may be administered substantially simultaneously, or within about 30 minutes, about 1 hour, about 2 hours, about 4 hours, about 8 hours, about 16 hours, about 24 hours, about 36 hours, about 72 hours, about 4 days, about 7 days, or about 2 weeks of one another.

In one embodiment, the invention provides a method using an effective amount of a subject synthetic CXCR3 ligand and pirfenidone or a specific pirfenidone analog in the treatment of cancer, a fibrotic disorder, or an angiogenic disorder in a patient comprising administering to the patient a dosage of a subject synthetic CXCR3 ligand containing an amount of from about 0.1 µg to about 1000 µp per dose of a subject synthetic CXCR3 ligand, subcutaneously qd, qod, tiw, or biw, or per day substantially continuously or continuously, in combination with a dosage of pirfenidone or a specific pirfenidone analog containing an amount of about 100 mg to about 1,000 mg of drug per dose of pirfenidone or a specific pirfenidone analog orally qd, optionally in two or more divided doses per day, for the desired treatment duration.

In one embodiment, the invention provides a method using an effective amount of synthetic CXCR3 ligand and pirfenidone or a specific pirfenidone analog in the treatment of cancer, a fibrotic disorder, or an angiogenic disorder in a patient comprising administering to the patient a dosage of synthetic CXCR3 ligand containing an amount of from about 0.1 µg to about 1000 µg of drug per dose of synthetic CXCR3 ligand, subcutaneously qd, qod, tiw, or biw, or per day substantially continuously or continuously, in combination with a dosage of pirfenidone or a specific pirfenidone analog containing an amount of about 100 mg to about 1,000 mg of drug per dose orally qd, optionally in two or more divided doses per day, for the desired treatment duration.

In one embodiment, the invention provides a method using an effective amount of synthetic CXCR3 ligand and pirfenidone or a specific pirfenidone analog in the treatment of cancer, a fibrotic disorder, or an angiogenic disorder in a patient comprising administering to the patient a dosage of synthetic CXCR3 ligand containing an amount of from about 0.1 µg to about 1000 µg of drug per dose of synthetic CXCR3 ligand, intramuscularly qd, qod, tiw, or biw, or per day substantially continuously or continuously, in combination with a dosage of pirfenidone or a specific pirfenidone analog containing an amount of about 100 mg to about 1,000 mg of drug per dose orally qd, optionally in two or more divided doses per day, for the desired treatment duration.

In one embodiment, the invention provides a method using an effective amount of synthetic CXCR3 ligand and pirfenidone or a specific pirfenidone analog in the treatment of cancer, a fibrotic disorder, or an angiogenic disorder in a patient comprising administering to the patient a dosage of synthetic CXCR3 ligand containing an amount of from about 0.1 µg to about 1000 µg of drug per dose of synthetic CXCR3 ligand, subcutaneously qd, qod, tiw, or biw, or per day substantially continuously or continuously, in combination with a dosage of pirfenidone or a specific pirfenidone analog containing an amount of about 50 mg to about 5,000 mg of drug per dose orally qd, optionally in two or more divided doses per day, for the desired treatment duration.

In one embodiment, the invention provides a method using an effective amount of synthetic CXCR3 ligand and pirfenidone or a specific pirfenidone analog in the treatment of cancer, a fibrotic disorder, or an angiogenic disorder in a patient comprising administering to the patient a dosage of synthetic CXCR3 ligand containing an amount of from about 0.1 µg to about 1000 µg of drug per dose of synthetic CXCR3 ligand, intramuscularly qd, qod, tiw, or biw, or per day substantially continuously or continuously, in combination with a dosage of pirfenidone or a specific pirfenidone analog containing an amount of about 50 mg to about 5,000 mg of drug per dose orally qd, optionally in two or more divided doses per day, for the desired treatment duration.

In one embodiment, the invention provides a method using an effective amount of synthetic CXCR3 ligand and pirfenidone or a specific pirfenidone analog in the treatment of cancer, a fibrotic disorder, or an angiogenic disorder in a patient comprising administering to the patient a dosage of synthetic CXCR3 ligand containing an amount of from about 0.1 µg to about 50 µg of drug per dose of synthetic CXCR3 ligand, subcutaneously qd, qod, tiw, or biw, or per day substantially continuously or continuously, in combination with a dosage of pirfenidone or a specific pirfenidone analog containing an amount of about 500 mg of drug per dose orally qd, optionally in two or more divided doses per day, for the desired treatment duration.

In one embodiment, the invention provides a method using an effective amount of synthetic CXCR3 ligand and pirfenidone or a specific pirfenidone analog in the treatment of cancer, a fibrotic disorder, or an angiogenic disorder in a patient comprising administering to the patient a dosage of synthetic CXCR3 ligand containing an amount of from about 0.1 µg to about 50 µg of drug per dose of synthetic CXCR3 ligand, intramuscularly qd, qod, tiw, or biw, or per day substantially continuously or continuously, in combination with a dosage of pirfenidone or a specific pirfenidone analog containing an amount of about 500 mg of drug per dose orally qd, optionally in two or more divided doses per day, for the desired treatment duration.

### Synthetic CXCR3 ligand and Type I interferon receptor agonist combination therapy

In some embodiments, a subject synthetic CXCR3 ligand is administered in combination therapy with a Type I interferon receptor agonist for the treatment of cancer, a fibrotic disorder, or an angiogenic disorder. The methods generally involve administering to an individual in need thereof an effective amount of a subject synthetic CXCR3 ligand and an effective amount of a Type I interferon receptor agonist. In some embodiments, the Type I interferon receptor agonist is IFN-a.

Effective dosages of IFN-α can range from 0.3 µg to 100 µg. Effective dosages of Infergen® consensus IFN-α contain an amount of about 3 µg, about 9 µg, about 15 µg, about 18 µg, or about 27 µg of drug per dose. Effective dosages of IFN-α2a and IFN-α2b contain an amount of about 3 million Units (MU) to about 10 MU of drug per dose. Effective dosages of PEGASYS®PEGylated IFN-α2a contain an amount of about 90 µg to about 180 µg, or about 135 µg, of drug per dose. Effective dosages of PEG-INTRON®PEGylated IFN-α2b contain an amount of about 0.5 µg to about 1.5 µg of drug per kg of body weight per dose. Effective dosages of PEGylated hybrid interferon (PEG-CIFN) contain an amount of about 18 µg to about 90 µg, or about 27 µg to about 60 µg, or about 45 µg, of CIFN amino acid weight per dose of PEG-CIFN. Effective dosages of monoPEG (30 kD, linear)-ylated CIFN can contain an amount of about 3 µg µg to about 300 µg, or about 9 µg to about 90 µg, or about 27 µg, of drug per dose.

IFN-α is typically administered subcutaneously. For example, IFN-α can be administered subcutaneously qd, qod, tiw, or biw, or per day substantially continuously or continuously for a period of from about 2 weeks to about 52 weeks, from about 52 weeks to about 2 years, or longer.

In one embodiment, the invention provides a method using an effective amount of INFERGEN®consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of INFERGEN® containing an amount of about 1 µg to about 30 µg of drug per dose of INFERGEN®, subcutaneously qd, qod, tiw, or biw, or per day substantially continuously or continuously, in combination with a dosage of pirfenidone or a specific pirfenidone analog containing an amount of about 50 mg to about 5,000 mg of drug per dose orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod; tiw, or biw, or per day substantially continuously or continuously, for the desired treatment duration.

In another embodiment, the invention provides a method using an effective amount of INFERGEN®consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of INFERGEN® containing an amount of about 1 µg to about 9 µg of drug per dose of INFERGEN®, subcutaneously qd, qod, tiw, or biw, or per day substantially continuously or continuously, in combination with a dosage of pirfenidone or a specific pirfenidone analog containing an amount of about 100 mg to about 1,000 mg of drug per dose orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod, tiw, or biw, or per day substantially continuously or continuously, for the desired treatment duration.

In another embodiment, the invention provides a method using an effective amount of INFERGEN®consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of INFERGEN® containing an amount of about 9 µg of drug per dose of INFERGEN®, subcutaneously qd, qod, tiw, or biw, or per day substantially continuously or continuously, in combination with a dosage of pirfenidone or a specific pirfenidone analog containing an amount of about 500 mg of drug per dose orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod, tiw, or biw, or per day substantially continuously or continuously, for the desired treatment duration.

In another embodiment, the invention provides a method using an effective amount of INFERGEN®consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of INFERGEN® containing an amount of about 30 µg of drug per dose of INFERGEN®, subcutaneously qd, qod, tiw, or biw, or per day substantially continuously or continuously, in combination with a dosage of pirfenidone or a specific pirfenidone analog containing an amount of about 1,000 mg to about 2,000 mg of drug per dose orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod, tiw, or biw, or per day substantially continuously or continuously, for the desired treatment duration.

In another embodiment, the invention provides a method using an effective amount of a consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of PEGylated consensus IFN-α (PEG-CIFN) containing an amount of about 10 µg to about 150 µg of CIFN amino acid weight per dose of PEG-CIFN, subcutaneously qw, qow, three times per month, or monthly, in combination with a dosage of pirfenidone or a specific pirfenidone analog containing an amount of about 50 mg to about 5,000 mg of drug per dose orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod, tiw, or biw, or per day substantially continuously or continuously, for the desired treatment duration.

In another embodiment, the invention provides a method using an effective amount of a consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of monoPEG (30 kD, linear)-ylated consensus IFN-α containing an amount of about 3 µg to about 300 µg of drug per dose, subcutaneously qw, qow, three times per month, or monthly, in combination with a dosage of pirfenidone or a specific pirfenidone analog containing an amount of about 50 mg to about 5,000 mg of drug orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod, tiw, or biw, or per day substantially continuously or continuously, for the desired treatment duration.

In another embodiment, the invention provides a method using an effective amount of INFERGEN®consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of INFERGEN® containing an amount of about 5 µg to about 150 µg of drug per dose of INFERGEN®, subcutaneously qd, qod, tiw, or biw, or per day substantially continuously or continuously, in combination with a dosage of pirfenidone or a specific pirfenidone analog containing an amount of about 1,000 mg to about 10,000 mg of drug per dose orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod, tiw, or biw, or per day substantially continuously or continuously, for the desired treatment duration.

In another embodiment, the invention provides a method using an effective amount of INFERGEN®consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of INFERGEN® containing an amount of about 5 µg to about 45 µg of drug per dose of INFERGEN®, subcutaneously qd, qod, tiw, or biw, or per day substantially continuously or continuously, in combination with a dosage of pirfenidone or a specific pirfenidone analog containing an amount of about 1,000 mg to about 3,000 mg of drug per dose orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod, tiw, or biw, or per day substantially continuously or continuously, for the desired treatment duration.

In another embodiment, the invention provides a method using an effective amount of INFERGEN®consensus IFN-α, synthetic CXCR3 ligand, and pirfenidone or a specific pirfenidone analog in the treatment of cancer, a fibrotic disorder, or an angiogenic disorder in a patient comprising administering to the patient a dosage of INFERGEN® containing an amount of about 45 µg of drug per dose of INFERGEN®, subcutaneously qd, qod, tiw, or biw, or per day substantially continuously or continuously, in combination with a dosage of pirfenidone or a specific pirfenidone analog containing an amount of about 1,000 mg to about 2,000 mg of drug per dose orally qd, optionally in two or more divided doses per day, and a dosage of synthetic CXCR3 ligand containing an amount of from about 0.1 µg to about 1000 µg of drug per dose of synthetic CXCR3 ligand, intramuscularly or subcutaneously qd, qod, tiw, or biw, or per day substantially continuously or continuously, for the desired treatment duration.

### Synthetic CXCR3 ligand and Type II interferon receptor agonist combination therapy

In another aspect, the present invention provides combination therapy for the treatment of cancer, a fibrotic disorder, an angiogenic disorder, or a bacterial infection, comprising co-administering to the patient effective amounts of a subject synthetic CXCR3 ligand and a Type II interferon receptor agonist. In some embodiments, the Type II interferon receptor agonist is IFN-γ.

Effective dosages of a subject synthetic CXCR3 ligand are those discussed above.

Effective dosages of IFN-γ range from about 0.5 µg/m² to about 500 µg/m², usually from about 1.5 µg/m² to 200 µg/m², depending on the size of the patient. This activity is based on 10⁶ international units (U) per 50 µg of protein. IFN-γ can be administered daily, every other day, three times a week, twice per week, or substantially continuously or continuously for a period of from about 2 weeks to about 52 weeks, from about 52 weeks to about 2 years, or longer.

In certain embodiments, IFN-γ is administered to an individual in a unit dosage form of from about 25 µg to about 500 µg, from about 50 µg to about 400 µg, or from about 100 µg to about 300 µg. In particular embodiments of interest, the dose is about 200 µg IFN-γ. In many embodiments of interest, IFN-γ1b is administered.

Where the dosage is 200 µg IFN-γ per dose, the amount of IFN-γ per body weight (assuming a range of body weights of from about 45 kg to about 135 kg) is in the range of from about 4.4 µg IFN-γ per kg body weight to about 1.48 µg IFN-γ per kg body weight

The body surface area of subject individuals generally ranges from about 1.33 m² to about 2.50 m². Thus, in many embodiments, an IFN-γ dosage ranges from about 150 µg/m² to about 20 µg/m². For example, an IFN-γ dosage ranges from about 20 µg/m² to about 30 µg/m², from about 30 µg/m² to about 40 µg/m², from about 40 µg/m² to about 50 µg/m², from about 50 µg/m₂ to about 60 µg/m², from about 60 µg/m² to about 70 µg/m², from about 70 µg/m² to about 80 µg/m², from about 80 µg/m² to about 90 µg/m², from about 90 µg/m² to about 100 µg/m², from about 100 µg/m²to about 110 µg/m², from about 110 µg/m² to about 120 µg/m², from about 120 µg/m² to about 130 µg/m², from about 130 µg/m² to about 140 µg/m², or from about 140 µg/m² to about 150 µg/m². In some embodiments, the dosage groups range from about 25 µg/m² to about 100 µg/m². In other embodiments, the dosage groups range from about 25 µg/m² to about 50 µg/m².

### Further combination therapies

The present invention further contemplates combination therapies for the treatment of cancer, a fibrotic disorder, or an angiogenic disorder, involving administering an effective amount of a subject synthetic CXCR3 ligand, an effective amount of a Type I interferon receptor agonist (e.g., an IFN-α), and an effective amount of pirfenidone or a pirfenidone analog. Effective amounts are those discussed above.

The present invention further contemplates combination therapies for the treatment of cancer, a fibrotic disorder, or an angiogenic disorder, involving administering an effective amount of a synthetic CXCR3 ligand, an effective amount of a Type II interferon receptor agonist (e.g., an IFN-γ)_{;} and an effective amount of pirfenidone or a pirfenidone analog. Effective amounts are those discussed above.

The present invention further contemplates combination therapies for the treatment of cancer, a fibrotic disorder, or an angiogenic disorder, involving administering an effective amount of a synthetic CXCR3 ligand, an effective amount of a Type I interferon receptor agonist (e.g., IFN-γ), an effective amount of a Type II interferon receptor agonist (e.g., an IFN-α), and an effective amount of pirfenidone or a pirfenidone analog. Effective amounts are those discussed above.

### Synthetic CXCR3 ligand combination therapy as adjuvant therapy for cancer

In some embodiments, the present invention provides methods for combination therapy using synthetic CXCR3 ligand therapy as adjuvant therapy to a standard cancer therapy. Standard cancer therapies include surgery (e.g., surgical removal of cancerous tissue), radiation therapy, bone marrow transplantation, chemotherapeutic treatment, biological response modifier treatment, and certain combinations of the foregoing.

Radiation therapy includes, but is not limited to, x-rays or gamma rays that are delivered from either an externally applied source such as a beam, or by implantation of small radioactive sources.

Chemotherapeutic agents are non-peptidic (i.e., non-proteinaceous) compounds that reduce proliferation of cancer cells, and encompass cytotoxic agents and cytostatic agents. Non-limiting examples of chemotherapeutic agents include alkylating agents, nitrosoureas, antimetabolites, antitumor antibiotics, plant (vinca) alkaloids, and steroid hormones.

Agents that act to reduce cellular proliferation are known in the art and widely used. Such agents include alkylating agents, such as nitrogen mustards, nitrosoureas, ethylenimine derivatives, alkyl sulfonates, and triazenes, including, but not limited to, mechlorethamine, cyclophosphamide (Cytoxan^{™}), melphalan (L-sarcolysin), carmustine (BCNU), lomustine (CCNU), semustine (methyl-CCNU), streptozocin, chlorozotocin, uracil mustard, chlormethine, ifosfamide, chlorambucil, pipobroman, triethylenemelamine, triethylenethiophosphoramine, busulfan, dacarbazine, and temozolomide.

Antimetabolite agents include folic acid analogs, pyrimidine analogs, purine analogs, and adenosine deaminase inhibitors, including, but not limited to, cytarabine (CYTOSAR-U), cytosine arabinoside, fluorouracil (5-FU), floxuridine (FudR), 6-thioguanine, 6-mercaptopurine (6-MP), pentostatin, 5-fluorouracil (5-FU), methotrexate, 10-propargyl-5,8-dideazafolate (PDDF, CB3717), 5,8-dideazatetrahydrofolic acid (DDATHF), leucovorin, fludarabine phosphate, pentostatine, and gemcitabine.

Suitable natural products and their derivatives, (e.g., vinca alkaloids, antitumor antibiotics, enzymes, lymphokines, and epipodophyllotoxins), include, but are not limited to, Ara-C, paclitaxel (Taxol®), docetaxel (Taxotere®), deoxycoformycin, mitomycin-C, L-asparaginase, azathioprine; brequinar; alkaloids, *e.*g. vincristine, vinblastine, vinorelbine, vindesine, *etc*.; podophyllotoxins, *e.g*. etoposide, teniposide, *etc*.; antibiotics, *e.g*. anthracycline, daunorubicin hydrochloride (daunomycin, rubidomycin, cerubidine), idarubicin, doxorubicin, epirubicin and morpholino derivatives, etc.; phenoxizone biscyclopeptides, e.g. dactinomycin; basic glycopeptides, *e.*g. bleomycin; anthraquinone glycosides, *e.g*. plicamycin (mithramycin); anthracenediones, *e.*g. mitoxantrone; azirinopyrrolo indolediones, *e.g.* mitomycin; macrocyclic immunosuppressants, *e.g.* cyclosporine, FK-506 (tacrolimus, prograf), rapamycin, etc.; and the like.

Other anti-proliferative cytotoxic agents are navelbene, CPT-11, anastrazole, letrazole, capecitabine, reloxafine, cyclophosphamide, ifosamide, and droloxafine.

Microtubule affecting agents that have antiproliferative activity are also suitable for use and include, but are not limited to, allocolchicine (NSC 406042), Halichondrin B (NSC 609395), colchicine (NSC 757), colchicine derivatives (e.g., NSC 33410), dolstatin 10 (NSC 376128), maytansine (NSC 153858), rhizoxin (NSC 332598), paclitaxel (Taxol®), Taxol® derivatives, docetaxel (Taxotere®), thiocolchicine (NSC 361792), trityl cysterin, vinblastine sulfate, vincristine sulfate, natural and synthetic epothilones including but not limited to, eopthilone A, epothilone B, discodermolide; estramustine, nocodazole, and the like.

Hormone modulators and steroids (including synthetic analogs) that are suitable for use include, but are not limited to, adrenocorticosteroids, *e.g.* prednisone, dexamethasone, *etc.;* estrogens and pregestins, e.g. hydroxyprogesterone caproate, medroxyprogesterone acetate, megestrol acetate, estradiol, clomiphene, tamoxifen; *etc.*; and adrenocortical suppressants, *e.g.* aminoglutethimide; 17α-ethinylestradiol; diethylstilbestrol, testosterone, fluoxymesterone, dromostanolone propionate, testolactone, methylprednisolone, methyl-testosterone, prednisolone, triamcinolone, chlorotrianisene, hydroxyprogesterone, aminoglutethimide, estramustine, medroxyprogesterone acetate, leuprolide, Flutamide (Drogenil), Toremifene (Fareston), and Zoladex®. Estrogens stimulate proliferation and differentiation, therefore compounds that bind to the estrogen receptor are used to block this activity. Corticosteroids may inhibit T cell proliferation.

Other chemotherapeutic agents include metal complexes, *e.g.* cisplatin (cis-DDP), carboplatin, *etc.;* ureas, *e.g.* hydroxyurea; and hydrazines, *e.g.* N-methylhydrazine; epidophyllotoxin; a topoisomerase inhibitor; procarbazine; mitoxantrone; leucovorin; tegafur; etc.. Other anti-proliferative agents of interest include immunosuppressants, e.g. mycophenolic acid, thalidomide, desoxyspergualin, azasporine, leflunomide, mizoribine, azaspirane (SKF 105685); Iressa® (ZD 1839, 4-(3-chloro-4-fluorophenylamino)-7-methoxy-6-(3-(4-morpholinyl)propoxy)quinazoline); etc.

"Taxanes" include paclitaxel, as well as any active taxane derivative or pro-drug. "Paclitaxel" (which should be understood herein to include analogues, formulations, and derivatives such as, for example, docetaxel, TAXOL™ TAXOTERE™(a formulation of docetaxel), 10-desacetyl analogs of paclitaxel and 3'N-desbenzoyl-3'N-t-butoxycarbonyl analogs of paclitaxel) may be readily prepared utilizing techniques known to those skilled in the art (see also WO 94/07882, WO 94/07881, WO 94/07880, WO 94/07876, WO 93/23555, WO 93/10076; U.S. Pat. Nos. 5,294,637; 5,283,253; 5,279,949; 5,274,137; 5,202,448; 5,200,534; 5,229,529; and EP 590,267), or obtained from a variety of commercial sources, including for example, Sigma Chemical Co., St. Louis, Mo. (T7402 from *Taxus brevifolia;* or T-1912 from *Taxus yannanensis*).

Paclitaxel should be understood to refer to not only the common chemically available form of paclitaxel, but analogs and derivatives (e.g., Taxotere™ docetaxel, as noted above) and paclitaxel conjugates (e.g., paclitaxel-PEG, paclitaxel-dextran, or paclitaxel-xylose).

Also included within the term "taxane" are a variety of known derivatives, including both hydrophilic derivatives, and hydrophobic derivatives. Taxane derivatives include, but not limited to, galactose and mannose derivatives described in International Patent Application No. WO 99/18113; piperazino and other derivatives described in WO 99/14209; taxane derivatives described in WO 99/09021, WO 98/22451, and U.S. Patent No. 5,869,680; 6-thio derivatives described in WO 98/28288; sulfenamide derivatives described in U.S. Patent No. 5,821,263; and taxol derivative described in U.S. Patent No. 5,415,869. It further includes prodrugs of paclitaxel including, but not limited to, those described in WO 98/58927; WO 98/13059; and U.S. Patent No. 5,824,701.

Biological response modifiers suitable for use in connection with the methods of the invention include, but are not limited to, (1) inhibitors of tyrosine kinase (RTK) activity; (2) inhibitors of serine/threonine kinase activity; (3) tumor-associated antigen antagonists, such as antibodies that bind specifically to a tumor antigen; (4) apoptosis receptor agonists; (5) interleukin-2; (6) IFN-α; (7) IFN-γ (8) colony-stimulating factors; (9) inhibitors of angiogenesis; and (10) antagonists of tumor necrosis factor.

In one aspect, the invention contemplates synthetic CXCR3 ligand therapy as an adjuvant to any therapy in which the cancer patient receives treatment with least one additional antineoplastic drug, where the additional drug is a tyrosine kinase inhibitor. In some embodiments, the tyrosine kinase inhibitor is a receptor tyrosine kinase (RTK) inhibitor, such as type I receptor tyrosine kinase inhibitors (e.g., inhibitors of epidermal growth factor receptors), type II receptor tyrosine kinase inhibitors (e.g., inhibitors of insulin receptor), type III receptor tyrosine kinase inhibitors (e.g., inhibitors of platelet-derived growth factor receptor), and type IV receptor tyrosine kinase inhibitors (e.g., fibroblast growth factor receptor). In other embodiments, the tyrosine kinase inhibitor is a non-receptor tyrosine kinase inhibitor, such as inhibitors of src kinases or janus kinases.

In another aspect, the invention contemplates synthetic CXCR3 ligand therapy as an adjuvant to any therapy in which the cancer patient receives treatment with least one additional antineoplastic drug, where the additional drug is an inhibitor of a receptor tyrosine kinase involved in growth factor signaling pathway(s). In some embodiments, the inhibitor is genistein. In other embodiments, the inhibitor is an EGFR tyrosine kinase-specific antagonist, such as IRESSA™gefitinib (ZD18398; Novartis), TARCEVA™erolotinib (OSI-774; Roche; Genentech; OSI Pharmaceuticals), or tyrphostin AG1478 (4-(3-chloroanilino)-6,7-dimethoxyquinazoline. In still other embodiments, the inhibitor is any indolinone antagonist of Flk-1/KDR (VEGF-R2) tyrosine kinase activity described in U.S. Patent Application Publication No. 2002/0183364 A1, such as the indolinone antagonists of FIk-1/KDR (VEGF-R2) tyrosine kinase activity disclosed in Table 1 on pages 4-5 thereof. In further embodiments, the inhibitor is any of the substituted 3-[(4,5,6,7-tetrahydro-1H-indol-2-yl) methylene]-1,3-dihydroindol-2-one antagonists of Flk-1/KDR (VEGF-R2), FGF-R1 or PDGF-R tyrosine kinase activity disclosed in Sun, L., et al., J. Med. Chem., 43 14 : 2655-2663 (2000). In additional embodiments, the inhibitor is any substituted 3-[(3- or 4-carboxyethylpyrrol-2-yl) methylidenyl]indolin-2-one antagonist of Flt-1 (VEGF-R1), Flk-1KDR (VEGF-R2), FGF-R1 or PDGF-R tyrosine kinase activity disclosed in Sun, L., et al., J. Med. Chem., 42(25): 5120-5130 (1999).

In another aspect, the invention contemplates synthetic CXCR3 ligand therapy as an adjuvant to any therapy in which the cancer patient receives treatment with least one additional antineoplastic drug, where the additional drug is an inhibitor of a non-receptor tyrosine kinase involved in growth factor signaling pathway(s). In some embodiments, the inhibitor is an antagonist of JAK2 tyrosine kinase activity, such as tyrphostin AG490 (2-cyano-3-(3,4-dihydroxyphenyl)-N-(benzyl)-2-propenamide). In other embodiments, the inhibitor is an antagonist of bcr-abl tyrosine kinase activity, such as GLEEVEC™imatinib mesylate (STI-571; Novartis).

In another aspect, the invention contemplates synthetic CXCR3 ligand therapy as an adjuvant to any therapy in which the cancer patient receives treatment with least one additional antineoplastic drug, where the additional drug is a serine/threonine kinase inhibitor. In some embodiments, the serine/threonine kinase inhibitor is a receptor serine/threonine kinase inhibitor, such as antagonists of TGF-β receptor serine/threonine kinase activity. In other embodiments, the serine/threonine kinase inhibitor is a non-receptor serine/threonine kinase inhibitor, such as antagonists of the serine/threonine kinase activity of the MAP kinases, protein kinase C (PKC), protein kinase A (PKA), or the cyclin-dependent kinases (CDKs).

In another aspect, the invention contemplates the combination of synthetic CXCR3 ligand therapy as an adjuvant to any therapy in which the cancer patient receives treatment with least one additional antineoplastic drug, where the additional drug is an inhibitor of one or more kinases involved in cell cycle regulation. In some embodiments, the inhibitor is an antagonist of CDK2 activation, such as tryphostin AG490 (2-cyano-3-(3,4-dihydroxyphenyl)-N-(benzyl)-2-propenamide). In other embodiments, the inhibitor is an antagonist of CDKl/cyclin B activity, such as alsterpaullone. In still other embodiments, the inhibitor is an antagonist of CDK2 kinase activity, such as indirubin-3'-monoxime. In additional embodiments, the inhibitor is an ATP pool antagonist, such as lometrexol (described in U.S. Patent Application Publication No. 2002/0156023 A1).

In another aspect, the invention contemplates the combination of synthetic CXCR3 ligand therapy as an adjuvant to any therapy in which the cancer patient receives treatment with least one additional antineoplastic drug, where the additional drug is an a tumor-associated antigen antagonist, such as an antibody antagonist. In some embodiments involving the treatment of HER2-expressing tumors, the tumor-associated antigen antagonist is an anti-HER2 monoclonal antibody, such as HERCEPTIN™ trastuzumab. In some embodiments involving the treatment of CD20-expressing tumors, such as B-cell lymphomas, the tumor-associated antigen antagonist is an anti-CD20 monoclonal antibody, such as RITUXAN™ rituximab.

In another aspect, the invention contemplates the combination of synthetic GXCR3 ligand therapy as an adjuvant to any therapy in which the cancer patient receives treatment with least one additional antineoplastic drug, where the additional drug is a tumor growth factor antagonist. In some embodiments, the tumor growth factor antagonist is an antagonist of epidermal growth factor (EGF), such as an anti-EGF monoclonal antibody. In other embodiments, the tumor growth factor antagonist is an antagonist of epidermal growth factor receptor erbB1 (EGFR), such as an anti-EGFR monoclonal antibody-inhibitor of EGFR activation or signal transduction.

In another aspect, the invention contemplates the combination of synthetic CXCR3 ligand therapy as an adjuvant to any therapy in which the cancer patient receives treatment with least one additional antineoplastic drug, where the additional drug is an Apo-2 ligand agonist. In some embodiments, the Apo-2 ligand agonist is any of the Apo-2 ligand polypeptides described in WO 97/25428.

In another aspect, the invention contemplates the combination of synthetic CXCR3 ligand therapy as an adjuvant to any therapy in which the cancer patient receives treatment with least one additional antineoplastic drug, where the additional drug is an anti-angiogenic agent. In some embodiments, the anti-angiogenic agent is a vascular endothelial cell growth factor (VEGF) antagonist, such as an anti-VEGF monoclonal antibody, e.g. AVASTIN™ bevacizumab (Genentech). In other embodiments, the anti-angiogenic agent is a retinoic acid receptor (RXR) ligand, such as any RXR ligand described in U.S. Patent Application Publication No. 2001/0036955 A1 or in any of U.S. Pat. Nos. 5,824,685; 5,780,676; 5,399,586; 5,466,861; 4,810,804; 5,770,378; 5,770,383; or 5,770,382. In still other embodiments, the anti-angiogenic agent is a peroxisome proliferator-activated receptor (PPAR) gamma ligand, such as any PPAR gamma ligand described in U.S. Patent Application Publication No. 2001/0036955 A1.

Exemplary non-limiting examples of combination therapies that include treatment with radiation, a subject synthetic CXCR3 ligand, or treatment with a chemotherapeutic agent and a subject synthetic CXCR3 ligand, are as follows:

1) a dosage of a subject synthetic CXCR3 ligand containing from about 0.1 µg to about 1000 µg synthetic CXCR3 ligand per dose; and cisplatin in a dosage range of from about 5 mg/m² to about 150 mg/m²_{;}

2) a dosage of a subject synthetic CXCR3 ligand containing from about 0.1 µg to about 1000 µg synthetic CXCR3 ligand per dose; and carboplatin in a dosage range of from about 5 mg/m² to about 1000 mg/m²;

3) a dosage of a subject synthetic CXCR3 ligand containing from about 0.1 µg to about 1000 µg synthetic CXCR3 ligand per dose; and radiation in a dosage range of from about 200 cGy to about 8000 cGy;

4) a dosage of a subject synthetic CXCR3 ligand containing from about 0.1 µg to about 1000 µg synthetic CXCR3 ligand per dose; and paclifaxel in a dosage range of from about 40 mg/m² to about 250 mg/m²;

5) a dosage of a subject synthetic CXCR3 ligand containing from about 0.1 µg to about 1000 µg synthetic CXCR3 ligand per dose; paclitaxel in a dosage range of from about 40 mg/m² to about 250 mg/m²; and carboplatin in a dosage range of from about 5 mg/m² to about 1000 mg/m²;

6) a dosage of a subject synthetic CXCR3 ligand containing from about 0.1 µg to about 1000 µg synthetic CXCR3 ligand per dose; 5FU in a dosage range of from about 5 mg/m² to about 5000 mg/m²; and leucovorin in a dosage range of from about 5 mg/m² to about 1000 mg/m²;

7) a dosage of a subject synthetic CXCR3 ligand containing from about 0.1 µg to about 1000 µg synthetic CXCR3 ligand per dose; and trastuzumab in an initial loading dose of 4 mg/kg and a weekly maintenance dose of 2 mg/kg;

8) a dosage of a subject synthetic CXCR3 ligand containing from about 0.1 µg to about 1000 µg synthetic CXCR3 ligand per dose; trastuzumab in an initial loading dose of 4 mg/kg and a weekly maintenance dose of 2 mg/kg; and paclitaxel in a dosage range of from about 40 mg/m² to about 250 mg/m²;

9) a dosage of a subject synthetic CXCR3 ligand containing from about 0.1 µg to about 1000 µg synthetic CXCR3 ligand per dose; paclitaxel in a dosage range of from about 40 mg/m² to about 250 mg/m²; and estramustine phosphate (Emcyte®) in a dosage range of from about 5 mg/m² to about 1000 mg/m²;

10) a dosage of a subject synthetic CXCR3 ligand containing from about 0.1 µg to about 1000 µg synthetic CXCR3 ligand per dose; cisplatin in a dosage range of from about 5 mg/m² to about 150 mg/m²; and 5FU in a dosage range of from about 5 mg/m² to about 5000 mg/m²;

11) a dosage of a subject synthetic CXCR3 ligand containing from about 0.1 µg to about 1000 µg synthetic CXCR3 ligand per dose; 5FU in a dosage range of from about 5 mg/m² to about 5000 mg/m²; and radiation in a dose of from about 200 cGy to about 8000 cGy;

12) a dosage of a subject synthetic CXCR3 ligand containing from about 0.1 µg to about 1000 µg synthetic CXCR3 ligand per dose; 5PU in a dosage range of from about 5 mg/m² to about 5000 mg/m²; and paclitaxel in a dosage range of from about 40 mg/m² to about 250 mg/m²;

In some embodiments, any of the above-described regimens (e.g., regimens 1-12) further comprises administering a dosage of pirfenidone or a specific pirfenidone analog containing an amount of from about 100 mg to about 1000 mg of drug per dose.

### Synthetic CXCR3 ligand combination therapy for treating fibrotic disorders

The present invention provides combination therapies for treating fibrotic disorders, generally involving administering effective amounts of a subject synthetic CXCR3 ligand and at least a second therapeutic agent. Combination therapies for fibrotic disorders involving administering effective amounts of a subject synthetic CXCR3 ligand and pirfenidone or a pirfenidone analog are described above. Combination therapies for fibrotic disorders involving administering effective amounts of a subject synthetic CXCR3 ligand and a Type I interferon receptor agonist (e.g., IFN-α) are described above. Combination therapies for fibrotic disorders involving administering effective amounts of a subject synthetic CXCR3 ligand and a Type II interferon receptor agonist (e.g., IFN-γ) are described above.

In some embodiments, present invention provides combination therapies for treating fibrotic disorders, generally involving administering effective amounts of a subject synthetic CXCR3 ligand and a second therapeutic agent selected from a TNF antagonist, a TNF-β antagonist, and an endothelin receptor antagonist. In some of these embodiments, any of these combination therapy methods is modified to include administering one or more of: pirfenidone or a pirfenidone analog; a Type II interferon receptor agonist; a Type I interferon receptor agonist; and a SAPK inhibitor (other than pirfenidone).

### Synthetic CXCR3 ligand in combination therapy with a TNF antagonist to treat fibrotic disorders

In some embodiments, a subject therapeutic regimen for treating a fibrotic disorder involves administering effective amounts of a subject synthetic CXCR3 ligand and a TNF antagonist. Effective dosages of a TNF-α antagonist range from 0.1 µg to 40 mg per dose, e.g., from about 0.1 µg to about 0.5 µg per dose, from about 0.5 µg to about 1.0 *µg per dose, from about 1.0 µg per dose to about 5.0 µg per dose, from about 5.0 µg to about 10 µg per dose, from about 10 µg to about 20 µg per dose, from about 20 µg per dose to about 30 µg per dose, from about 30 µg per dose to about 40 µg per dose, from about 40 µg per dose to about 50 µg per dose, from about 50 µg per dose to about 60 µg per dose, from about 60 µg per dose to about 70 µg per dose, from about 70 µg to about 80 µg per dose, from about 80 µg per dose to about 100 µg per dose, from about 100 µg to about 150 µg per dose, from about 150 µg to about 200 µg per dose, from about 200 µg per dose to about 250 µg per dose, from about 250 µg to about 300 µg per dose, from about 300 µg to about 400 µg per dose, from about 400 µg to about 500 µg per dose, from about 500 µg to about 600 µg per dose, from about 600 µg to about 700 µg per dose, from about 700 µg to about 800 µg per dose, from about 800 µg to about 900 µg per dose, from about 900 µg to about 1000 µg per dose, from about 1 mg to about 10 mg per dose, from about 10 mg to about 15 mg per dose, from about 15 mg to about 20 mg per dose, from about 20 mg to about 25 mg per dose, from about 25 mg to about 30 mg per dose, from about 30 mg to about 35 mg per dose, or from about 35 mg to about 40 mg per dose.

In some embodiments, the TNF-α antagonist is ENBREL® etanercept. Effective dosages of etanercept range from about 0.1 µg to about 40 mg per dose, from about 0.1 µg to about 1 µg per dose, from about 1 µg to about 10 µg per dose, from about 10 µg to about 100 µg per dose, from about 100 µg to about 1 mg per dose, from about 1 mg to about 5 mg per dose, from about 5 mg to about 10 mg, from about 10 mg to about 15 mg per dose, from about 15 mg to about 20 mg per dose, from about 20 mg to about 25 mg per dose, from about 25 mg to about 30 mg per dose, from about 30 mg to about 35 mg per dose, or from about 35 mg to about 40 mg per dose.

In some embodiments, effective dosages of a TNF-α antagonist are expressed as mg/kg body weight. In these embodiments, effective dosages of a TNF-α antagonist are from about 0.1 mg/kg body weight to about 10 mg/kg body weight, e.g., from about 0.1 mg/kg body weight to about 0.5 mg/kg body weight, from about 0.5 mg/kg body weight to about 1.0 mg/kg body weight, from about 1.0 mg/kg body weight to about 2.5 mg/kg body weight, from about 2.5 mg/kg body weight to about 5.0 mg/kg body weight, from about 5.0 mg/kg body weight to about 7.5 mg/kg body weight, or from about 7.5 mg/kg body weight to about 10 mg/kg body weight.

In some embodiments, the TNF-α antagonist is REMICADE® infliximab. Effective dosages of REMICADE® range from about 0.1 mg/kg to about 10 mg/kg, from about 0.1 mg/kg to about 0.5 mg/kg, from about 0.5 mg/kg to about 1.0 mg/kg, from about 1.0 mg/kg to about 1.5 mg/kg, from about 1.5 mg/kg to about 2.0 mg/kg, from about 2.0 mg/kg to about 2.5 mg/kg, from about 2.5 mg/kg to about 3.0 mg/kg, from about 3.0 mg/kg to about 3.5 mg/kg, from about 3.5 mg/kg to about 4.0 mg/kg, from about 4.0 mg/kg to about 4.5 mg/kg, from about 4.5 mg/kg to about 5.0 mg/kg, from about 5.0 mg/kg to about 7.5 mg/kg, or from about 7.5 mg/kg to about 10 mg/kg per dose.

In some embodiments the TNF-α antagonist is HUMIRA™ adalimumab. Effective dosages of HUMIRA™ range from about 0.1 µg to about 35 mg, from about 0.1 µg to about 1 µg, from about 1 µg to about 10 µg, from about 10 µg to about 100 µg, from about 100 µg to about 1 mg, from about 1 mg to about 5 mg, from about 5 mg to about 10 mg, from about 10 mg to about 15 mg, from about 15 mg to about 20 mg, from about 20 mg to about 25 mg, from about 25 mg to about 30 mg, from about 30 mg to about 35 mg, or from about 35 mg to about 40 mg per dose.

In many embodiments, a TNF-α antagonist is administered for a period of about 1 day to about 7 days, or about 1 week to about 2 weeks, or about 2 weeks to about 3 weeks, or about 3 weeks to about 4 weeks, or about I month to about 2 months, or about 3 months to about 4 months, or about 4 months to about 6 months, or about 6 months to about 8 months, or about 8 months to about 12 months, or at least one year, and may be administered over longer periods of time. The TNF-α antagonist can be administered tid, bid, qd, qod, biw, tiw, qw, qow, three times per month, once monthly, substantially continuously, or continuously.

In many embodiments, multiple doses of a TNF-α antagonist are administered. For example, a TNF-α antagonist is administered once per month, twice per month, three times per month, every other week (qow), once per week (qw), twice per week (biw), three times per week (tiw), four times per week, five times per week, six times per week, every other day (qod), daily (qd), twice a day (bid), or three times a day (tid), substantially continuously, or continuously, over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

Those of skill in the art will readily appreciate that dose levels can vary as a function of the specific compounds, the severity of the symptoms and the susceptibility of the subj ect to side effects. Preferred dosages for a given compound are readily determinable by those of skill in the art by a variety of means. A preferred means is to measure the physiological potency of a given compound.

A subject synthetic CXCR3 ligand and a TNF antagonist are generally administered in separate formulations. A subject synthetic CXCR3 ligand and a TNF antagonist may be administered substantially simultaneously, or within about 30 minutes, about 1 hour, about 2 hours, about 4 hours, about 8 hours, about 16 hours, about 24 hours, about 36 hours, about 72 hours, about 4 days, about 7 days, or about 2 weeks of one another.

In one embodiment, the invention provides a method using an effective amount of a subject synthetic CXCR3 ligand and a TNF antagonist in the treatment of a fibrotic disorder in a patient, comprising administering to the patient: a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously; and b) a dosage of a TNF antagonist containing an amount of from about 0.1 µg to 40 mg administered subcutaneously tid, bid, qd, qod, biw, tiw, qw, qow, three times per month, once monthly, for the desired treatment duration, to treat the fibrotic disorder.

In one embodiment, the invention provides a method using an effective amount of a subject synthetic CXCR3 ligand and a TNF antagonist in the treatment of a fibrotic disorder in a patient, comprising administering to the patient: a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously; and b) a dosage of a TNF-α antagonist selected from the group consisting of (i) ENBREL® in an amount of about 25 mg of drug subcutaneously biw (ii) REMICADE® in an amount of about 3 mg/kg to about 10 mg/kg of drug intravenously qw, qow, three times per month, once monthly, once every 6 weeks, or once every 8 weeks or (iii) HUMIRA™ in an amount of about 40 mg of drug subcutaneously qw, qow, three times per month, once monthly, once every 6 weeks, or once every 8 weeks, for the desired treatment duration, to treat the fibrotic disorder.

In some embodiments, the invention provides a combination therapy method involving modification of any one of the above-described treatment regimens featuring administering a subject CXCR3 ligand and a TNF antagonist, where the modification involves further administering an effective amount of pirfenidone or a pirfenidone analog. Effective amounts of pirfenidone or a pirfenidone analog are discussed above.

In one embodiment, the invention provides a method using an effective amount of a subject synthetic CXCR3 ligand, a TNF antagonist, and pirfenidone or a pirfenidone analog in the treatment of a fibrotic disorder in a patient, comprising administering to the patient: a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously; b) a dosage of a TNF-α antagonist selected from the group consisting of (i) ENBREL® in an amount of about 25 mg of drug subcutaneously biw (ii) REMICADE® in an amount of about 3 mg/kg to about 10 mg/kg of drug intravenously qw, qow, three times per month, once monthly, once every 6 weeks, or once every 8 weeks or (iii) HUMIRA™ in an amount of about 40 mg of drug subcutaneously qw, qow, three times per month, once monthly, once every 6 weeks, or once every 8 weeks, for the desired treatment duration; and c) a dosage of pirfenidone or a pirfenidone analog containing a dosage of pirfenidone or a specific pirfenidone analog containing an amount of about 100 mg to about 1,000 mg of drug per dose of pirfenidone or a specific pirfenidone analog orally qd, optionally in two or more divided doses per day, for the desired treatment duration to treat the fibrotic disorder.

In some embodiments, the invention provides a combination therapy method involving modification of any one of the above-described treatment regimens featuring administering a subject CXCR3 ligand and a TNF antagonist, where the modification involves further administering an effective amount of a Type II interferon receptor agonist. Effective amounts of a Type II interferon receptor agonist are discussed above.

In one embodiment, the invention provides a method using an effective amount of a subject synthetic CXCR3 ligand, a TNF antagonist, and a Type II interferon receptor agonist in the treatment of a fibrotic disorder in a patient, comprising administering to the patient: a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously; b) a dosage of a TNF-α antagonist selected from the group consisting of (i) ENBREL® in an amount of about 25 mg of drug subcutaneously biw (ii) REMICADE® in an amount of about 3 mg/kg to about 10 mg/kg of drug intravenously qw, qow, three times per month, once monthly, once every 6 weeks, or once every 8 weeks or (iii) HUMIRA™ in an amount of about 40 mg of drug subcutaneously qw, qow, three times per month, once monthly, once every 6 weeks, or once every 8 weeks, for the desired treatment duration; and c) a dosage of Actimmune® IFN-γ1b containing an amount of about 25 µg, 50 µg, 100 µg, 150 µg, or 200 µg, administered subcutaneously tiw for the desired treatment duration, to treat the fibrotic disorder.

In some embodiments, the invention provides a combination therapy method involving modification of any one of the above-described treatment regimens featuring administering a subject CXCR3 ligand and a TNF antagonist, where the modification involves further administering an effective amount of a Type I interferon receptor agonist. Effective amounts of a Type I interferon receptor agonist are discussed above.

In one embodiment, the invention provides a method using an effective amount of a subject synthetic CXCR3 ligand, a TNF antagonist, and a Type I interferon receptor agonist in the treatment of a fibrotic disorder in a patient, comprising administering to the patient: a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously; b) a dosage of a TNF-α antagonist selected from the group consisting of (i) ENBREL® in an amount of about 25 mg of drug subcutaneously biw (ii) REMICADE® in an amount of about 3 mg/kg to about 10 mg/kg of drug intravenously qw, qow, three times per month, once monthly, once every 6 weeks, or once every 8 weeks or (iii) HUMIRA™ in an amount of about 40 mg of drug subcutaneously qw, qow, three times per month, once monthly, once every 6 weeks, or once every 8 weeks, for the desired treatment duration; and c) a dosage of an IFN-α selected from (i) INFERGEN® containing an amount of about 1 µg to about 30 µg of drug per dose of INFERGEN® subcutaneously qd, qod, tiw, biw, qw, qow, three times per month, once monthly, or per day continuously or substantially continuously (ii) PEGylated consensus IFN-α (PEG-CIFN) containing an amount of about 10 µg to about 100 µg, or about 45 µg to about 60 µg, of CIFN amino acid weight per dose of PEG-CIFN subcutaneously qw, qow, three times per month, or monthly (iii) IFN-α 2a, 2b or 2c containing an amount of about 3 MU to about 10 MU of drug per dose of IFN-α 2a, 2b or 2c subcutaneously qd, qod, tiw, biw, or per day continuously or substantially continuously (iv) PEGASYS® containing an amount of about 90 µg to about 360 µg, or about 180 µg, of drug per dose of PEGASYS® subcutaneously qw, qow, three times per month, or monthly (v) PEG-INTRON® containing an amount of about 0.75 µg to about 3.0 µg, or about 1.0 µg to about 1.5 µg, of drug per kilogram of body weight per dose of PEG-INTRON® subcutaneously biw, qw, qow, three times per month, or monthly or (vi) mono PEG(30 kD, linear)-ylated consensus IFN-α containing an amount of from about 100 µg to about 200 µg, or about 150 µg, of drug per dose of mono PEG(30 kD, linear)-ylated consensus IFN-α subcutaneously qw, qow, once every 8 days to once every 14 days, three times per month, or monthly for the desired treatment duration, to treat the fibrotic disorder.

In some embodiments, the invention provides a combination therapy method involving modification of any one of the above-described treatment regimens featuring administering a subject CXCR3 ligand and a TNF antagonist, where the modification involves further administering an effective amount of a SAPK inhibitor.

Effective dosages of a SAPK inhibitor range from about 5 µg to about 3000 mg, e.g., from about 5 µg to about 10 µg, from about 10 µg to about 25 µg, from about 25 µg to about 50 µg, from about 50 µg to about 100 µg, from about 100 µg to about 250 µg, from about 250 µg to about 500 µg, from about 500 µg to about 750 µg, from about 750 µg to about 1 mg, from about 1 mg to about 5 mg, from about 5 mg to about 50 mg, from about 50 mg to about 100 mg, from about 100 mg to about 500 mg, from about 500 mg to about 1000 mg, from about 1000 mg to about 1500 mg, from about 1500 mg to about 2000 mg, from about 2000 mg to about 2500 mg, or from about 2500 mg to about 3000 mg.

A SAPK inhibitor can be administered once per month, twice per month, three times per month, once per week, twice per week, three times per week, four times per week, five times per week, six times per week, every other day, daily, twice daily, or in divided daily doses ranging from once daily to 5 times daily.

A SAPK inhibitor can be administered at any frequency, and over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

In one embodiment, the invention provides a method using an effective amount of a subject synthetic CXCR3 ligand, a TNF antagonist, and a SAPK inhibitor in the treatment of a fibrotic disorder in a patient, comprising administering to the patient: a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously for the desired treatment duration; b) a dosage of a TNF-α antagonist selected from the group consisting of (i) ENBREL® in an amount of about 25 mg of drug subcutaneously biw (ii) REMICADE® in an amount of about 3 mg/kg to about 10 mg/kg of drug intravenously qw, qow, three times per month, once monthly, once every 6 weeks, or once every 8 weeks or (iii) HUMIRA™ in an amount of about 40 mg of drug subcutaneously qw, qow, three times per month, once monthly, once every 6 weeks, or once every 8 weeks, for the desired treatment duration; and c) a dosage of a SAPK inhibitor, in a weight-based dosage in the range from about 10 µg/kg/day to about 10 mg/kg/day, or a fixed dosage of about 100 µg to about 1000 mg per day, or about 100 µg to about 1 mg per day, or about 1 mg to about 10 mg per day, or about 10 mg to about 100 mg per day, or about 100 mg to about 1000 mg per day, administered orally for the desired treatment duration, to treat the fibrotic disorder.

In some embodiments, the invention provides a combination therapy method involving modification of any one of the above-described treatment regimens featuring administering a subject CXCR3 ligand and a TNF antagonist, where the modification involves further administering an effective amount of a TGF-β antagonist. Effective amounts of a TGF-β antagonist are discussed below.

In some embodiments, the invention provides a combination therapy method involving modification of any one of the above-described treatment regimens featuring administering a subject CXCR3 ligand and a TNF antagonist, where the modification involves further administering an effective amount of an endothelin receptor antagonist. Effective amounts of an endothelin receptor antagonist are discussed below.

### Synthetic CXCR3 ligand in combination therapy with a TGF-β antagonist to treat fibrotic disorders

In some embodiments, a subject therapeutic regimen for treating a fibrotic disorder involves administering effective amounts of a subject synthetic CXCR3 ligand and a TGF-β antagonist. Effective amounts of a TGF-β antagonist include a weight-based dosage in the range from about 0.25 mg/kg/day to about 25 mg/kg/day, or a fixed dosage of about from about 25 µg to about 1000 mg per day (e.g., from about 25 µg to about 50 µg, from about 50 µg to about 75 µg, from about 75 µg to about 100 µg, from about 100 µg to about 200 µg, from about 200 µg to about 500 µg, from about 500 µg to about 1 mg, from about 1 mg to about 10 mg, from about 10 mg to about 25 mg, from about 25 mg to about 50 mg, from about 50 mg to about 100 mg, from about 100 mg to about 200 mg, from about 200 mg to about 300 mg, from about 300 mg to about 400 mg, from about 400 mg to about 500 mg, from about 500 mg to about 750 mg, or from about 750 mg to about 1000 mg, per day), administered orally, subcutaneously, intravenously, or intramuscularly. The dosage will depend, in part, on the specific TGF-β antagonist administered.

In some embodiments, the TGF-β antagonist is GLEEVEC™. Suitable dosages of GLEEVEC™ include, e.g., from about 25 mg to about 1000 mg daily, e.g., 25 mg to 50 mg, 50 mg to 100 mg, 100 mg to 200 mg, 200 mg to 300 mg, 300 mg to 400 mg, 400 mg to 500 mg, 500 mg to 600 mg, 600 mg to 700 mg, 700 mg to 800 mg, 800 mg to 900 mg, or 900 mg to 1000 mg of Gleevec™, daily. In certain embodiments, the total daily dose is administered to a subject as two daily doses of 25 mg to 50 mg, 50 mg to 100 mg, 100 mg to 200 mg, 200 mg to 300 mg, 300 mg to 400 mg, or 400 mg to 500 mg. In a particular embodiment, GLEEVEC™ is administered in an amount of 400 mg GLEEVEC™ orally daily. In another particular embodiment, GLEEVEC™ is administered in an amount of 600 mg GLEEVEC™ orally daily.

A TGF-β antagonist is administered once per month, twice per month, three times per month, once per week, twice per week, three times per week, four times per week, five times per week, six times per week, daily, or in divided daily doses ranging from once daily to 5 times daily over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

Multiple doses of a TGF-β antagonist can be administered, e.g., the TGF-β antagonist can be administered once per month, twice per month, three times per month, once per week, twice per week, three times per week, four times per week, five times per week, six times per week, or daily, over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

In some embodiments, the invention provides a combination therapy method using combined effective amounts of i) a subject synthetic CXCR3 ligand and ii) a TGF-β antagonist in the treatment of a fibrotic disorder in a patient, the method comprising co-administering to the patient a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously for the desired treatment duration; and b) a dosage of a TGF-β antagonist containing an amount of from about 25 µg to about 1000 mg per day, administered orally, subcutaneously, intravenously, or intramuscularly tid, bid, qd, qod, biw, tiw, qw, qow, three times per month, once monthly, for the desired treatment duration, to treat the fibrotic disorder.

In some embodiments, the invention provides a combination therapy method using combined effective amounts of i) a subject synthetic CXCR3 ligand and ii) a TGF-β antagonist in the treatment of a fibrotic disorder in a patient, the method comprising co-administering to the patient a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously for the desired treatment duration; and b) a dosage of GLEEVEC™ containing an amount of 400 mg or 600 mg per day, administered orally once daily for the desired treatment duration, to treat the fibrotic disorder.

In some embodiments, the invention provides a combination therapy method involving modification of any one of the above-described treatment regimens featuring administering a subject CXCR3 ligand and a TGF-β antagonist, where the modification involves further administering an effective amount of pirfenidone or a pirfenidone analog. Effective amounts of pirfenidone or a pirfenidone analog are discussed above.

In some embodiments, the invention provides a combination therapy method involving modification of any one of the above-described treatment regimens featuring administering a subject CXCR3 ligand and a TGF-β antagonist, where the modification involves further administering an effective amount of a Type II interferon receptor agonist. Effective amounts of a Type II interferon receptor agonist are discussed above.

In some embodiments, the invention provides a combination therapy method involving modification of any one of the above-described treatment regimens featuring administering a subject CXCR3 ligand and a TGF-β antagonist, where the modification involves further administering an effective amount of a Type I interferon receptor agonist. Effective amounts of a Type I interferon receptor agonist are discussed above.

In some embodiments, the invention provides a combination therapy method involving modification of any one of the above-described treatment regimens featuring administering a subject CXCR3 ligand and a TGF-β antagonist, where the modification involves further administering an effective amount of a SAPK inhibitor. Effective amounts of a SAPK inhibitor are discussed above.

In some embodiments, the invention provides a combination therapy method involving modification of any one of the above-described treatment regimens featuring administering a subject CXCR3 ligand and a TGF-β antagonist, where the modification involves further administering an effective amount of a TNF antagonist. Effective amounts of a TNF antagonist are discussed above.

In some embodiments, the invention provides a combination therapy method involving modification of any one of the above-described treatment regimens featuring administering a subject CXCR3 ligand and a TGF-β antagonist, where the modification involves further administering an effective amount of an endothelin receptor antagonist. Effective amounts of an endothelin receptor antagonist are discussed below.

### Synthetic CXCR3 ligands in combination therapy with an endothelin receptor antagonist to treat fibrotic disorders

In some embodiments, a subject therapeutic regimen for treating a fibrotic disorder involves administering effective amounts of a subject synthetic CXCR3 ligand and an endothelin receptor antagonist. Effective dosages of an endothelin receptor antagonist include a weight-based dosage in the range from about 0.25 mg/kg/day to about 25 mg/kg/day, or a fixed dosage of about from about 25 µg to about 1000 mg per day (e.g., from about 25 µg to about 50 µg, from about 50 µg to about 75 µg, from about 75 µg to about 100 µg, from about 100 µg to about 200 µg, from about 200 µg to about 500 µg, from about 500 µg to about 1 mg, from about 1 mg to about 10 mg, from about 10 mg to about 25 mg, from about 25 mg to about 50 mg, from about 50 mg to about 100 mg, from about 100 mg to about 200 mg, from about 200 mg to about 300 mg, from about 300 mg to about 400 mg, from about 400 mg to about 500 mg, from about 500 mg to about 750 mg, or from about 750 mg to about 1000 mg, per day). The dosage will depend, in part, on the specific endothelin receptor antagonist administered. An endothelin receptor antagonist is generally administered orally, subcutaneously, intravenously, or intramuscularly, although other routes of administration are also possible.

In some embodiments, the endothelin receptor antagonist is TRACLEER™. Suitable dosages of TRACLEER™ include, e.g., from about 25 mg to about 150 mg once or twice daily, e.g., from about 25 mg to about 30 mg, from about 30 mg to about 40 mg, from about 40 mg to about 50 mg, from about 50 mg to about 60 mg, from about 60 mg to about 70 mg, from about 70 mg to about 80 mg, from about 80 mg to about 90 mg, from about 90 mg to about 100 mg, from about 100 mg to about 125 mg, or from about 125 mg to about 150 mg of TRACLEER™ once or twice daily. In some embodiments, TRACLEER™ is administered in an amount of 62.5 mg TRACLEER™ orally bid for 4 weeks, followed by administering TRACLEER™ in an amount of 125 mg bid orally for the desired treatment duration.

An endothelin receptor antagonist is administered once per month, twice per month, three times per month, once per week, twice per week, three times per week, four times per week, five times per week, six times per week, daily, or in divided daily doses ranging from once daily to 5 times daily over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

Multiple doses of an endothelin receptor antagonist can be administered, e.g., the endothelin receptor antagonist can be administered once per month, twice per month, three times per month, every other week (qow), once per week (qw), twice per week (biw), three times per week (tiw), four times per week, five times per week, six times per week, every other day (qod), daily (qd), twice a day (bid), or three times a day (tid), over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

In some embodiments, the invention provides a combination therapy method using combined effective amounts of i) a subject synthetic CXCR3 ligand and ii) an endothelin receptor antagonist in the treatment of a fibrotic disorder in a patient, the method comprising co-administering to the patient a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously for the desired treatment duration; and b) a dosage of an endothelin receptor antagonist containing an amount of from about 25 µg to about 1000 mg per day, administered *orally*, *subcutaneously*, intravenously, or intramuscularly tid, bid, qd, qod, biw, tiw, qw, qow, three times per month, or once monthly, for the desired treatment duration, to treat the fibrotic disorder.

In some embodiments, the invention provides a combination therapy method using combined effective amounts of i) a subject synthetic CXCR3 ligand and ii) an endothelin receptor antagonist in the treatment of a fibrotic disorder in a patient, the method comprising co-administering to the patient a) a dosage of a subject synthetic CXCR3 ligand containing an amount of from about 0.1 µg to about 1000 µg per dose of a subject synthetic CXCR3 ligand, intramuscularly or subcutaneously qd, qod, tiw, or biw, or per day, substantially continuously or continuously for the desired treatment duration; and b) a dosage of TRACLEER™ containing an amount of 62.5 mg or 125 mg, administered orally bid, for the desired treatment duration, to treat the fibrotic disorder.

In some embodiments, the invention provides a combination therapy method involving modification of any one of the above-described treatment regimens featuring administering a subject CXCR3 ligand and an endothelin receptor antagonist, where the modification involves further administering an effective amount of pirfenidone or a pirfenidone analog. Effective amounts of pirfenidone or a pirfenidone analog are discussed above.

In some embodiments, the invention provides a combination therapy method involving modification of any one of the above-described treatment regimens featuring administering a subject CXCR3 ligand and an endothelin receptor, where the modification involves further administering an effective amount of a Type II interferon receptor agonist. Effective amounts of a Type II interferon receptor agonist are discussed above.

In some embodiments, the invention provides a combination therapy method involving modification of any one of the above-described treatment regimens featuring administering a subject CXCR3 ligand and an endothelin receptor, where the modification involves further administering an effective amount of a Type I interferon receptor agonist. Effective amounts of a Type I interferon receptor agonist are discussed above.

In some embodiments, the invention provides a combination therapy method involving modification of any one of the above-described treatment regimens featuring administering a subject CXCR3 ligand and an endothelin receptor antagonist, where the modification involves further administering an effective amount of a SAPK inhibitor. Effective amounts of a SAPK inhibitor are discussed above.

In some embodiments, the invention provides a combination therapy method involving modification of any one of the above-described treatment regimens featuring administering a subject CXCR3 ligand and an endothelin receptor antagonist, where the modification involves further administering an effective amount of a TNF antagonist. Effective amounts of a TNF antagonist are discussed above.

In some embodiments, the invention provides a combination therapy method involving modification of any one of the above-described treatment regimens featuring administering a subject CXCR3 ligand and an endothelin receptor antagonist, where the modification involves further administering an effective amount of a TGF-β antagonist. Effective amounts of a TGF-β antagonist are discussed above.

### Synthetic CXCR3 ligand in combination therapy with N-acetyl cysteine to treat fibrotic disorders

In some embodiments, a subject method of treating a fibrotic disorder involves administering effective amounts of a subject synthetic CXCR3 ligand and N-acetyl cysteine. Furthermore, any of the above-described treatment regimens for treating a fibrotic disorder can be modified to include administering an effective amount of N-acetylcysteine (NAC).

Effective dosages of NAC can range from about 100 mg to about 1000 mg per day, or from about 100 mg to about 500 mg per day, or from about 500 mg to about 750 mg per day, or from about 750 mg to about 1000 mg per day, or from about 400 mg to about 3600 mg per day, or from about 800 mg to about 2400 mg per day, or from about 1000 mg to about 1800 mg per day, or from about 1200 mg to about 1600 mg per day.

NAC can be administered once per month, twice per month, three times per month, once per week, twice per week, three times per week, four times per week, five times per week, six times per week, every other day, daily, twice daily, three times per day, or in divided daily doses ranging from 2 to 5 times daily.

NAC can be administered at any frequency, and over a period of time ranging from about one day to about one week, from about two weeks to about four weeks, from about one month to about two months, from about two months to about four months, from about four months to about six months, from about six months to about eight months, from about eight months to about 1 year, from about 1 year to about 2 years, or from about 2 years to about 4 years, or more.

In some embodiments, NAC is administered throughout the entire course of the subject combination therapy. In other embodiments, NAC is administered less than the entire course of the combination therapy, e.g., only during the first phase of the combination therapy, only during the second phase of the combination therapy, or some other portion of the combination therapy treatment regimen.

In some embodiments, NAC is administered at a dosage of NAC containing an amount of from about 500 mg to about 3000 mg of NAC per day, administered orally, optionally in two or more divided doses per day, for the desired treatment duration.

In some embodiments, NAC is administered at a dosage of 500 mg of drug per dose orally once daily, twice daily or three times daily, for the desired treatment duration.

In other embodiments, NAC is administered at a dosage of 600 mg of drug per dose orally once daily, twice daily or three times daily, for the desired treatment duration.

In other embodiments, NAC is administered at a dosage of 750 mg of drug per dose orally once daily, twice daily or three times daily, for the desired treatment duration.

In other embodiments, NAC is administered at a dosage of 1000 mg of drug per dose orally once daily, twice daily or three times daily, for the desired treatment duration.

### Further combinations

As non-limiting examples, any of the above-described treatment methods featuring a Type II interferon receptor agonist regimen can be modified to replace the subject Type II interferon receptor agonist regimen with a regimen of IFN-γ comprising administering a dosage of IFN-γ containing an amount of 25 µg of drug per dose, subcutaneously three times per week for the desired treatment duration.

As non-limiting examples, any of the above-described treatment methods featuring a Type II interferon receptor agonist regimen can be modified to replace the subject Type II interferon receptor agonist regimen with a regimen of IFN-γ comprising administering a dosage of IFN-γ containing an amount of 50 µg of drug per dose, subcutaneously three times per week for the desired treatment duration.

As non-limiting examples, any of the above-described treatment methods featuring a Type II interferon receptor agonist regimen can be modified to replace the subject Type II interferon receptor agonist regimen with a regimen of IFN-γ comprising administering a dosage of IFN-γ containing an amount of 100 µg of drug per dose, subcutaneously three times per week for the desired treatment duration.

As non-limiting examples, any of the above-described treatment methods featuring a Type II interferon receptor agonist regimen can be modified to replace the subject Type II interferon receptor agonist regimen with a regimen of IFN-γ comprising administering a dosage of IFN-γ containing an amount of 200 µg of drug per dose, subcutaneously three times per week for the desired treatment duration.

As non-limiting examples, any of the above-described treatment methods featuring a TGF-β antagonist regimen can be modified to replace the subject TGF-β antagonist regimen with a regimen of Gleevec™ comprising administering a dosage of Gleevec™ containing an amount of 400 mg to 800 mg, or 600 mg, of drug orally per day, optionally in two or more divided doses per day, for the desired treatment duration.

As non-limiting examples, any of the above-described treatment methods featuring an endothelin receptor antagonist regimen can be modified to replace the subj ect endothelin receptor antagonist regimen with an endothelin receptor antagonist regimen comprising administering a dosage of Tracleer™ containing an amount of 62.5 mg of drug orally twice per day for the first 4 weeks of therapy, followed by a dosage of Tracleer™ containing an amount of 125 mg of drug orally twice per day for the remainder of therapy; for the desired treatment duration.

As non-limiting examples, any of the above-described treatment methods featuring a TNF antagonist regimen can be modified to replace the subject TNF antagonist regimen with a TNF antagonist regimen comprising administering a dosage of a TNF antagonist selected from the group of: (i) etanercept in an amount of 25 mg of drug per dose subcutaneously twice per week, (ii) infliximab in an amount of 3 mg of drug per kilogram of body weight per dose intravenously at weeks 0, 2 and 6, and every 8 weeks thereafter, or (iii) adalimumab in an amount of 40 mg of drug per dose subcutaneously once weekly or once every 2 weeks; for the desired treatment duration.

The subject invention provides any of the above-described treatment methods, modified to include administering an effective amount of a side effect management agent (a "palliative agent") for the desired treatment duration. In many embodiments, side effect management agents are selected from one or more of acetaminophen, ibuprofen, and other NSAIDs, H2 blockers, and antacids. For example, where the second therapeutic agent in a subject combination therapy is an IFN-α or an IFN-γ, a side effect management agent may be administered.

Suitable NSAIDs include include, but are not limited to, 1) the oxicams, such as piroxicam, isoxicam, tenoxicam, and sudoxicam; 2) the salicylates, such as aspirin, disalcid, benorylate, trilisate, safapryn, solprin, diflunisal, and fendosal; 3) the acetic acid derivatives, such as diclofenac, fenclofenac, indomethacin, sulindac, tolmetin, isoxepac, furofenac, tiopinac, zidometacin, acematacin, fentiazac, zomepiract, clidanac, oxepinac, and felbinac; 4) the fenamates, such as mefenamic, meclofenamic, flufenamic, niflumic, and tolfenamic acids; 5) the propionic acid derivatives, such as ibuprofen, naproxen, benoxaprofen, flurbiprofen, ketoprofen, fenoprofen, fenbufen, indoprofen, pirprofen, carprofen, oxaprozin, pranoprofen, miroprofen, tioxaprofen, suprofen, alminoprofen, and tiaprofenic; and 6) the pyrazoles, such as phenylbutazone, oxyphenbutazone, feprazone, azapropazone, and trimethazone, mixtures of these non-steroidal anti-inflammatory agents may also be employed, as well as the pharmaceutically-acceptable salts and esters of these agents.

Suitable H2 blockers (histamine type 2 receptor antagonists) that are suitable for use as a palliative agent in a subject therapy include, but are not limited to, Cimetidine (e.g., Tagamet, Peptol, Nu-cimet, apo-cimetidine, non-cimetidine); Ranitidine (e.g., Zantac, Nu-ranit, Novorandine, and apo-ranitidine); and Famotidine (Pepcid, Apo-Famotidine, and Novo-Famotidine).

### SUBJECT SUITABLE FOR TREATMENT

The subject methods are suitable for treatment with a subject method of treating a fibrotic disorder include individuals diagnosed as having a fibrotic disease, such as IPF, liver fibrosis or renal fibrosis. The subject methods are also suitable for treatment of individuals who are at risk of developing a fibrotic disease.

Subjects suitable for treatment with a method of cancer treatment of the present invention include individuals having any type of cancer. In particular embodiments, a suitable subject is one having a cancer that is susceptible to treatment with synthetic CXCR3 ligand therapy.

Individuals with liver fibrosis who are suitable for treatment according to the methods of the invention include individuals who have been clinically diagnosed with liver fibrosis, as well as individuals who have not yet developed clinical liver fibrosis but who are considered at risk of developing liver fibrosis. Such individuals include, but are not limited to, individuals who are infected with HCV; individuals who are infected with HBV; individuals who are infected with *Schistosoma mansoni*; individuals who have been exposed to chemical agents known to result in liver fibrosis; individuals who have been diagnosed with Wilson's disease; individuals diagnosed with hemochromatosis; and individuals with alcoholic liver disease; individuals with non-alcoholic steatohepatitis; individuals with autoimmune hepatitis; individuals with primary sclerosing cholangitis, primary biliary cirrhosis, or alpha-1-antitrysin deficiency.

Individuals who have been clinically diagnosed as infected with HCV are of particular interest in many embodiments. Individuals who are infected with HCV are identified as having HCV RNA in their blood, and/or having anti-HCV antibody in their serum. In many embodiments, individuals of interest include those who exhibit severe fibrosis or early cirrhosis (non-decompensated, Child's-Pugh class A or less), or more advanced cirrhosis (decompensated, Child's-Pugh class B or C) due to chronic HCV infection and who are viremic despite prior anti-viral treatment with IFN-α-based therapies or who cannot tolerate IFN-α-based therapies, or who have a contraindication to such therapies. In particular embodiments of interest, HCV-positive individuals with stage 3 or 4 liver fibrosis according to the METAVIR scoring system are suitable for treatment with the methods of the present invention. In other embodiments, individuals suitable for treatment with the methods of the instant invention are patients with decompensated cirrhosis with clinical manifestations, including patients with far-advanced liver cirrhosis, including those awaiting liver transplantation. In still other embodiments, individuals suitable for treatment with the methods of the instant invention include patients with milder degrees of fibrosis including those with early fibrosis (stages 1 and 2 in the METAVIR, Ludwig, and Scheuer scoring systems; or stages 1, 2, or 3 in the Ishak scoring system.).

The subject methods are suitable for treatment of individuals diagnosed as having IPF. The methods are also suitable for treatment of individuals having IPF who were previously treated with corticosteroids within the previous 24 months, and who failed to respond to previous treatment with corticosteroids. Also included are subjects who have an FVC at the outset of treatment that is at least 55% of the predicted FVC. The percent predicted FVC values are based on normal values, which are known in the art. See, e.g., Crapo et al. (1981) Am. Rev. Respir. Dis. 123:659-664. FVC is measured using standard methods of spirometry.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

## Claims

1. A synthetic CXCR3 polypeptide ligand comprising a polypeptide of from about 70 to about 125 amino acids in length, optionally further including an additional methionine attached to the ordinarily first amino acid at the N-terminus, the amino acid sequence of the polypeptide comprising, in sequence, discrete sub-sequences corresponding in amino acid identity and number to sub-sequences of different, naturally occurring CXCR3 ligands selected from IP-10, I-TAC, and Mig, where the amino acid sequence of the synthetic CXCR3 polypeptide differs from the amino acid sequence of naturally occurring CXCR3 ligands IP-10, I-TAC, and Mig.

2. The synthetic CXCR3 polypeptide ligand of claim 1, wherein the CXCR3 ligand comprises the amino acid sequence as set forth in any one of SEQ ID NOs: 15-20.

3. A synthetic CXCR3 ligand comprising a polypeptide of from about 70 to about 125 amino acids in length, optionally further including an additional methionine attached to the ordinarily first amino acid at the N-terminus, the amino acid sequence of the polypeptide comprising those amino acid residues that are common to IP-10, Mig, and ITAC, and which comprises, at one or more of those positions where there is no amino acid common to IP-10, Mig, and 1-TAC, an amino acid which predominantly occurs at that position.

4. The synthetic CXCR3 polypeptide ligand of claim 3, wherein the CXCR3 ligand comprises the amino acid sequence as set forth in any one of SEQ ID NOs: 1, 2, and 3.

5. A composition comprising the synthetic CXCR3 ligand of any of claims 1-4.

6. A polynucleotide comprising a nucleotide sequence encoding a synthetic CXCR3 ligand of any of claims 1-4.

7. An expression vector comprising the polynucleotide of claim 6 operably linked to a promoter.

8. A host cell comprising the polynucleotide of claim 6 or the expression vector of claim 7.

9. A method for producing a synthetic CXCR3 ligand, the method comprising: culturing the host cell of claim 8 under conditions that favor production of the synthetic CXCR3 ligand; and isolating the synthetic CXCR3 ligand from the culture.

10. An antibody that specifically binds a synthetic CXCR3 ligand of any one of claims 1-4.

11. Synthetic CXCR3 ligand for use in a method of treating or preventing a fibrotic disease in an individual, the method comprising administering to an individual suffering from a fibrotic disease an amount of said synthetic CXCR3 ligand that is effective in the treatment or prophylaxis of the fibrotic disease in the individual.

12. The synthetic CXCR3 ligand for use according to claim 11, wherein the fibrotic disease is selected from the group consisting of pulmonary fibrosis, preferably idiopathic pulmonary fibrosis or a pulmonary fibrosis of known etiology, liver fibrosis, renal fibrosis, cardiac fibrosis, and scleroderma.

13. Synthetic CXCR3 ligand for use in a method of reducing tumor growth in an individual having a tumor, the method comprising administering to the individual an effective amount of said synthetic CXCR3 ligand.

14. The synthetic CXCR3 ligand for use according to claim 13, wherein the method further comprises administering an effective amount of an anti-neoplastic agent selected from an alkylating agent, a nitrosourea, an antimetabolite, an antitumor antibiotic, a plant (vinca) alkaloid, a taxane, and a steroid hormone.

15. The method of any of claims 11-14, wherein the individual is a human.


SEQUENCE LISTING

<110>  Intermune, Inc.

<120>  Synthetic chemokine receptor ligands and methods of use thereof

<130>  P47516

<140>
<141>  2004-05-13

<150>  60/471,404
<151>  2003-05-16

<160>  20

<170>  PatentIn version 3.5

<210>  1
<211>  77
<212>  PRT
<213>  Artificial Sequence

<220>
<223>  consensus IP-10 sequence

<400>  1

Val Pro Leu Ser Arg Thr Gly Arg Cys Thr Cys Ile Ser Ile Ser Asn
1               5                   10                  15

Gln Pro Val Asn Pro Arg Ser Leu Glu Lys Leu Glu Ile Ile Pro Pro
            20                  25                  30

Ser Gln Phe Cys Pro Lys Ile Glu Ile Ile Ala Thr Leu Lys Lys Asn
        35                  40                  45

Gly Glu Gln Arg Cys Leu Asn Pro Glu Ser Lys Ala Ile Lys Asn Leu
    50                  55                  60

Ile Lys Lys Val Ser Arg Glu Met Ser Lys Lys Ser Pro
65                  70                  75

<210>  2
<211>  74
<212>  PRT
<213>  Artificial Sequence

<220>
<223>  consensus I-TAC sequence

<400>  2

Phe Pro Met Phe Arg Arg Gly Arg Cys Leu Cys Ile Ser Pro Gly Val
1               5                   10                  15

Lys Ala Val Lys Val Ala Ser Leu Glu Lys Leu Ser Ile Met Tyr Pro
            20                  25                  30

Ser Asn Asn Cys Asp Lys Ile Glu Ile Ile Ala Thr Leu Lys Lys Asn
        35                  40                  45

Gly Gly Gln Arg Cys Leu Asn Pro Lys Ser Lys Gln Ala Lys Leu Leu
    50                  55                  60

Ile Lys Lys Val Glu Arg Lys Lys Asn Phe
65                  70

<210> 3
<211> 104
<212> PRT
<213> Artificial Sequence

<220>
<223> consensus Mig sequence

<400> 3

Thr Pro Val Val Arg Lys Gly Arg Cys Ser Cys Ile Ser Thr Asn Gln
1               5                   10                  15

Gly Thr Val His Leu Gln Ser Leu Glu Lys Leu Lys Ile Phe Ala Pro
            20                  25                  30

Ser Pro Ser Cys Glu Lys Ile Glu Ile Ile Ala Thr Leu Lys Lys Asn
        35                  40                  45

Gly Val Gln Arg Cys Leu Asn Pro Asp Ser Lys Asp Val Lys Glu Leu
    50                  55                  60

Ile Lys Lys Trp Glu Lys Gln Val Ser Gln Lys Lys Lys Gln Lys Asn
65                  70                  75                  80

Gly Lys Lys His Gln Lys Lys Lys Val Leu Lys Val Arg Lys Val Gln
                85                  90                  95

Arg Ser Arg Gln Lys Lys Thr Thr
            100

<210> 4
<211> 10
<212> PRT
<213> Artificial Sequence

<220>
<223> epitope tag

<400> 4

Cys Tyr Pro Tyr Asp Val Pro Asp Tyr Ala
1               5                   10

<210>  5
<211>  8
<212>  PRT
<213>  Artificial Sequence

<220>
<223>  epitope tag

<400>  5

Asp Tyr Lys Asp Asp Asp Asp Lys
1               5


<210>  6
<211>  11
<212>  PRT
<213>  Artificial Sequence

<220>
<223>  epitope tag

<400>  6

Cys Glu Gln Lys Leu Ile Ser Glu Glu Asp Leu
1               5                   10


<210>  7
<211>  5
<212>  PRT
<213>  Artificial Sequence

<220>
<223>  protease cleavage site

<400>  7

Asp Asp Asp Asp Lys
1               5


<210>  8
<211>  4
<212>  PRT
<213>  Artificial Sequence

<220>
<223>  protease cleavage site

<400>  8

Ile Glu Gly Arg
1


<210>  9
<211>  6
<212>  PRT
<213>  Artificial Sequence

<220>

<223>  protease cleavage site

<400>  9

Leu Val Pro Arg Gly Ser
1               5


<210>  10
<211>  8
<212>  PRT
<213>  Artificial Sequence

<220>
<223>  protease cleavage site

<400>  10

His Pro Phe His Leu Val Ile His
1               5


<210>  11
<211>  104
<212>  PRT
<213>  Artificial Sequence

<220>
<223>  Consensus Sequence for IP-10, I-TAC and Mig


<220>
<221>  misc_feature
<222>  (1)..(1)
<223>  Xaa can be any naturally occurring amino acid

<220>
<221>  misc_feature
<222>  (3)..(4)
<223>  Xaa can be any naturally occurring amino acid

<220>
<221>  misc_feature
<222>  (6)..(6)
<223>  Xaa can be any naturally occurring amino acid

<220>
<221>  misc_feature
<222>  (10)..(10)
<223>  Xaa can be any naturally occurring amino acid

<220>
<221>  misc_feature
<222>  (14)..(18)
<223>  Xaa can be any naturally occurring amino acid

<220>
<221>  misc_feature
<222>  (20)..(22)
<223>  Xaa can be any naturally occurring amino acid

<220>
<221>  misc_feature
<222>  (28)..(28)

<223>  Xaa can be any naturally occurring amino acid

<220>
<221>  misc_feature
<222>  (30)..(31)
<223>  Xaa can be any naturally occurring amino acid

<220>
<221>  misc_feature
<222>  (34)..(35)
<223>  Xaa can be any naturally occurring amino acid

<220>
<221>  misc_feature
<222>  (37)..(37)
<223>  Xaa can be any naturally occurring amino acid

<220>
<221>  misc_feature
<222>  (50)..(50)
<223>  Xaa can be any naturally occurring amino acid

<220>
<221>  misc_feature
<222>  (57)..(57)
<223>  Xaa can be any naturally occurring amino acid

<220>
<221>  misc_feature
<222>  (60)..(61)
<223>  Xaa can be any naturally occurring amino acid

<220>
<221>  misc_feature
<222>  (63)..(63)
<223>  Xaa can be any naturally occurring amino acid

<220>
<221>  misc_feature
<222>  (68)..(94)
<223>  Xaa can be any naturally occurring amino acid

<220>
<221>  misc_feature
<222>  (96)..(96)
<223>  Xaa can be any naturally occurring amino acid

<220>
<221>  misc_feature
<222>  (98)..(100)
<223>  Xaa can be any naturally occurring amino acid

<220>
<221>  misc_feature
<222>  (103)..(104)
<223>  Xaa can be any naturally occurring amino acid

<400>  11

Xaa Pro Xaa Xaa Arg Xaa Gly Arg Cys Xaa Cys Ile Ser Xaa Xaa Xaa
1               5                   10                  15

Xaa Xaa Val Xaa Xaa Xaa Ser Leu Glu Lys Leu Xaa Ile Xaa Xaa Pro

20                  25                  30

Ser Xaa Xaa Cys Xaa Lys Ile Glu Ile Ile Ala Thr Leu Lys Lys Asn
        35                  40                  45

Gly Xaa Gln Arg Cys Leu Asn Pro Xaa Ser Lys Xaa Xaa Lys Xaa Leu
    50                  55                  60

Ile Lys Lys Xaa Xaa Xaa Xaa Xaa Xaa Xaa Xaa Xaa Xaa Xaa Xaa Xaa
65                  70                  75                  80

Xaa Xaa Xaa Xaa Xaa Xaa Xaa Xaa Xaa Xaa Xaa Xaa Xaa Xaa Val Xaa
                85                  90                  95

Arg Xaa Xaa Xaa Lys Lys Xaa Xaa
            100

<210>  12
<211>  98
<212>  PRT
<213>  Homo sapiens

<400>  12

Met Asn Gln Thr Ala Ile Leu Ile Cys Cys Leu Ile Phe Leu Thr Leu
1               5                   10                  15

Ser Gly Ile Gln Gly Val Pro Leu Ser Arg Thr Val Arg Cys Thr Cys
            20                  25                  30

Ile Ser Ile Ser Asn Gln Pro Val Asn Pro Arg Ser Leu Glu Lys Leu
        35                  40                  45

Glu Ile Ile Pro Ala Ser Gln Phe Cys Pro Arg Val Glu Ile Ile Ala
    50                  55                  60

Thr Met Lys Lys Lys Gly Glu Lys Arg Cys Leu Asn Pro Glu Ser Lys
65                  70                  75                  80

Ala Ile Lys Asn Leu Leu Lys Ala Val Ser Lys Glu Met Ser Lys Arg
                85                  90                  95

Ser Pro

<210>  13
<211>  94
<212>  PRT
<213>  Homo sapiens

<400>  13

Met Ser Val Lys Gly Met Ala Ile Ala Leu Ala Val Ile Leu Cys Ala
1               5                   10                  15

Thr Val Val Gln Gly Phe Pro Met Phe Lys Arg Gly Arg Cys Leu Cys
            20                  25                  30

Ile Gly Pro Gly Val Lys Ala Val Lys Val Ala Asp Ile Glu Lys Ala
        35                  40                  45

Ser Ile Met Tyr Pro Ser Asn Asn Cys Asp Lys Ile Glu Val Ile Ile
    50                  55                  60

Thr Leu Lys Glu Asn Lys Gly Gln Arg Cys Leu Asn Pro Lys Ser Lys
65                  70                  75                  80

Gln Ala Arg Leu Ile Ile Lys Lys Val Glu Arg Lys Asn Phe
                85                  90

<210>  14
<211>  125
<212>  PRT
<213>  Homo sapiens

<400>  14

Met Lys Lys Ser Gly Val Leu Phe Leu Leu Gly Ile Ile Leu Leu Val
1               5                   10                  15

Leu Ile Gly Val Gln Gly Thr Pro Val Val Arg Lys Gly Arg Cys Ser
            20                  25                  30

Cys Ile Ser Thr Asn Gln Gly Thr Ile His Leu Gln Ser Leu Lys Asp
        35                  40                  45

Leu Lys Gln Phe Ala Pro Ser Pro Ser Cys Glu Lys Ile Glu Ile Ile
    50                  55                  60

Ala Thr Leu Lys Asn Gly Val Gln Thr Cys Leu Asn Pro Asp Ser Ala
65                  70                  75                  80

Asp Val Lys Glu Leu Ile Lys Lys Trp Glu Lys Gln Val Ser Gln Lys
                85                  90                  95

Lys Lys Gln Lys Asn Gly Lys Lys His Gln Lys Lys Lys Val Leu Lys
            100                 105                 110

Val Arg Lys Ser Gln Arg Ser Arg Gln Lys Lys Thr Thr
        115                 120                 125

<210>  15

<211>  98
<212>  PRT
<213>  Artificial Sequence

<220>
<223>  hybrid CXCR3 ligand

<400>  15

Met Lys Lys Ser Gly Val Leu Phe Leu Leu Gly Ile Ile Leu Leu Val
1               5                   10                  15

Leu Ile Gly Val Gln Gly Phe Pro Met Phe Lys Arg Gly Arg Cys Leu
            20                  25                  30

Cys Ile Gly Pro Gly Val Lys Pro Val Asn Pro Arg Ser Leu Glu Lys
        35                  40                  45

Leu Glu Ile Ile Pro Ala Ser Gln Phe Cys Pro Arg Ile Glu Ile Ile
    50                  55                  60

Ala Thr Leu Lys Asn Gly Val Gln Thr Cys Leu Asn Pro Asp Ser Lys
65                  70                  75                  80

Gln Ala Arg Leu Ile Ile Lys Lys Val Ser Lys Glu Met Ser Lys Arg
                85                  90                  95

Ser Pro



<210>  16
<211>  124
<212>  PRT
<213>  Artificial Sequence

<220>
<223>  hybrid CXCR3 ligand

<400>  16

Met Asn Gln Thr Ala Ile Leu Ile Cys Cys Leu Ile Phe Leu Thr Leu
1               5                   10                  15

Ser Gly Ile Gln Gly Val Pro Leu Ser Arg Thr Val Arg Cys Leu Cys
            20                  25                  30

Ile Gly Pro Gly Val Lys Ala Val Lys Val Ala Asp Ile Glu Lys Ala
        35                  40                  45

Ser Ile Met Tyr Pro Ser Asn Asn Cys Asp Lys Ile Glu Ile Ile Ala
    50                  55                  60

Thr Leu Lys Asn Gly Val Gln Thr Cys Leu Asn Pro Asp Ser Ala Asp

65                  70                  75                  80

Val Lys Glu Leu Ile Lys Lys Trp Glu Lys Gln Val Ser Gln Lys Lys
                85                  90                  95

Lys Gln Lys Asn Gly Lys Lys His Gln Lys Lys Lys Val Leu Lys Val
            100                 105                 110

Arg Lys Ser Gln Arg Ser Arg Gln Lys Lys Thr Thr
        115                 120

<210>  17
<211>  125
<212>  PRT
<213>  Artificial Sequence

<220>
<223>  hybrid CXC3 ligand

<400>  17

Met Asn Gln Thr Ala Ile Leu Ile Cys Cys Leu Ile Phe Leu Cys Ala
1               5                   10                  15

Thr Val Val Gln Gly Phe Pro Met Phe Lys Arg Gly Arg Cys Leu Cys
            20                  25                  30

Ile Gly Pro Gly Val Lys Ala Val Lys Val Ala Asp Ile Glu Lys Ala
        35                  40                  45

Ser Ile Met Tyr Pro Ser Asn Asn Cys Asp Lys Ile Glu Val Ile Ile
    50                  55                  60

Thr Leu Lys Glu Asn Lys Gly Gln Arg Cys Leu Asn Pro Lys Ser Ala
65                  70                  75                  80

Asp Val Lys Glu Leu Ile Lys Lys Trp Glu Lys Gln Val Ser Gln Lys
                85                  90                  95

Lys Lys Gln Lys Asn Gly Lys Lys His Gln Lys Lys Lys Val Leu Lys
            100                 105                 110

Val Arg Lys Ser Gln Arg Ser Arg Gln Lys Lys Thr Thr
        115                 120                 125

<210>  18
<211>  124
<212>  PRT
<213>  Artificial Sequence

<220>
<223>  hybrid CXCR3 ligand

<400>  18

Met Asn Gln Thr Ala Ile Leu Ile Cys Cys Leu Ile Phe Leu Thr Leu
1               5                   10                  15

Ser Gly Ile Gln Gly Val Pro Leu Ser Arg Thr Val Arg Cys Thr Cys
            20                  25                  30

Ile Ser Ile Ser Asn Gln Ala Val Lys Val Ala Asp Ile Glu Lys Ala
        35                  40                  45

Ser Ile Met Tyr Pro Ser Asn Asn Cys Asp Lys Ile Glu Val Ile Ile
    50                  55                  60

Thr Leu Lys Glu Asn Lys Gly Gln Arg Cys Leu Asn Pro Lys Ser Lys
65                  70                  75                  80

Gln Ala Arg Leu Ile Ile Lys Lys Glu Lys Gln Val Ser Gln Lys Lys
                85                  90                  95

Lys Gln Lys Asn Gly Lys Lys His Gln Lys Lys Lys Val Leu Lys Val
            100                 105                 110

Arg Lys Ser Gln Arg Ser Arg Gln Lys Lys Thr Thr
        115                 120


<210>  19
<211>  124
<212>  PRT
<213>  Artificial Sequence

<220>
<223>  hybrid CXCR3 ligand

<400>  19

Met Asn Gln Thr Ala Ile Leu Ile Cys Cys Leu Ile Phe Leu Thr Leu
1               5                   10                  15

Ser Gly Ile Gln Gly Phe Pro Met Phe Lys Arg Gly Arg Cys Ser Cys
            20                  25                  30

Ile Ser Thr Asn Gln Gly Thr Ile His Leu Gln Ser Leu Lys Asp Leu
        35                  40                  45

Glu Ile Ile Pro Ala Ser Gln Phe Cys Pro Arg Ile Glu Val Ile Ile
    50                  55                  60

Thr Leu Lys Glu Asn Lys Gly Gln Arg Cys Leu Asn Pro Lys Ser Lys
65                  70                  75                  80

Gln Ala Arg Leu Ile Ile Lys Lys Glu Lys Gln Val Ser Gln Lys Lys

85                  90                  95

Lys Gln Lys Asn Gly Lys Lys His Gln Lys Lys Lys Val Leu Lys Val
            100                 105                 110

Arg Lys Ser Gln Arg Ser Arg Gln Lys Lys Thr Thr
        115                 120

<210>  20
<211>  98
<212>  PRT
<213>  Artificial Sequence

<220>
<223>  hybrid CXCR3 ligand

<400>  20

Met Ser Val Lys Gly Met Ala Ile Ala Leu Ala Val Ile Leu Cys Ala
1               5                   10                  15

Thr Val Ile Gln Gly Val Pro Leu Ser Arg Thr Val Arg Cys Thr Cys
            20                  25                  30

Ile Ser Ile Ser Asn Gln Thr Ile His Leu Gln Ser Leu Lys Asp Leu
        35                  40                  45

Lys Gln Phe Ala Pro Ser Pro Ser Cys Glu Lys Val Glu Ile Ile Ala
    50                  55                  60

Thr Met Lys Lys Lys Gly Glu Lys Arg Cys Leu Asn Pro Glu Ser Lys
65                  70                  75                  80

Gln Ala Arg Leu Ile Ile Lys Lys Val Ser Lys Glu Met Ser Lys Arg
                85                  90                  95

Ser Pro